# EUROPEAN PATENT SPECIFICATION

(11) **EP 4 247 246 B1**
(45) Date of publication and mention of the grant of the patent: **13.05.2026**
(21) Application number: 21893131.9
(22) Date of filing: 16.11.2021
(51) Int. Cl.: A61B 5/024, A61B 5/026, A61B 5/293, A61B 5/00, A61B 5/369, A61B 5/021, A61B 5/0205, A61B 5/11, A61B 5/1455

(54) **SYSTEMS FOR DETERMINATION OF TREATMENT THERAPEUTIC WINDOW, DETECTION, PREDICTION, AND CLASSIFICATION OF NEUROELECTRICAL, CARDIAC AND/OR PULMONARY EVENTS, AND OPTIMIZATION OF TREATMENT ACCORDING TO THE SAME**
SYSTEME ZUR BESTIMMUNG DES THERAPIEFENSTERS, NACHWEIS, VORHERSAGE UND KLASSIFIZIERUNG NEUROELEKTRISCHER, HERZ- UND/ODER LUNGENEREIGNISSE UND OPTIMIERUNG DER BEHANDLUNG DAFÜR
SYSTÈMES POUR LA DÉTERMINATION D'UNE FENÊTRE THÉRAPEUTIQUE DE TRAITEMENT, LA DÉTECTION, LA PRÉDICTION ET LA CLASSIFICATION D'ÉVÉNEMENTS NEUROÉLECTRIQUES, CARDIAQUES ET/OU PULMONAIRES, ET L'OPTIMISATION DU TRAITEMENT EN FONCTION DE CEUX-CI

(30) Priority: 18.11.2020 US 202063115363 P; 09.03.2021 US 202163158833 P; 26.04.2021 US 202163179604 P; 12.07.2021 US 202163220797 P
(43) Date of publication of application: 27.09.2023
(60) Divisional application: 26167709.0
(73) Proprietor: Epiminder Limited, East Melbourne VIC 3002 (AU); Cochlear Limited, Macquarie University, New South Wales 2109 (AU)
(72) Inventor: HEASMAN, John Michael, Melbourne, Victoria 3002 (AU); COOK, Mark James, Fitzroy, Victoria 3065 (AU); KLUPACS, Robert John, Melbourne, Victoria 3002 (AU); HOARE, Rohan, Melbourne, Victoria 3002 (AU)
(74) Representative: Ipsilon
(86) International application number: PCT/AU2021/051355
(87) International publication number: WO 2022/104412

(56) References cited:
- WO-A2-2011/076884
- US-A1- 2010 228 103
- US-A1- 2011 213 222
- US-A1- 2012 047 105
- US-A1- 2017 196 497
- US-A1- 2019 298 248
- US-A1- 2019 328 306
- US-A1- 2019 347 476
- US-B2- 8 684 921

## Description

### TECHNICAL FIELD

The present disclosure relates to systems and methods for monitoring various types of physiological activity in a subject. In particular, the disclosure relates to systems and methods for monitoring neurological activity in a subject and, more particularly, to detecting and classifying events occurring in the subject that are, or appear similar to, epileptic events. The disclosure also relates particularly to methods and systems for monitoring electroencephalographical and photoplethysmographical activity in a subject and, more particularly, to determining a therapeutic window of a treatment, and detecting, predicting, classifying neuroelectrical, vestibular, cochlear, cardiac, and pulmonary events and conditions occurring in the subject, and using the detection, prediction, and classification, combined with the determined therapeutic window to optimize treatment.

### BACKGROUND OF THE DISCLOSURE

Epilepsy is considered the world's most common serious brain disorder, with an estimated 50 million sufferers worldwide and 2.4 million new cases occurring each year.

Epilepsy is a condition of the brain characterized by epileptic seizures that vary from brief and barely detectable seizures to more conspicuous seizures in which a sufferer vigorously shakes. Epileptic seizures are unprovoked, recurrent, and due to unexplained causes.

Additionally, epilepsy is but one of a variety of physiopathologies that have neurological components. Among these, epilepsy, inner ear disorders, and certain sleep disorders affect tens of millions of patients and account for a variety of symptoms with effects ranging from mild discomfort to death. Vestibular disorders, sometimes caused by problems with signaling between the inner ear's vestibular system and the brain, and other times caused by damage or other issues with the physical structures in the inner ear, can cause dizziness, blurred vision, disorientation, falls, nausea, and other symptoms that can range from uncomfortable to debilitating. Cochlear disorders are commonly associated with changes in the ability to hear, including hearing loss and tinnitus, and may be temporary, long-lasting, or permanent. Sleep apnea, meanwhile, is a sleep disorder in which breathing may stop while a person is sleeping. Sleep apnea may be obstructive in nature (e.g., the physiology of the throat may block the airway), or may be neurological (central sleep apnea) in nature. The effects of sleep apnea may be relatively minor (e.g., snoring, trouble sleeping, etc.) and lead to poor sleep quality, irritability, headaches, trouble focusing, and the like, or can be more severe including causing neurological damage or even cardiac arrest and death.

Diagnosing these disorders can be challenging, especially where, as with epilepsy or sleep apnea, diagnosis typically requires detailed study of both clinical observations and electrical and/or other signals in the patient's brain and/or body. Diagnosing epilepsy typically requires detailed study of both clinical observations and electrical and/or other signals in the patient's brain and/or body. Particularly with respect to studying electrical activity in the patient's brain (e.g., using electroencephalography to produce an electroencephalogram (EEG)), such study usually requires the patient to be monitored for some period of time. The monitoring of electrical activity in the brain requires the patient to have a number of electrodes placed on the scalp, each of which electrodes is typically connected to a data acquisition unit that samples the signals continuously (e.g., at a high rate) to record the signals for later analysis. Medical personnel monitor the patient to watch for outward signs of epileptic or other events, and review the recorded electrical activity signals to determine whether an event occurred, whether the event was epileptic in nature and, in some cases, the type of epilepsy and/or region(s) of the brain associated with the event. Because the electrodes are wired to the data acquisition unit, and because medical personnel must monitor the patient for outward clinical signs of epileptic or other events, the patient is typically confined to a small area (e.g., a hospital or clinical monitoring room) during the period of monitoring, which can last anywhere from several hours to several days. Moreover, where the number of electrodes placed on or under the patient's scalp is significant, the size of the corresponding wire bundle coupling the sensors to the data acquisition unit may be significant, which may generally require the patient to remain generally inactive during the period of monitoring, and may prevent the patient from undertaking normal activities that may be related to the onset of symptoms.

While ambulatory encephalograms (aEEGs) allow for longer-term monitoring of a patient outside of a clinical setting, aEEGs are typically less reliable than EEGs taken in the clinical setting, because clinical staff do not constantly monitor the patient for outward signs of epileptic events or check if the electrodes remain affixed to the scalp and, as a result, are less reliable when it comes to determining the difference between epileptic and non-epileptic events.

The use of EEG in the determination of whether an individual has epilepsy, the type of epilepsy, and its location (or foci) in the brain is fundamental in the diagnostic pathway of individuals suspected of epilepsy. Unfortunately, while the EEG offers a rich source of information relating to the disease, the EEG signal can suffer from a poor signal to noise ratio, is, for the most part, manually reviewed by trained clinical personnel, and the review is limited to a short period of monitoring, either in-patient, as described above, or ambulatory recordings, each being no more than seven days in duration. As a result of these limitations, the current diagnosis paradigm suffers from the following deficiencies: (1) the limited recording window (up to 7 days) may not be adequate to capture the clinical relevant events in the EEG due to the infrequency of the epileptic events; (2) clinical events thought to be epileptic may be confused for other, non-epileptic events, such as drug side-effects or psychogenic seizures that are of non-epileptic origin. The reporting of these clinical events is done via subjective patient feedback or paper/electronic seizure diaries. These have been demonstrated to be highly unreliable; (3) the lack of long-term monitoring of the patients after administration of the treatment (e.g., drugs) creates an ambiguity in the disease state of the individual. For example, many events reported subjectively by the patient may be either (a) epileptic, (b) drug side-effects, and/or (c) of non-epileptic origin. Proper treatment of the patient must be based on determining an objective and accurate characterization of the disease state across the care continuum of the patient; (4) inaccurate self-reporting of seizure incidence can result in over- or under-medicalization of the patient; and (5) human review of the multiple streams of data required to determine if each individual event is (a) epileptic, (b) caused by a drug side-effect; and/or (c) non-epileptic in origin is not possible because (i) the sheer volume of data requiring review when long-term monitoring is performed and (ii) the inability to extract patterns of behavior / biomarkers across multiple streams of data.

Diagnosing sleep disorders, such as sleep apnea, which may be episodic and/or intermittent in nature, presents similar challenges. Typically, sleep apnea is diagnosed following a sleep study in which a patient spends a night under observation by a sleep specialist who monitors the patient's breathing and other body functions while the patient sleeps. This monitoring can also include monitoring of electrical activity in the patient's brain (e.g., EEG). Unfortunately, being in an unfamiliar environment, an unfamiliar bed, and being tethered to a variety of sensors can interfere with the ability of the patient to sleep comfortably or normally, and can, therefore, sometimes affect the reliability of the resulting diagnosis.

Vestibular and cochlear disorders may be similarly episodic and/or intermittent in nature and, therefore, may present similar challenges in terms of diagnosis.

Importantly, the episodic and/or intermittent nature of these conditions makes it inherently difficult to predict when these conditions, or events caused by these conditions will occur, how frequently they will occur, how long they will last, and how and for how long they will affect the short- and long-term well-being of the patient experiencing them.

Further, treatment of these disorders is hardly an exact science. For example, the standard of care for an individual with either suspected or diagnosed epilepsy is to administer one or more anti-epileptic drugs (AEDs) in an effort to minimize or eliminate epileptic seizures in the individual. Typically, such drugs are administered in oral form and taken regularly (e.g., daily) at a dosage that is determined by the treating physician (e.g., neurologist). The specific dose and administration frequency that is effective for a particular patient is specific to the patient and is generally determined by titrating the dose until a perceived effective dose is determined.

One problem with this approach is that the on-going prescription of these AEDs is based on subjective reports by the patient on the perceived incidence and severity of the recurring seizures and drug side-effects. These subjective reports can vary in accuracy across individuals and may or may not be an accurate representation of the individual's state away from a clinic; for example, many types of epileptic seizures are extremely subtle, and the individual may not remember or recognize the seizure (e.g., absence seizures). Similarly, side effects from certain AEDs may be mistaken as seizures and reported as such to the treating physician. As a result of these deficiencies, AEDs are frequently administered or prescribed at sub-therapeutic levels (i.e., insufficient dose to control the condition), at super-therapeutic levels that induce side effects worse than the condition they may or may not control at those levels, or at therapeutic levels that nevertheless cause undesirable side-effects, even when a side-effect-free therapeutic level could be prescribed. Treatments using neurostimulator devices, such as vagal nerve stimulators, require similar experimentation with titration and timing in order to achieve a therapeutic level that is free of, or at least minimizes, side-effects. Systems and methods for monitoring biomarkers for neurologic diseases and managing corresponding treatments are known from US 2019/328306 A1 and US 2011/213222 A1.

Treatment regimens for other disorders including sleep apnea and cochlear and vestibular disorders may suffer from similar challenges when intervention is pharmacological or neurostimulatory in nature.

It is desirable to have a safe, reliable, and comfortable method of detecting the occurrence of epileptic seizures to enable monitoring of seizure frequency and severity with a view to diagnosing epilepsy and/or determining appropriate seizure control strategies.

It is desirable to have a safe, reliable, and comfortable method of determining the side-effect free therapeutic treatment regimen, whether of an oral medication, an intravenous medication (e.g., administered by portable pump), application of a neurostimulator device, or other treatments such as medications administered by inhalation.

It is also desirable to have a safe, reliable, and comfortable method of detecting, predicting, and classifying both the occurrence of these conditions and related events, and the effects, both immediate and future, of these events on the patient. It is still further desirable to treat these conditions appropriately in view of the effects on the patient and to do so using an optimized treatment regimen.

Any discussion of documents, acts, materials, devices, articles, or the like which has been included in the present background is not to be taken as an admission that any or all of these matters form part of the prior art base or were common general knowledge in the field relevant to the present disclosure as it existed before the priority date of each claim of this application.

### BRIEF DESCRIPTION OF THE FIGURES

Fig. 1A is a block diagram of an example system according to a first set of described embodiments.
Fig. 1B is a block diagram of an example system according to second and third sets of described embodiments.
Fig. 2A is a cross-sectional side view of an example electrode device.
Fig. 2B is a top view of the example electrode device of Fig. 2A.
Fig. 2C illustrates sub-scalp placement of the example electrode of Figs. 2A and 2B.
Figs. 3A and 3B show side and top views, respectively, of another example electrode device.
Figs. 3C through 3E show cross-sectional views of portions of the electrode device of Figs. 3A and 3B.
Figs. 3F and 3G show top and side views, respectively, of a distal end portion of the electrode device of Figs. 3A and 3B.
Fig. 3H illustrates an example implantation location of electrodes of an electrode device.
Fig. 3I illustrates an example implantation location of an electrode device.
Fig. 4 depicts an example sensor array including a plurality of electrodes and a local processing device.
Fig. 5A is a block diagram depicting an electrode assembly including a local processing device.
Fig. 5B is a block diagram depicting a PPG sensor assembly including a local processing device.
Fig. 6A is a block diagram of an embodiment including a sensor array, a microphone, and an accelerometer.
Fig. 6B is a block diagram of an embodiment including a sensor array, a microphone, an accelerometer, and a PPG sensor.
Fig. 6C is a block diagram of an embodiment including a sensor array and a PPG sensor.
Fig. 7A is a block diagram of an embodiment including a sensor array and a microphone.
Fig. 7B is a block diagram of an embodiment including a sensor array, a microphone, and a PPG sensor.
Fig. 8A is a block diagram of an embodiment including a sensor array and an accelerometer.
Fig. 8B is a block diagram of an embodiment including a sensor array, an accelerometer, and a PPG sensor.
Fig. 9A is a block diagram of an embodiment including a sensor array with a biochemical transducer and, optionally, a microphone and/or an accelerometer.
Fig. 9B is a block diagram of an embodiment including a sensor array with a biochemical transducer, a PPG sensor and, optionally, a microphone and/or an accelerometer.
Figs. 10A-13B correspond generally to the figures 6A-9B, but illustrate that the embodiments thereof can implement a trained AI model instead of a static model.
Figs. 13C-13E are block diagrams depicting embodiments in which evaluative functions take place on an external device, rather than on a local processor device.
Figs. 14A and 14B are block diagrams depicting example systems for use in creating a trained AI model.
Figs. 15A and 15B are block diagrams depicting example systems for collecting second sets of training data for creating the trained Al model.
Figs. 16A and 16B depict embodiments of a first set of AI training data.
Figs. 16C and 16D depict embodiments of a second set of AI training data.
Figs. 17A-17F depict various embodiments of classification results that may be output by the various embodiments described herein.
Fig. 18A is a block diagram depicting an example system for use in creating a trained AI model according to another embodiment.
Fig. 18B is a block diagram depicting an example system for collecting a second set of training data for creating the trained AI model according to the embodiment of Fig. 18A.
Fig. 18C depicts an embodiment of a first set of AI training data according to the embodiments depicted in Figs. 18A and 18B.
Figs. 18D depicts an embodiment of a second set of AI training data according to the embodiments depicted in Figs. 18A and 18B.
Figs. 18E-18G depict various embodiments of classification results that may be output by the various embodiments described with respect to Figs. 18A-18B.
Fig. 19A depicts an example method for creating a trained AI model according to disclosed embodiments.
Figs. 19B and 19C depict example methods for using a static model or a trained AI model to classify events in various embodiments.
Figs. 20A-20H are block diagrams depicting various embodiments of sensor arrays according to the disclosed embodiments.
Figs. 21A-21E are block diagrams depicting various embodiments of processor devices according to the disclosed embodiments.
Figs. 22A-22G are block diagrams depicting various embodiments of combinations of sensors/sensor arrays with processor devices according to the disclosed embodiments.
Figs. 23A-23B are block diagrams depicting respective embodiments of communication between a sensor array and a processor device.
Figs. 24A-24C are block diagrams depicting various communication schemes between the processor device and external equipment according to the disclosed embodiments.
Figs. 25A-25D are block diagrams depicting various communication schemes between the processor device and treatment equipment according to the disclosed embodiments.
Fig. 26 illustrates the general concept of a therapeutic treatment window.
Fig. 27 is a block diagram depicting a treatment window routine.
Figs. 28-30B are flow charts depicting example algorithms for adjusting a treatment according to the classification results according to various embodiments.
Fig. 31 depicts an example method for adjusting a treatment according to the classification results.

### DETAILED DESCRIPTION

The present invention is directed to a system as defined in appended claim 1. Preferred embodiments of the invention are defined in the dependent claims. No methods fall within the scope of the claims by themselves. Embodiments of the present disclosure relate to the monitoring and classification of electrical activity ir body tissue of a subject using an array of sensors disposed on or in the patient's body, in cooperation with computer algorithms programmed to detect and classify events of interest. Certain embodiments relate, for example, to electrode arrays implanted in a head of a subject to monitor brain activity such as epileptic brain activity, and coupled to processor devices configured to monitor and classify the brain activity to determine when events occur and/or whether any particular type of event is an epileptic event and/or what type of event has occurred, if not an epileptic event. However, the sensor arrays according to the present disclosure may be for implanting in a variety of different locations of the body, may sense electrical signals, including those generated by electrochemical sensors, and may cooperate with processing devices in various instances in which monitoring and classification of electrical or chemical activity is desired in the human nervous system.

Other embodiments of the present disclosure relate to the monitoring and classification of biomarkers in body tissue of a subject using an array of sensors disposed on or in the patient's body, in cooperation with computer algorithms programmed to detect, predict, and/or classify events of interest, monitor and adjust treatment protocols to determine the presence and absence of side-effects and therapeutic effect of the treatment protocols, and apply the treatment protocols according to detected and/or predicted events to mitigate or treat the effects of the events of interest. Certain embodiments relate, for example, to electrode arrays (e.g., electroencephalograph (EEG) sensors) implanted in a head of a subject to monitor brain activity that may be indicative of epileptic brain activity, auditory and vestibular system function, and other activity that may relate to conditions and disorders. The electrode arrays and other sensors, including photoplethysmography sensors (referred to herein for convenience as "PPG sensors"), may be coupled to processor devices configured to monitor and classify the brain activity to determine when events occur and/or whether any particular type of event is, for example, an epileptic event and/or what type of event has occurred, if not an epileptic event. However, the sensor arrays according to the present disclosure may be for implanting in a variety of different locations of the body, may sense other electrical signals, and may cooperate with processing devices in various instances in which monitoring and classification of electrical activity is desired in the human nervous, auditory, and pulmonary systems.

Various aspects of the systems and methods are described throughout this specification. Unless otherwise specified, aspects of any embodiment that are compatible with another embodiment described herein are considered as contemplated and disclosed embodiments herein. For example, a feature of a particular embodiment described herein, if that feature would be recognized by a person of ordinary skill in the art to be compatible with the features of a second embodiment described herein, should be considered as a possible feature of the second embodiment. Further, embodiments describing features as optional should be considered as disclosing said embodiments both with and without the optional features, and with various optional features in any combination that, in view of this description, would be recognized by a person of ordinary skill in the art as being compatible.

Throughout the present disclosure, embodiments are described in which various elements are optional - present in some, but not all, embodiments of the system. Where such elements are depicted in the accompanying figures and, specifically, in figures depicting block diagrams, the optional elements are generally depicted in dotted lines to denote their optional nature.

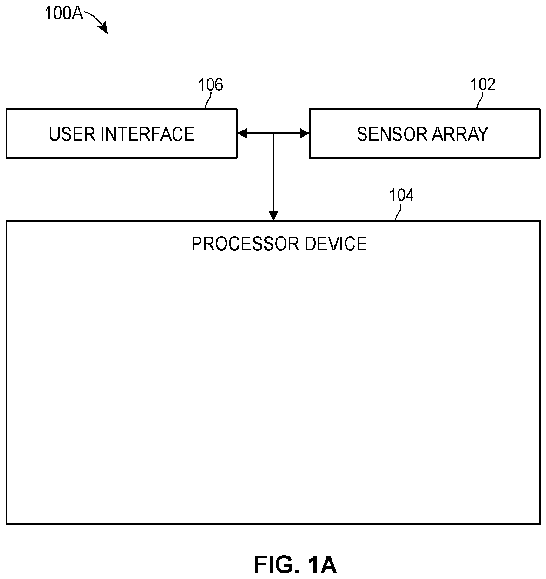

Fig. 1A depicts, in its simplest form, a block diagram of a contemplated system 100A ("first set of embodiments") directed to classification of neurological events. The system 100 includes a sensor array 102, a processor device 104, and a user interface 106. The sensor array 102 generally provides data, in the form of electrical signals, to the processor device 104, which receives the signals and uses the signals to detect and classify events in the electrical signal data. The user interface 106 may facilitate self-reporting by the patient of any of various data including events perceived by the patient, as well as medication types, doses, dose times, patient mood, potentially relevant environmental data, and the like. The user interface 106 may also facilitate output of classification results, programming of the unit for a particular patient, calibration of the sensor array 102, etc.

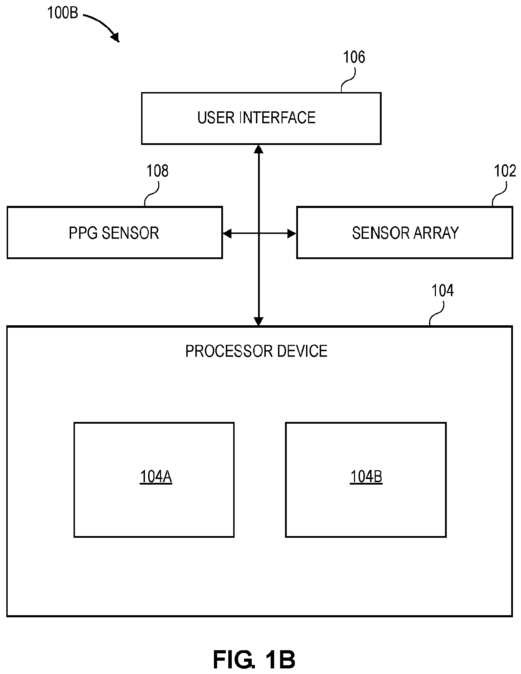

Fig. 1B depicts, in its simplest form, a block diagram of a contemplated system 100B for a variety of additional embodiments directed to determining a therapeutic window of a treatment, and detecting and classifying neuroelectrical, vestibular, cochlear, cardiac, and pulmonary events and conditions occurring in the subject, and using the detection and classification, combined with the determined therapeutic window to optimize treatment. Generally speaking, the systems and methods described with reference to Fig. 1B include two sub-systems 104A, 104B, which may be employed individually or together and, as will become apparent, are complementary to one another. Broadly speaking, these systems and methods are directed to improving the overall wellness of patients experiencing conditions related to epilepsy, cochlear disorders, vestibular disorders, and sleep/or disorders. These conditions affect the patients in a variety of manners that are directly and indirectly related to the associated events and symptoms experienced by the patients. As but one example, while epilepsy may outwardly manifest itself by a series of seizure events, those seizures may have associated effects on the patient's well-being related to blood pressure, blood oxygen saturation, heart rate, heart rate variability, cardiac output, respiration, and other metabolic, neurological, and/or cardio-pulmonary functions.

One set of embodiments of a sub-system described herein is directed to detecting and categorizing various events (e.g., seizures, apnea events, etc.) and symptoms (changes in blood pressure, heart rate, blood oxygen saturation, etc.) as clinical events, sub-clinical events, and/or side-effects of treatment. By way of example, and not limitation, the sub-system, using a static or trained AI model may determine, using EEG data and photoplethysmography data (PPG data), in addition, in embodiments, to microphone and/or accelerometer data, that a patient has just experienced or is experiencing a generalized tonic-clonic (i.e., grand mal) seizure.

Another set of embodiments of the sub-system described herein is directed to measuring, tracking, and predicting both the events (e.g., seizures, apnea events, etc.) and the well-being of the patients before, during, and after the events, and recommending or administering treatments to alleviate or mitigate the effects on the patient that are associated with those events. By way of example, and not limitation, the sub-system, using a static or trained AI model may determine, using EEG data and PPG data, that a patient has just experienced, is experiencing, or will experience (i.e., the system may predict) a generalized tonic-clonic (i.e., grand mal) seizure. The sub-system may also determine that the patient experiences or is likely to experience hypoxia during generalized tonic-clonic seizures, leading to generalized or specific symptoms of hypoxia that are the direct result of the seizures such as fatigue, numbness, nausea, etc. As such, the sub-system may recommend that oxygen be provided to the patient to address the hypoxia and, thereby, improve the overall well-being of the patient, and decrease the recovery time after the seizure. As will become apparent, the sub-system may make recommendations to the patient, to a care giver, to a physician, etc., or may adjust a treatment device (e.g., a neurostiumulator device, a drug pump, etc.) depending on the conditions to be treated, the events that are detected, and the patient's past experience, as reported both by the patient and by the computational analyses of the data from the EEG and PPG sensors.

A second sub-system described herein is directed to determining and optimizing a therapeutic window for treating the condition in question, whether that condition is epilepsy, a vestibular or cochlear disorder, a sleep disorder, such as apnea, or the like. The second sub-system may monitor for changes in various biomarkers over time and/or during specific time periods to determine whether a pharmacological intervention or other treatment for a condition is having a positive effect on the condition (e.g., lessening severity or frequency of events), is having a negative effect on the condition (e.g., increasing severity or frequency of events), is causing side-effects, or is having no effect at all. The second sub-system, as a result of these analyses, may recommend or implement a change in the dose or timing of the pharmacological intervention, a change in the intensity, timing, or other parameters of a neurostimulator application (such as vagal nerve stimulators, epicranial stimulation, etc.), or other changes to a treatment device or regimen according to the particular condition being treated. In doing so, the sub-system may continue to monitor the patient to iteratively determine a "treatment window" that has maximal benefit to the patient, while minimizing or eliminating some or all side-effects. As will be apparent in view of the description below, the patient (e.g., via a user interface) or a physician or clinician (e.g., via an external device) may adjust the target therapeutic effect within the treatment window to arrive at the desired balance between absence of symptoms and presence of side-effects. For example, in patients with epilepsy, it is common that the patient would like to have their seizures minimized, even at the expense of the side-effects. That is, the patient may be happy to live with the side-effects of treatment, if it allows them to be seizure-free.

Of course, it will become apparent that these two sub-systems may be deployed cooperatively such that a treatment for the condition can be optimized while monitoring, detecting, and predicting both the onset of clinical events and the ancillary effects of those clinical events, and mitigating or treating the ancillary effects of those clinical events. For example, the second sub-system may be used to optimize a patient's treatment for epilepsy by finding an optimal treatment regimen to minimize (or optimize) the severity and/or frequency of seizure events while minimizing (or optimizing) any side-effects of the treatment regimen. That is, it is not necessary to minimize the events or the side-effects, but rather, in some implementations the goal may be to maximize patient well-being even if events and/or side-effects remain higher than the possible minimum. In another example, the second sub-system may be used to optimize a patient's treatment for epilepsy by finding an optimal treatment regimen to minimize the severity and/or frequency of seizure events and, thereafter, the first sub-system may be used to detect or predict seizure events that still occur, to determine or predict measures of patient well-being as a result of those seizure events, and/or to recommend or implement therapeutic interventions to mitigate those effects and/or support the well-being of the patient in view of those effects. In another example, the first sub-system may be used to detect seizure events, to determine measures of patient well-being as a result of those seizure events. The second sub-system may be used to try to reduce the overall severity and frequency of those events, while concurrently addressing potential side-effects, by optimizing the patient's treatment regimen. In another example, the first sub-system may be used to detect or predict seizure events, to determine or predict measures of patient well-being as a result of those seizure events, and/or to recommend or implement therapeutic interventions to mitigate those effects and/or support the well-being of the patient in view of those effects. Once support of the patient in view of seizure events that are occurring and/or predicted is achieved, the second sub-system may be used to try to reduce the overall severity and frequency of those events by optimizing the patient's treatment regimen. Of course, there is no requirement that the two sub-systems be used sequentially, as it should be apparent from the present description that the two sub-systems may operate concurrently and/or iteratively to achieve their respective objectives.

Moreover, the first sub-system 104A may adapt and/or retrain itself to recognize patient-specific patterns in the biomarkers that may be either related to the patient's condition and symptoms (e.g., related to the patient's epilepsy), or caused by the second sub-system 104B being active and changing the behavior of the patient's condition and symptoms (e.g., via the applied therapy).

While described herein primarily with respect to epilepsy, it will be clear from the description that the systems and methods herein, especially with respect to embodiments related to Fig. 1B, can be used with and applied to other conditions, as well. That is, the biomarkers that can be sensed and monitored by the EEG and PPG sensors may be used to monitor, detect, and/or predict events related to other conditions, to support patient well-being in view of the effects of those events and conditions, and/or may be used to optimize a treatment regimen for those conditions. Together, EEG and PPG sensors and, in embodiments, microphones and/or accelerometers, may provide data from which the biomarker data related to the patient(s) may be extracted. As used herein, the term "biomarker" refers to a broad subcategory of objective indications of medical state, observable from outside the patient, that can be measured accurately and reproducibly. (Biomarkers differ from symptoms, which are generally perceived by patients themselves.)

Various signals detectable within EEG data may signal an ictal event, as specific patterns of electrical activity in various regions of the brain are associated with the onset, duration, and offset of a seizure event. Such biomarker patterns are referred to as epileptiforms. Additionally, shorter duration biomarkers including "spikes," having durations between 30 and 80 ms, and "sharps," having durations between 70 and 200 ms, may occur between seizures. The various biomarkers associated with ictal activity may be indicative of the types of seizures occurring. For example, absence seizures are frequently associated with generalized "spike" activity, though spike activity is not exclusive to absence seizures. Features of epileptiforms may signal additional biomarkers, and interictal (between seizure), pre-ictal, and post-ictal EEG data may provide additional biomarker information related to detection and/or prediction of seizures. At the same time, PPG data may include biomarker data related to interictal, pre-ictal, post-ictal (and ictal) state of the patient. For instance, oxygen desaturation is known to occur in a significant portion of focal seizures, including those without convulsive activity, before, during, or after a seizure. Similarly, changes in blood pressure, heart rate, or heart rate variability - all detectable within PPG data - can occur before, during, or after a seizure event. By observing EEG data and PPG data concurrently, over periods of time, additional relationships between biomarkers in EEG data and PPG data can reveal relationships and patterns that facilitate the detection and, perhaps more importantly, prediction of ictal events, and, in some embodiments establish biomarkers relating to drug side-effects and quality of life metrics that may relate to the long-term use of the applied therapeutic treatment(s). For example, it may be desirable to minimize compromised sleep for individuals with epilepsy taking drugs to treat their disease, as many of the anti-epileptic drugs negatively impact sleep quality if taken excessively or at the wrong times of day. Other biomarkers may, in embodiments, be detected from microphone and/or accelerometer data, as will become clear from the following description.

Biomarkers present in EEG data and PPG data may be telling, for example, with respect to sleep disorders. EEG data can provide information about a variety of biomarkers related to sleep disorders, including, by way of example and not limitation, the stage of sleep that a patient is in, how frequently the patient changes from one stage of sleep to another, transitions from one stage of sleep to another, EEG spindle magnitude, EEG spindle duration, EEG spindle frequency, EEG spindle prevalence, and EEG desaturation events. At the same time, PPG data can provide information regarding a variety of biomarkers relevant to events related to sleep disorders and, especially, sleep apnea. Sleep apnea is the repetitive pausing of breathing occur more than normal. As such, this compromised respiration can affect a number of the biomarkers that are detectable from PPG data such as heart rate, heart rate variability, blood pressure, respiration rate, and blood oxygen saturation, some or all of which may be associated with desaturation events related to compromised respiration. Other biomarkers may, in embodiments, be detected from microphone and/or accelerometer data, as will become clear from the following description.

Similarly, biomarkers present in EEG data and PPG data may be indicative of cochlear and/or vestibular disorders. EEG data can provide information about biomarkers related to these disorders and, in particular, biomarkers such as hearing thresholds, cognitive effort, and hearing perception. PPG data, meanwhile, can provide information about systemic infections that may propagate to the cochlear or vestibular system by, for example, detecting the changes in respiration, blood oxygen saturation levels, heart rate variability, and blood pressure biomarkers that can indicate systemic infections. PPG data may also provide direct evidence of vestibular system dysfunction, as dysfunction in the vestibular system can be accompanied by a change (i.e., a drop) in the patient's blood pressure. Other biomarkers may, in embodiments, be detected from microphone and/or accelerometer data, as will become clear from the following description.

Fig. 1B depicts, in its simplest form, a block diagram of the contemplated systems 100B according to the second set of embodiments. The system 100B includes a sensor array 102 (e.g., an EEG sensor array with or without one or more accelerometers and/or microphones), a processor device 104, a user interface 106, and a PPG sensor 108. The sensor array 102 and the PPG sensor 108 generally provide respective data, in the form of electrical signals, to the processor device 104, which receives the signals and uses the signals to detect and classify events according to biomarkers in the electrical signal data received from the sensor array 102 and the PPG sensor 108. The user interface 106 may facilitate self-reporting by the patient of any of various data including events perceived by the patient or caregivers, as well as medication types, doses, dose times, patient mood, potentially relevant environmental data, and the like. The user interface 106 may also facilitate output of classification results, programming of the unit for a particular patient, calibration of the sensor array 102 or the PPG sensor 108, etc.

The processor device 104 in system 100B is depicted as including the first and second sub-systems, 104A and 104B, respectively. While depicted in Fig. 1B as separate blocks 104A and 104B, the first and second sub-systems 104A and 104B are depicted as separate blocks only to illustrate that they may be implemented independently, using the same PPG sensor(s) 108, sensor array 102, and user interface hardware 106. Of course, the sub-systems 104A and 104B may share some or all of the hardware resources (e.g., processor, memory, communications circuitry, etc.) in the processor device 104 and may even share certain elements of the software or routines used therein (e.g., user interface routines or portions thereof, communications routines, data pre-processing routines, feature identification routines, etc.). While the first sub-system 104A may be implemented in separate sets of hardware (e.g., separate PPG sensors 108, separate sensor arrays 102, separate processor devices 104, separate user interfaces 106), in implementations in which the patient will interact with both sub-systems 104A, 104B, either sequentially or concurrently, it is contemplated that the patient will not be burdened with two separate sets of sensor arrays 102, two PPG sensors 108, and two processor devices 104. At most, in implementations in which the patient interacts with the first and second sub-systems 104a, 104b sequentially (first then second, or second then first), the patient may utilize a separate processor device during each period of interaction, connecting the two different processor devices 104 (and perhaps user interfaces 106 integrated therein) to the sensor array 102 and the PPG sensor 108. Of course, in preferred embodiments, the same physical (i.e., hardware) processor device 104 may implement two different applications, or two different routines within the same application, to implement each of the two sub-systems.

The following description of the sensor array 102 is illustrative in nature. While one of skill in the art would recognize a variety of sensor arrays that may be compatible with the described embodiments, the sensor arrays 102 explicitly described herein may have particular advantages and, in particular, the sensor arrays 102 may include the sensors described in U.S. Patent Application 16/124,152 (U.S. Patent Application Publication No. 2019/0053730 A1) and U.S. Patent Application 16/124,148 (U.S. Patent No. 10,568,574).

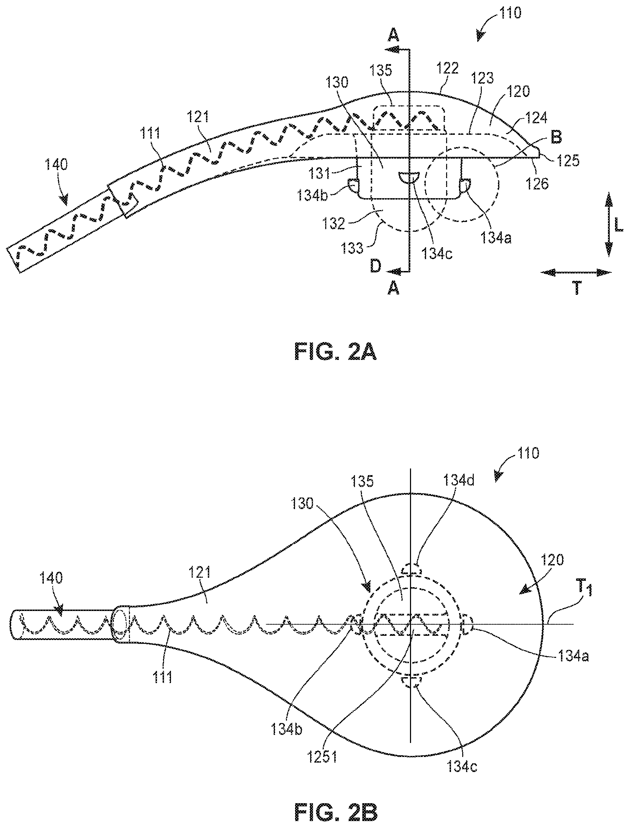

With reference to Figs. 2A and 2B, in one embodiment an electrode device 110 is provided including a head 120 and a shaft 130, the shaft 130 being connected to the head 120. The shaft 130 includes a shaft body 131, a conductive element 132 and a plurality of discrete anchor elements 134a-134d. The shaft 130 extends distally from the head 120 in an axial direction L of the shaft 130. The conductive element 132 has a conductive surface 133 at a distal end D of the shaft 130. The elements 134a-d project from an outer surface of the shaft body 131 in a transverse direction T of the shaft that is perpendicular to the axial direction L. The electrode device 110 also includes a lead 140 to provide electrical connection to the electrode device 110. The electrode device includes a conductive wire 111 extending through the lead 140 and the head 120, the conductive wire 111 being electrically connected to the conductive element 132. In alternative embodiments, the electrode device may comprise a port for connecting to a separate lead.

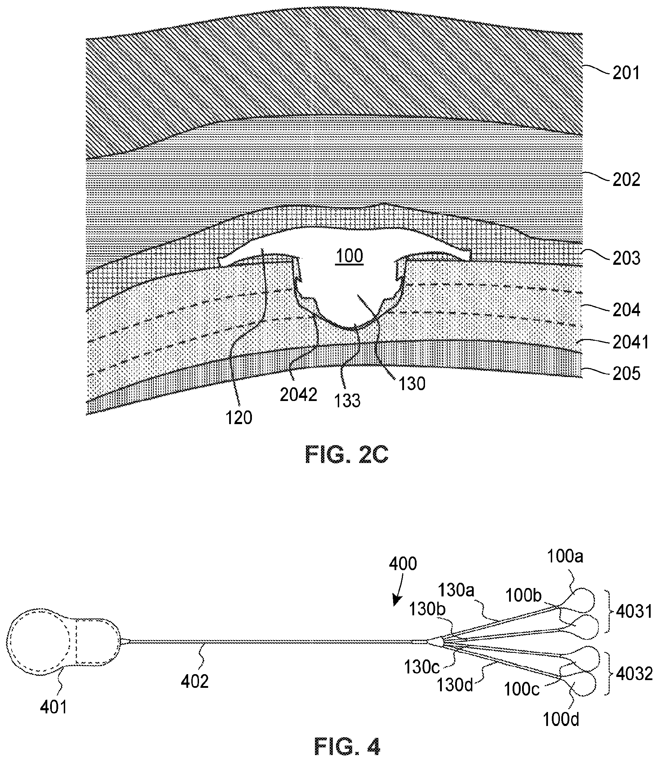

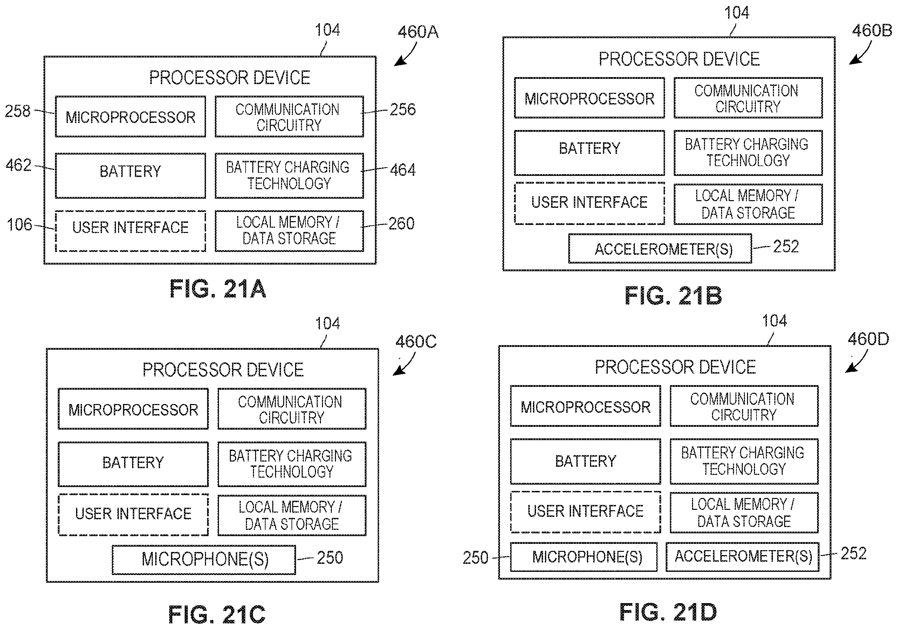

With reference to Fig. 2C, the electrode device 110 is configured to be at least partially implanted at a cranium 204 of a subject, and specifically such the shaft 130 projects into a recess 2042 formed in the cranium 204. The recess 2042 can be a burr hole, for example, which may be drilled and/or reamed into the cranium 204, e.g., to the depth of the lower table, without being exposed to the dura mater 205. Fig. 2C illustrates the positioning of the device 110 relative to various tissue layers adjacent to the cranium 204. The tissue layers illustrated include: skin 201; connective tissue 202; pericranium 203; cranium (bone) 204, including the lower table 2041 of the cranium 204; and the dura mater 205. As can be seen, substantially the entire axial dimension of the shaft 130 of the electrode device 110 extends into the recess 2042 while at least a rim at an outer edge of the head 120 abuts the outer surface of the cranium 204, in a pocket underneath the pericranium 203. The conductive surface 133 at the distal end D of the shaft 130 is positioned in the lower table 2041 of the cranium 204 such that it can receive electrical brain signals originating from the brain and/or apply electrical stimulation signals to the brain.

The electrode device 110 includes a number of features to assist in removably securing the shaft 130 at least partially in the recess 2042 in the cranium 204 (or a recess in any other bone or tissue structure where electrical monitoring and/or stimulation may be carried out). These features include, among other things, the anchor elements 134a-d. The anchor elements 134a-d are generally in the form of flexible and/or compressible lugs or barbs, which are configured to distort as the shaft 130 is inserted into the recess 2042 such that the anchor elements 134a-d press firmly against and grip the inner surfaces defining the recess 2042.

In this embodiment, referring to Figs. 2A and 2B, the plurality of discrete anchor elements 134a-d include four spaced apart anchor elements 134a-d that are evenly distributed around a circumference of the outer surface of the shaft body 131 but which are in an offset or staggered arrangement in the axial direction L of the shaft body. Thus, some anchor elements 134a, 134b are located, in the axial direction L, closer to the distal end D of the shaft 120 than other anchor elements 124c, 124d. More specifically, in this embodiment, a first pair of the anchor elements 124a, 124b is located, in the axial direction L, at a first distance from the distal end D of the shaft, and a second pair of the anchor elements 134c, 134d is located, in the axial direction L, at a second distance from the distal end D of the shaft, the second distance being greater than the first distance. This arrangement of anchor elements 134a-d ensures that at least one of the pairs of anchor elements 134a-d is in contact with the inner surface of the recess 2042 and can allow for easier insertion of the shaft into the recess 2042. With reference to Fig. 2B, the anchor elements 134a, 134b of the first pair are located on opposite sides of the shaft body 131 along a first transverse axis T1 of the shaft 130 and the anchor elements 134c, 134d of the second pair are located on opposite sides of the shaft body 131 along a second transverse axis T2 of the shaft 130, the first and second transverse axes T1 , T2 being substantially orthogonal to each other.

The shaft body 131 is formed of a first material, the first material being an elastomeric material and more specifically a first silicone material in embodiments. The anchor elements 134a-d are formed of a second material, the second material being an elastomeric material and more specifically a second silicone material in embodiments. The first and second materials have different properties. In particular, the second material has a lower durometer than the first material. Accordingly, the second material is softer than the first material and thus the anchor elements 134a-d are formed of softer material than the shaft body 131. By forming the shaft body 131 of a relatively hard elastomeric material, the shaft body can be flexible and compressible, yet still substantially retain its shape on insertion into the recess 2042 in the bone. The stiffening core provided by the conductive element 132 also assists in this regard. On the other hand, by forming the anchor elements 134a-d of a relatively soft elastomeric material, the anchor elements are more flexible and compressible, which can allow easier removal of the shaft 130 from the recess 2042 after use of the electrode device 110. Indeed, the soft material may be provided such that anchor elements 134a-d distort significantly upon removal of the shaft 130 from the recess 2042.

The anchor elements 134a-d are configured to remain intact during removal of the shaft 130 from the recess 2042. Thus, no part of the electrode device may be left behind in the body after removal. The anchor elements 134a-d remain connected to the outer surface of the shaft body 131 during and after removal. Further, the anchor elements substantially retain their original shape and configuration after removal of the electrode device from the recess 2042.

As discussed above, the electrode device 110 includes a lead 140 that is connected to the head 120 of the electrode device 110, a conductive wire 111 extending through the lead 140 and the head 120, and electrically connecting to the conductive element 132. With reference to Fig. 2A, the conductive wire 111 is helically arranged such that it can extend and contract upon flexing of the electrode device including the lead 140 and the head 120. The conductive wire 111 contacts and electrically connects to a proximal end surface 135 of the conductive element 132. The conductive wire 111 is permanently fixed to the proximal end surface 135, e.g. by being welded or soldered to the proximal end surface 135. In this embodiment, the proximal end surface 135 of the conductive element 132 is located inside the head 120 of the conductive device 110. The proximal end surface 135 of the conductive element includes a recess 1251 in which the conductive wire 111 contacts and electrically connects to the proximal end surface 135. The recess 1251 is a channel in this embodiment, which extends across an entire diameter of the proximal end surface 135. The recess 1251 can retain molten material during the welding or soldering of the conductive wire 111 to the proximal end surface 135. Moreover, material forming the head 120 of the electrode device can extend into the channel, e.g. while in a fluid state during manufacture, helping to secure the conductive element 132 in position and helping to protect the connection between the conductive wire 111 and the conductive element 132.

In this embodiment, in addition to the shaft body 131 being integrally formed, in one-piece, with the head 120, the lead 140 is also integrally formed, in one-piece, with the head 120. A continuous body of elastomeric material is therefore provided in the electrode device 110, which continuous body of elastomeric material extends across the lead 140, the head 120 and the shaft body 130. The continuous body of elastomeric material covers the conductive wire 111 within the lead 140 and the head 120, covers the proximal end surface 135 of the conductive element 132 within the head 120 and surrounds sides of the conductive element 132 of the shaft 130. The arrangement is such that the lead 140, head 120 and shaft 130 are permanently fixed together and cannot be disconnected during normal use. Following manufacture, no parts of the electrode device 110 may need to be connected together by a user such as a surgeon. The one-piece nature of the electrode device 110 may increase strength and cleanliness of the electrode device 110 and may also improve ease of use.

Referring to Fig. 2A, the lead 140 is connected to the head 120 of the electrode device 110 at a strain relief portion 121 of the head 120. The strain relief portion 121 is a tapered section of the head 120 that provides a relatively smooth transition from the head 120 to the lead 140. Specifically, the stain relief portion 121 is a portion of the head 120 that tapers in width, generally across a transverse plane of electrode device, to a connection with the lead 140. As evident from Fig. 2B, the head 120, including the strain relief portion 121, has a tear-drop shape.

The strain relief portion 121 is curved. The curvature is provided to match a curvature of the cranium 204 such that a reduced pressure, or no pressure, is applied by the strain relief portion 121 to the skull when the electrode device is implanted in position.

As can be seen in Fig. 2A, the head 120 has a convex outer (proximal-facing) surface 122 and a concave inner (distally-facing) surface 123. An outer portion 124 of the head 120 that extends radially outwardly of the shaft body 131, to an outer edge 125 of the head 120, curves distally as it extends towards the outer edge 125. Nevertheless, at the outer edge 125, the head 120 includes a flattened, rim portion 126 to provide a surface for atraumatic abutment and sealing with tissue. The outer portion 124 of the head 120 is resiliently flexible. Due to its resilient flexibility and curved shape, the outer portion 124 of the head 120 can act as a spring to place a tension on the anchor elements 134a-d when the shaft 130 is in the recess 2042. In general, the curved head arrangement may conform to curvature of tissue, e.g. the skull, at which the electrode device 110 is located and may enable tissue layers to slide over its outer surface 122 without significant adhesion. The rim portion 126 of the head 120 may seal around the recess 2042 in which the shaft 130 is located. The seal may reduce electrical leakage through tissue and reduce tissue growing under the head 110. The flexible outer portion 126 of the head 120 may also flex in a manner that enables the shaft 130 to reach into recess to a range of depths.

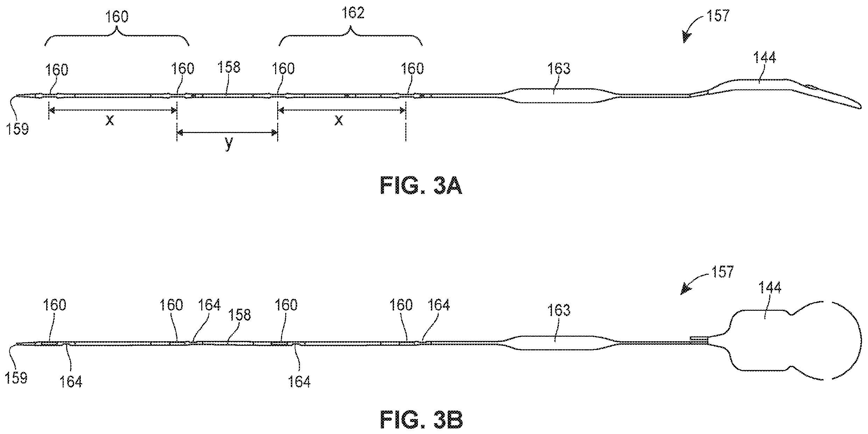

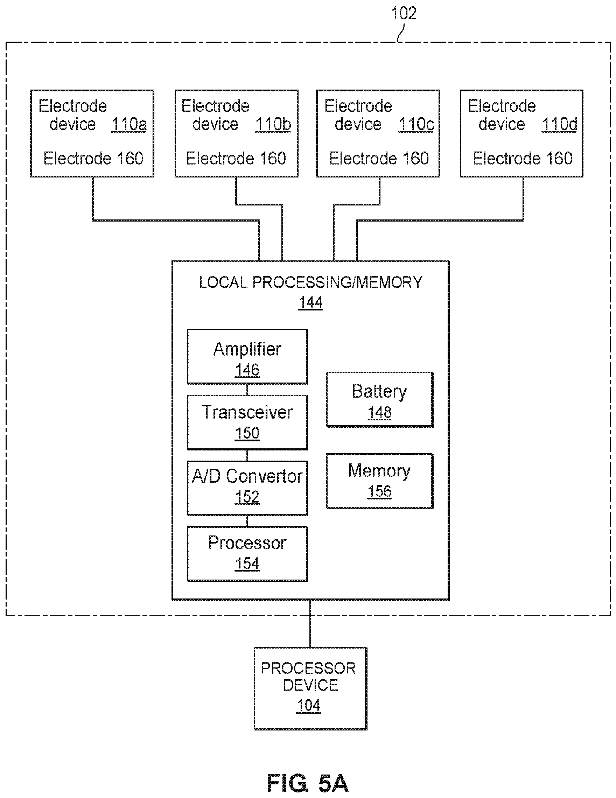

Figs. 3A through 3I illustrate an alternative embodiment of a sensor array 102, such as that described in U.S. Patent Application No. 16/797,315, entitled "Electrode Device for Monitoring and/or Stimulating Activity in a Subject,'. With reference to Figs. 3A and 3B, in one embodiment an electrode device 157 is provided comprising an elongate, implantable body 158 and a plurality of electrodes 160 positioned along the implantable body 158 in the length direction of the implantable body 158. At a proximal end of the implantable body 158, a processing unit 144 is provided for processing electrical signals that can be sent to and/or received from the electrodes 160. Though not required, in some embodiments, an electrical amplifier 163 (e.g., a pre-amp) is positioned in the implantable body 158 between the electrodes 160 and the processing unit 144. In an alternative embodiment, as illustrated in Fig. 5A, the electrical amplifier 163 may be integrated into the processing unit 144 of the electrode device 157, instead of being positioned in the implantable body 158.

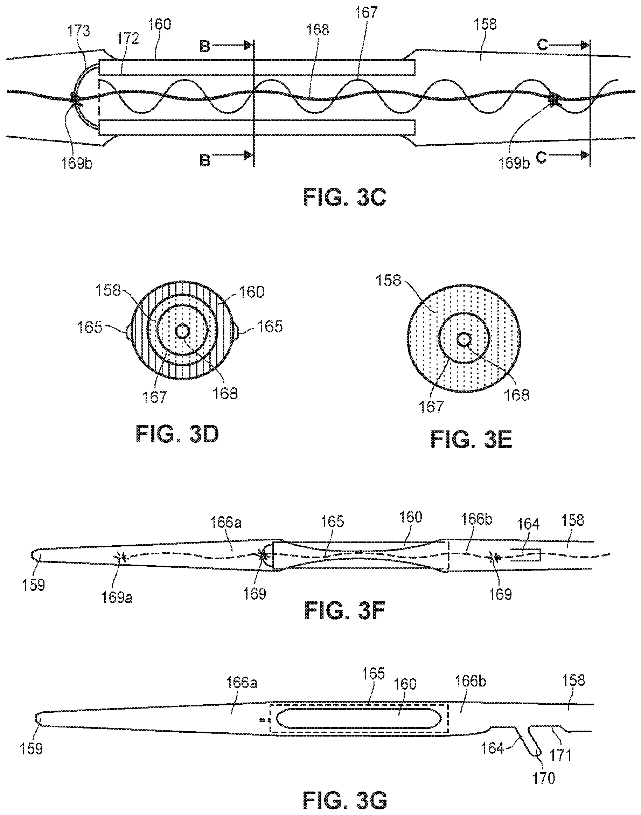

With reference to Fig. 3C, which shows a cross-section of a portion of the electrode device 157 adjacent one of the electrodes 160, the electrodes 160 are electrically connected, e.g., to the amplifier 163 and processing unit 144, by an electrical connection 167 that extends through the implantable body 158. A reinforcement device 168 is also provided in the electrode device 157, which reinforcement device 168 extends through the implantable body 158 and limits the degree by which the length of the implantable body 158 can extend under tension.

In this embodiment, referring to Figs. 3A and 3B, four electrodes 160 are provided that are spaced along the implantable body 158 between the amplifier 163 and a distal tip 159 of the implantable body 158. The distal tip 159 of the implantable body 158 is tapered. The four electrodes 160 are configured into two electrical pairs 161, 162 of electrodes, the two most distal electrodes 160 providing a first pair of electrodes 161 and the two most proximal electrodes 160 providing a second pair of electrodes 162. In this embodiment, the electrodes 160 of the first pair 160 are spaced from each other at a distance x of about 40 to 60mm, e.g., about 50 mm (measured from center-to-center of the electrodes 160) and the electrodes 160 of the second pair 122 are also spaced from each other at a distance x of about 40 to 60mm, e.g., about 50 mm (measured from center-to-center of the electrodes 160). The first and second electrode pairs 161, 162 are spaced from each other at a distance y of about 30 to 50 mm, e.g., about 40 mm (measured from center-to-center of the electrodes of the two pairs that are adjacent each other).

With reference to Figs. 3D and 3E, which provide cross-sectional views along lines B--B and C--C in Fig. 3C, respectively, the implantable body 158 has a round, e.g., substantially circular or ovate, cross-sectional profile. Similarly, each of the electrodes 160 has a round, e.g., substantially circular or ovate, cross sectional profile. Each of the electrodes 160 extend circumferentially, completely around a portion of the implantable body 158. By configuring the implantable body 158 and electrodes 160 in this manner, the exact orientation of the implantable body 158 and electrodes 160, when implanted in a subject, is less critical. For example, the electrodes 160 may interact electrically with tissue in substantially any direction. In this regard, the electrodes 160 may be considered to have a 360 degree functionality. The round cross-sectional configuration can also provide for easier insertion of the implantable portions of the electrode device 157 to the target location and with less risk of damaging body tissue. For example, the implantable body 158 can be used with insertion cannulas or sleeves and may have no sharp edges that might otherwise cause trauma to tissue.

In this embodiment, the implantable body 158 is formed of an elastomeric material such as medical grade silicone. Each electrode 160 comprises an annular portion of conductive material that extends circumferentially around a portion of the implantable body 158. More specifically, each electrode 160 comprises a hollow cylinder of conductive material that extends circumferentially around a portion of the implantable body 158 and, in particular, a portion of the elastomeric material of the implantable body 158. The electrodes 160 may be considered 'ring' electrodes.

Referring back to the embodiment of Figs. 3A and 3B, and with further reference to Figs. 3F and 3G, to strengthen the engagement between the electrodes 160 and the implantable body 158, straps 165 are provided in this embodiment that extend across an outer surface of each electrode 160. In this embodiment, two straps 165 are located on substantially opposite sides of each electrode 160 in a direction perpendicular to the direction of elongation of the implantable body 158. The straps 165 are connected between sections 166a, 166b of the implantable body 158 that are located on opposite sides of the electrodes 160 in the direction of elongation of implantable body, which sections 166a, 166b are referred to hereinafter as side sections. The straps 165 can prevent the side sections 166a, 166b from pulling or breaking away from the electrodes 160 when the implantable body 158 is placed under tension and/or is bent. In this embodiment, the straps 165 are formed of the same elastomeric material as the side sections 166a, 166b. The straps 165 are integrally formed with the side sections 166a, 166b. From their connection points with the side sections 166a, 166b, the straps 165 decrease in width towards a central part of the each electrode 160, minimizing the degree to which the straps 165 cover the surfaces of the electrodes 160 and ensuring that there remains a relatively large amount of electrode surface that is exposed around the circumference of the electrodes 160 to make electrical contact with adjacent body tissue. With reference to Fig. 3D, around a circumference of each electrode 160, at least 75% of the outer electrode surface, at least 80%, at least 85% or at least 90% of the outer electrode surface may be exposed for electrical contact with tissue, for example.

In alternative embodiments, a different number of straps 165 may be employed, e.g., one, three, four or more straps 165. Where a greater number straps 165 is employed, the width of each strap 165 may be reduced. The straps 165 may be distributed evenly around the circumference of each electrode 160 or distributed in an uneven manner. Nevertheless, in some embodiments, the straps 165 may be omitted, ensuring that all of the outer electrode surface is exposed for electrical contact with tissue, around a circumference of the electrode 160.

As indicated above, the implantable body 158 is formed of an elastomeric material such as silicone. The elastomeric material allows the implantable body 158 to bend, flex and stretch such that the implantable body 158 can readily contort as it is routed to a target implantation position and can readily conform to the shape of the body tissue at the target implantation position. The use of elastomeric material also ensures that any risk of trauma to the subject is reduced during implantation or during subsequent use.

In embodiments of the present disclosure the electrical connection 167 to the electrodes 160 comprises relatively fragile platinum wire conductive elements. With reference to Figs. 3C to 3E, for example, to reduce the likelihood that the platinum wires will break or snap during bending, flexing and/or stretching of the implantable body 158, the electrical connection 167 is provided with wave-like shape and, more specifically, a helical shape in this embodiment, although other non-linear shapes may be used. The helical shape, for example, of the electrical connection 167 enables the electrical connection 167 to stretch, flex and bend in conjunction with the implantable body 158. Bending, flexing and/or stretching of the implantable body 158 typically occurs during implantation of the implantable body 158 in a subject and upon any removal of the implantable body 158 from the subject after use.

As indicated above, a reinforcement device 168 is also provided in the electrode device 157, which reinforcement device 168 extends through the implantable body 158 and is provided to limit the degree by which the length of the implantable body 158 can extend under tension. The reinforcement device 168 can take the bulk of the strain placed on the electrode device 157 when the electrode device 157 is placed under tension. The reinforcement device 168 is provided in this embodiment by a fiber (e.g., strand, filament, cord or string) of material that is flexible and which has a high tensile strength. In particular, a fiber of ultra-high-molecular-weight polyethylene (UHMwPE), e.g., Dyneema^{™}, is provided as the reinforcement device 168 in the present embodiment. The reinforcement device 168 extends through the implantable body 158 in the length direction of the implantable body 158 and is generally directly encased by the elastomeric material of the implantable body 158.

The reinforcement device 168 may comprise a variety of different materials in addition to or as an alternative to UHMwPE. The reinforcement device may comprise other plastics and/or non-conductive material such as a poly-paraphenylene terephthalamide, e.g., Kevlar^{™}. In some embodiments, a metal fiber or surgical steel may be used.

Similar to the electrical connection 167, the reinforcement device 168 also has a wave-like shape and, more specifically, a helical shape in this embodiment, although other non-linear shapes may be used. The helical shape of the reinforcement device 168 is different from the helical shape of the electrical connection 167. For example, as evident from Figs. 3C to 3E, the helical shape of the reinforcement device 168 has a smaller diameter than the helical shape of the electrical connection 167. Moreover, the helical shape of the reinforcement device 168 has a greater pitch than the helical shape of the electrical connection 167.

When the implantable body 168 is placed under tension, the elastomeric material of the implantable body will stretch, which in turns causes straightening of the helical shapes of both the electrical connection 167 and the reinforcement device 168. As the electrical connection 167 and the reinforcement device straighten 168, their lengths can be considered to increase in the direction of elongation of the implantable body 158. Thus, the lengths of each of the electrical connection 167 and the reinforcement device 168, in the direction of elongation of the implantable body 158, are extendible when the implantable body 158 is placed under tension.

For each of the electrical connection 167 and the reinforcement device 168, a theoretical maximum length of extension in the direction of elongation of the implantable body 158 is reached when its helical shape (or any other non-linear shape that may be employed) is substantially completely straightened. However, due to the differences in the helical shapes of the electrical connection 167 and the reinforcement device 168, the maximum length of extension of the reinforcement device 168 is shorter than the maximum length of extension of the electrical connection 167. Therefore, when the implantable body 158 is placed under tension, the reinforcement device 168 will reach its maximum length of extension before the electrical connection 167 reaches its maximum length of extension. Indeed, the reinforcement device 168 can make it substantially impossible for the electrical connection 167 to reach its maximum length of extension. Since the electrical connection 167 can be relatively fragile and prone to breaking, particularly when placed under tension, and particularly when it reaches a maximum length of extension, the reinforcement device 168 can reduce the likelihood that the electrical connection 167 will be damaged when the implantable body 158 is placed under tension. In contrast to the electrical connection 167, when the reinforcement device 168 reaches its maximum length of extension, its high tensile strength allows it to bear a significant amount of strain placed on the electrode device 157, preventing damage to the electrical connection 167 and other components of the electrode device 157.

In consideration of other components of the electrode device 157 that are protected from damage by the reinforcement device 168, it is notable that the implantable body 158 can be prone to damage or breakage when it is placed under tension. The elastomeric material of the implantable body 158 has a theoretical maximum length of extension in its direction of elongation when placed under tension, the maximum length of extension being the point at which the elastomeric material reaches its elastic limit. In this embodiment, the maximum length of extension of the reinforcement device 168 is also shorter than the maximum length of extension of the implantable body 158. Thus, when the implantable body 158 is placed under tension, the reinforcement device 168 will reach its maximum length of extension before the implantable body 158 reaches its maximum length of extension. Indeed, the reinforcement device 168 can make it substantially impossible for the implantable body 158 to reach its maximum length of extension. Since elastomeric material of the implantable body 158 can be relatively fragile and prone to breaking, particularly when placed under tension, and particularly when it reaches its elastic limit, the reinforcement device 168 can reduce the likelihood that the implantable body 158 will be damaged when it is placed under tension.

In this embodiment, the helical shapes of the reinforcement device 168 and the electrical connection 158 are provided in a concentric arrangement. Due to its smaller diameter, the reinforcement device 168 can locate radially inside of the electrical connection 167. In view of this positioning, the reinforcement device 168 provides a form of strengthening core to the implantable body 158. The concentric arrangement can provide for increased strength and robustness while offering optimal surgical handling properties, with relatively low distortion of the implantable body 158 when placed under tension.

As indicated, the reinforcement device 168 is directly encased by the elastomeric material of the implantable body 158. The helically-shaped reinforcement device 168 therefore avoids contact with material other than the elastomeric material in this embodiment. The helically shaped reinforcement device is not entwined or intertwined with other strands or fibers, for example (e.g., as opposed to strands of a rope), ensuring that there is a substantial amount of give possible in relation to its helical shape. The helical shape can move to a straightened configuration under tension as a result, for example.

The arrangement of the reinforcement device 168 is such that, when the implantable body 158 is placed under tension, the length of the reinforcement device 168 is extendible by about 20% of its length when the implantable body 158 is not under tension. Nevertheless, in embodiments of the present disclosure, a reinforcement device 168 may be used that is extendible by at least 5%, at least 10%, at least 15%, at least 20% or at least 25% or otherwise, of the length of the reinforcement device when the implantable body 158 is not under tension. The maximum length of extension of the reinforcement device 168 in the direction of elongation of the implantable body 158 may be about 5%, about 10%, about 15%, about 20% or about 25% or otherwise of its length when the implantable body 158 is not under tension.

As represented in Fig. 3C, the reinforcement device 168 has a relatively uniform helical configuration along its length. However, in some embodiments, the shape of the reinforcement device 168 can be varied along its length. For example, the reinforcement device 168 can be straighter (e.g., by having a helical shape with smaller radius and/or greater pitch) adjacent the electrodes 160 in comparison to at other portions of the implantable body 158. By providing this variation in the shape of the reinforcement device 168, stretching of the implantable body 158 may be reduced adjacent the electrodes 160, where there could otherwise be a greater risk of the electrodes 160 dislocating from the implantable body 158. This enhanced strain relief adjacent the electrodes 160 can be provided while still maintaining the ability of the reinforcement device 168, and therefore implantable body 158, to stretch to a desirable degree at other portions of the implantable body 158.

As indicated, the electrical connection 167 in this embodiment comprises relatively fragile platinum wire conductive elements. At least 4 platinum wires are provided in the electrical connection 167 to each connect to a respective one of the four electrodes 160. The wires are twisted together and electrically insulated from each other. Connection of a platinum wire of the electrical connection 167 to the most distal of the electrodes 160 is illustrated in Fig. 3C. As can be seen, the wire is connected to an inner surface 172 of the electrode 160, adjacent a distal end of the electrode 160, albeit other connection arrangements can be used.

The reinforcement device 168 extends through the hollow center of each of the electrodes 160. The reinforcement device 168 extends at least from the distal most electrode 160, and optionally from a region adjacent the distal tip 159 of the implantable body 158, to a position adjacent the amplifier 163. In some embodiments, the reinforcement device 168 may also extend between the amplifier 163 and the processing unit 144. In some embodiments, the reinforcement device 168 may extend from the distal tip 159 and/or the distal most electrode 160 of the implantable body 158 to the processing unit 144.

To prevent the reinforcement device 168 from slipping within or tearing from the elastomeric material of the implantable body 158, a series of knots 169 are formed in the reinforcement device 168 along the length of the reinforcement device 168. For example, with reference to Fig. 3F, a knot 169a can be formed at least at the distal end of the reinforcement device 168, adjacent the distal tip 159 of the implantable body 158, and/or knots 169 can be formed adjacent one or both sides of each electrode 160. The knots may alone provide resistance to movement of the reinforcement device 168 relative to the elastic material of the implantable body 158 and/or may be used to fix (tie) the reinforcement device 168 to other features of the device 157.

In the present embodiment for example, as illustrated in Fig. 3C, the reinforcement device 168 is fixed, via a knot 169b, to each electrode 160. To enable the reinforcement device 168 to be fixed to the electrode 160, the electrode 160 comprises an extension portion 173 around which knots 169 of the reinforcement device 168 can be tied. As shown in Fig. 3C, the extension portion 173 can include a loop or arm of material that extends across an open end of the hollow cylinder forming the electrode 160.

With reference to Figs. 3A, 3B, 3F, and 3G, the electrode device 158 comprises at least one anchor 164, and in this embodiment of plurality of anchors 164. The plurality of anchors 164 are positioned along a length of the implantable body 158, each adjacent a respective one of the electrodes 160. Each anchor 164 is configured to project radially outwardly from the implantable body 158 and specifically, in this embodiment, at an angle towards a proximal end of the implantable body 158. Each anchor 164 is in the form of a flattened appendage or fin with a rounded tip 170. The anchors 164 are designed to provide stabilization to the electrode device 157 when it is in the implantation position. When implanted, a tissue capsule can form around each anchor 164, securing the anchor 164 and therefore the implantable body 158 into place. In this embodiment, the anchors 164 are between about 0.5 mm and 2 mm in length, e.g., about 1 mm or 1.5 mm in length.

So that the anchors 164 do not impede implantation of the electrode device 157, or removal of the electrode device 157 after use, each anchor 164 is compressible. The anchors 164 are compressible (e.g., foldable) to reduce the degree by which the anchors 164 projects radially outwardly from the implantable body 158. To further reduce the degree by which the anchors 164 project radially outwardly from the implantable body 158 when compressed, a recess 171 is provided in a surface of the implantable body 158 adjacent each anchor 164. The anchor 164 is compressible into the recess 171. In this embodiment, the anchors 164 project from a bottom surface of the respective recess 171 and the recess 171 extends on both proximal and distal sides of the anchor 164. Accordingly, the anchors 164 can be compressed into the respective recesses in either a proximal or distal direction. This has the advantage of allowing the anchors 164 to automatically move into a storage position in the recess 171 when pulled across a tissue surface or a surface of a implantation tool such as delivery device, in either of a proximal and a distal direction.

The electrode device 157 of the present embodiment is configured for use in monitoring electrical activity in the brain and particularly for monitoring electrical activity relating to epileptic events in the brain. The electrode device 157 is configured to be implanted at least partially in a subgaleal space between the scalp and the cranium. At least the electrodes 160 and adjacent portions of the implantable body 158 are located in the subgaleal space.

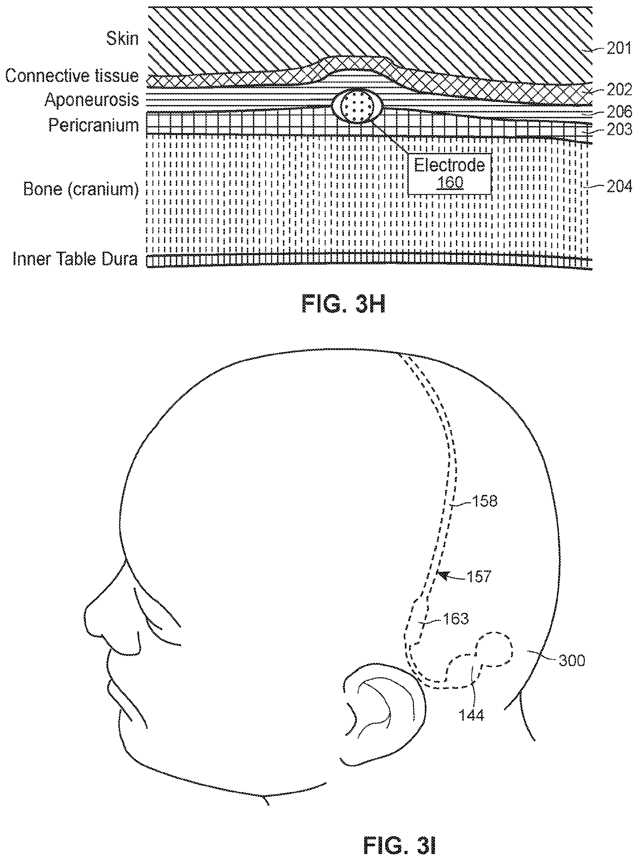

An illustration of the implantation location of the electrodes 160 is provided in Fig. 3H. As can be seen, the electrodes 160 locate in particular in a pocket between the galea aponeurotica 206 and the pericranium 203. When implanted, the first and second electrode pairs 161, 162 are located on respective sides of the midline of the head of the subject in a substantially symmetrical arrangement. The first and second electrode pairs 161, 162 therefore locate over the right and left hemispheres of the brain, respectively. For example, the first electrode pair 161 can be used to monitor electrical activity at right hemisphere of the brain and the second electrode pair 162 can be used to monitor electrical activity at the left hemisphere of the brain, or vice-versa. Independent electrical activity data may be recorded for each of the right and left hemispheres, e.g., for diagnostic purposes, To position the electrodes pairs 161, 162 over the right and left hemispheres of the brain, the implantable body 158 of the electrode device 157 is implanted in a medial-lateral direction over the cranium of the subject's head. The electrode pairs 161, 162 are positioned away from the subject's eyes and chewing muscles to avoid introduction of signal artifacts from these locations. The electrode device 157 implanted under the scalp in a position generally as illustrated in Fig. 3I.

Fig. 4 depicts the sensor array 102 as having a plurality of the electrode devices 110. Specifically, in the depicted embodiment, the sensor array 102 includes four electrode devices 110a-d, connected via the respective leads 140a-d of each, and further via a cable section 142, to a local processing device 144. Of course, in different embodiments, more or fewer numbers of electrode devices 110 may be implemented, according to the needs of the electrical signals required for the implementation of the methods described herein. In particular, the sensor array 102 may include, in embodiments, four, eight, 10, 12, 16, 20, 24, or more of the electrode devices 110. In the embodiment depicted in Fig. 4, the local processing device 144 and electrode devices 110a-d (along with their respective leads 140a-d and the cable section 142) are formed in the sensor array 102 as a one-piece construct. The arrangement is such that the local processing device 144 and the electrode devices 110a-d are permanently fixed together (for the purpose of normal operation and use). There is therefore no requirement or indeed possibility for a user, such as a surgeon, to connect these components of the sensor array 102 together prior to implantation, therefore increasing the strength, cleanliness and ease of use of the sensor array 102.

The local processing device 144 may be implanted under skin tissue. With reference to Fig. 5A, the local processing device 144 can include an electrical amplifier 146, a battery 148, a transceiver 150, an analogue to digital converter (ADC) 152, and a processor 154 to process electrical signals received from or transmitted to the electrodes devices 110a-d. The local processing device 144 can include a memory 156 to store signal processing data. The local processing device 144 may be similar to a processing device of a type commonly used with cochlear implants, although other configurations are possible.

The data processed and stored by the local processing device 144 may be raw EEG data or partially processed (e.g. partially or fully compressed) EEG data, for example. The EEG data may be transmitted from the local processing device 144 wirelessly, or via a wire, to the processor device 104 for further processing and analyzing of the data. The processor device 104 may analyze EEG signals (or other electrical signals) to determine if a target event has occurred. Data regarding the event may be generated by the processor device 104 on the basis of the analysis, as described further herein. In one example, the processor device 104 may analyze brain activity signals to determine if a target event such as an epileptic event has occurred and data regarding the epileptic event (e.g., classification of the event) may be generated by the processor device 104 on the basis of the analysis.

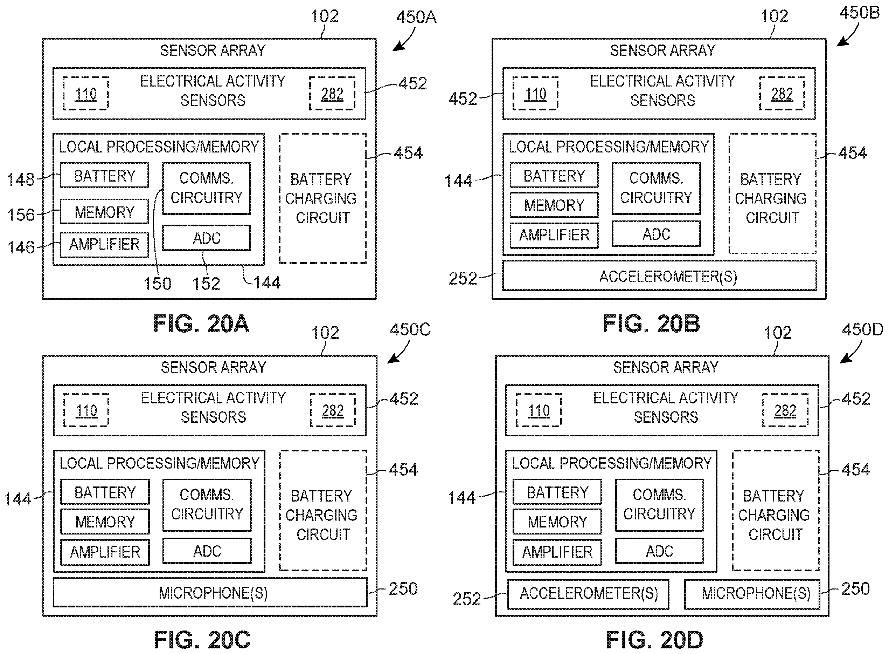

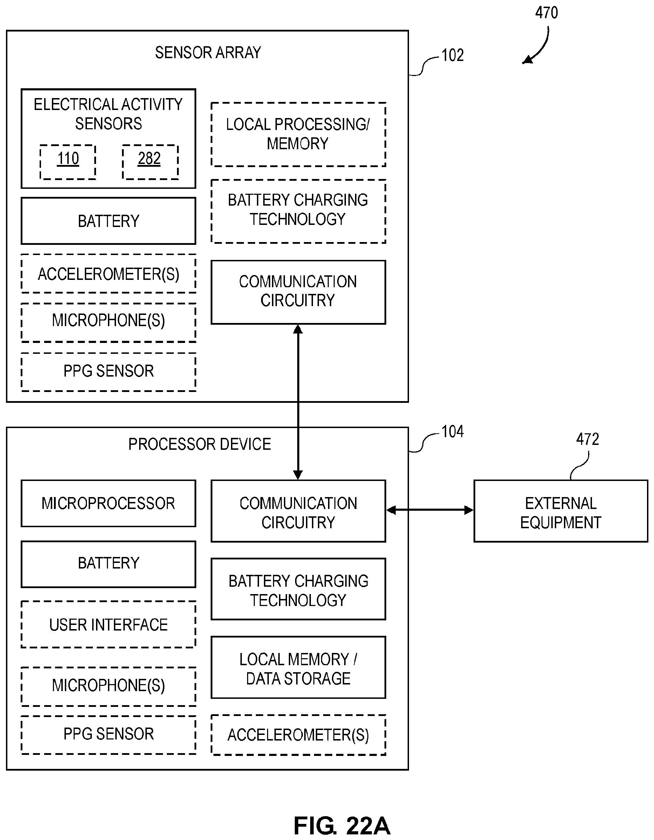

By carrying out data analysis externally to the sensor array 102, using the processor device 104 (whether separate from the sensor array 102 or integrated with the sensor array, as described with reference to Figs. 20A-20H and 22A-22G), for example, there may be a reduction in power consumption within the sensor array 102, enabling the sensor array 102 to retain a smaller geometrical form. Moreover, the processor device 104 may have significantly higher processing power than would be possible with any processor included in the sensor array 102. The processor device 104 may run software that continuously records electrical data received from the sensor array 102.

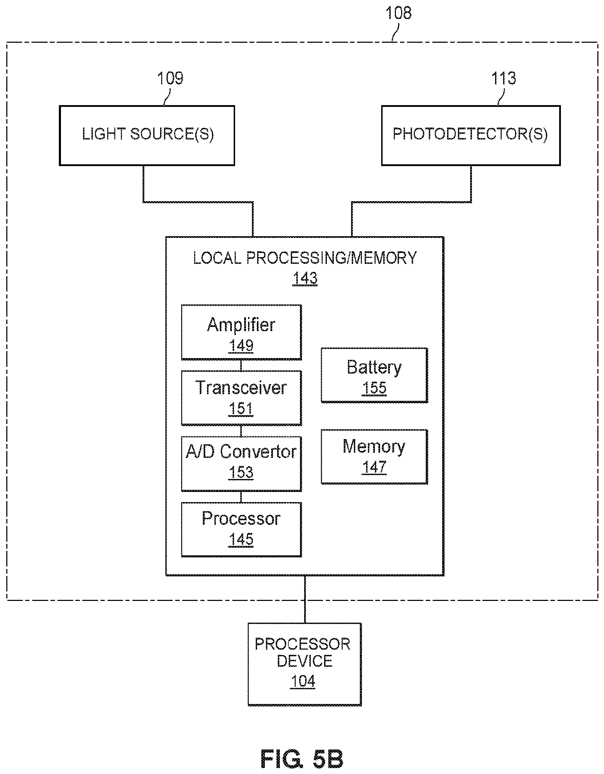

With reference to embodiments implementing the PPG sensor 108, Fig. 5B is a block diagram depicting components of the PPG sensor 108. Generally, in these embodiments, the PPG sensor 108 is configured to be disposed on a sensing location of the patient and, in particular embodiments, in locations that will be unobtrusive to the patient during long term use (e.g., several days or weeks) of the PPG sensor 108. As such, while the PPG sensor 108 may be configured as a fingertip type sensor (implementing transmissive absorption sensing) worn on the finger or on a toe, other sensing locations may be more advantageous in terms of comfort to the patient. For instance, PPG sensors implementing reflection sensing may allow for the sensor to be worn on a patient's wrist, much like a watch or other sensor band, or on the ankle. In embodiments, the PPG sensor 108 may, in fact, be integrated into a smart watch device.

As will be understood, in embodiments in which it is implemented, the PPG sensor 108 may use low-intensity infrared (IR) light to detect various biomarkers of the patient. Blood absorbs IR light more strongly than other, surrounding tissues and, as a result, changes in blood flow may be sensed as changes in the intensity of transmitted or reflected IR light. While the intricacies and details of the operation of a PPG sensor will not be elaborated upon in this specification, as a person of ordinary skill in the art will readily appreciate the design and operation of these devices, it should be understood generally that the PPG sensor 108 may be used to measure and/or determine any variety of biomarkers, including, but not limited to: heart rate, heart rate variability, blood pressure, cardiac output, respiration rate, and blood oxygen saturation.

With reference to Fig. 5B, the PPG sensor 108 generally includes one or more light sources 109 which may include IR light sources and, in embodiments, additional visible light sources. The PPG sensor 108 also includes one or more photodetectors 113 configured to detect the particular wavelengths of light from which the PPG data will be generated. The PPG sensor 108 also includes a local processing device 143 that, in turn, can include an electrical amplifier 149, a battery 155, a transceiver 151, an analogue to digital converter (ADC) 153, and a processor 145 to process electrical signals received from the photodetector(s) 113. The local processing device 143 can include a memory 147 to store signal processing data.

The data processed and stored by the local processing device 143 may be raw PPG data (i.e., unprocessed signal data) or processed PPG data (e.g., data from which the desired biomarkers have already been extracted), for example. The PPG data may be transmitted from the local processing device 143 wirelessly, or via a wired connection, to the processor device 104 for further processing and analyzing of the data. The processor device 104 may analyze PPG data, by itself or with the EEG data, to determine a state of the patient. Data regarding the patient state may be generated by the processor device 104 on the basis of the analysis, as described further herein. In one example, the processor device 104 may analyze brain activity signals and biomarkers to determine a current condition of the patient and/or predict a future condition of the patient.

By carrying out data analysis externally to the PPG sensor 108, using the processor device 104, (whether separate from the sensor array 102 or integrated with the sensor array, as described with reference to Figs. 20A-20H and 22A-22G), for example, there may be a reduction in power consumption within the PPG sensor 108, enabling the PPG sensor 108 to retain a smaller geometrical form. Moreover, the processor device 104 may have significantly higher processing power than would be possible with any processor included in the PPG sensor 108. The processor device 104 may run software that continuously records the data received from the PPG data 108.

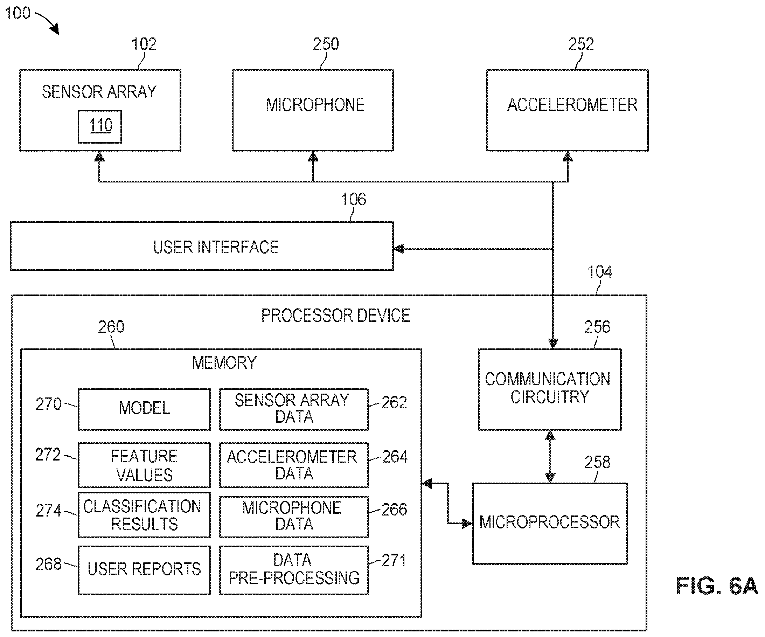

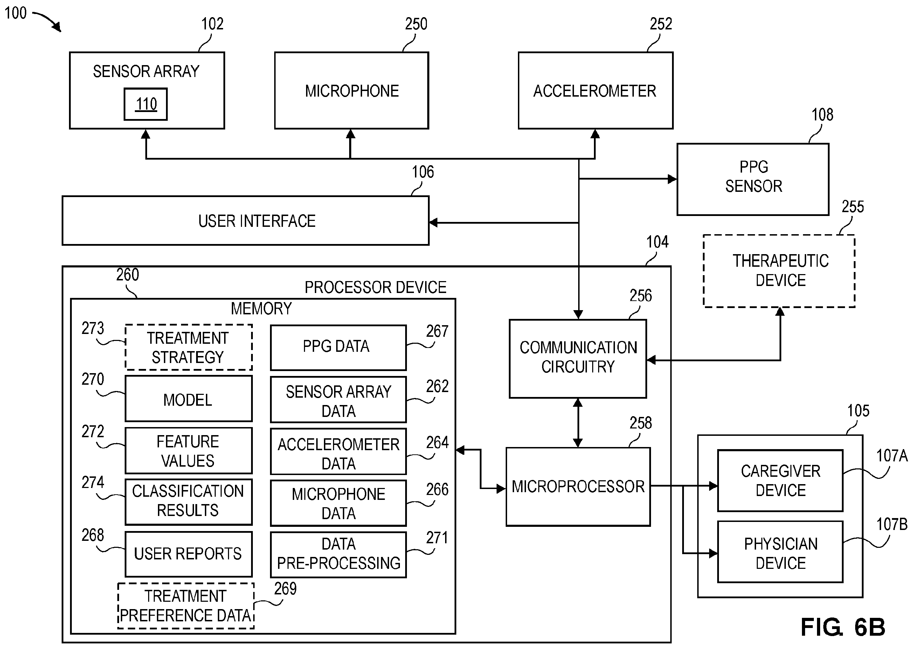

Turning now to Figs. 6A-6C, the systems 100 are presented as a block diagram in greater detail. As depicted in Figs. 6A and 6B, the system 100 includes, in embodiments, a microphone 250 and an accelerometer 252 and, in embodiments a therapeutic device 255 (Fig. 6B), in addition to the sensor array 102, the PPG sensor 108 (Fig. 6B), the processor device 104, and the user interface 106. Each of the sensor array 102, the PPG sensor 108 (in embodiments in which it is included), the microphone 250, and the accelerometer 252 may sense or collect respective data and communicate the respective data to the processor device 104. As should be understood at this point, in embodiments, the sensor array 102 may include an array of electrode devices 110 that provide electrical signal data and, in particular, provide electrical signal data indicative of brain activity of the patient (e.g., EEG signal data). As will be described further herein, the sensor array 102 may, additionally or alternatively, provide electrical signal data indicative of detected chemical biomarkers, in embodiments. As should also be understood in view of the description above, the sensor array 102 may be disposed beneath the scalp of the patient - on and extending into the cranium 204 - so as to facilitate accurate sensing of brain activity. However, in embodiments, it is also contemplated that the sensor array 102 need not be placed beneath the scalp.

With reference now to Fig. 6B, the PPG sensor 108 detects, using a photodetector circuit, light that is transmitted through or reflected from the patient after the light interacts with the blood just beneath the surface of the patient's skin. The PPG sensor 108 may be any type of PPG sensor suitable for disposal on the patient and, in particular, suitable for operation from a portable power source such as a battery. The PPG sensor 108 may be disposed at any of a variety of positions on the patient including, but not limited to, the patient's finger, toe, forehead, earlobes, nasal septum, wrist, ankle, arm, torso, leg, hand, or neck. In some embodiments, the PPG sensor 108 may be integrated with the sensor array 102 and placed on or beneath the scalp of the patient with the sensor array 102, while in others the PPG sensor 108 may be integrated with the processor device 104, and still in others the PPG sensor 108 may be distinct from both the sensor array 102 and the processor device 104. Of course, while depicted in the accompanying figures as a single PPG sensor, the PPG sensor 108 may be one or more PPG sensors, disposed as connected or distinct units on a variety of positions on the patient (so-called multi-site photoplethysmography). In embodiments implementing multiple PPG sensors, the multiple PPG sensors may be of the same type, or may be different, depending on the location of each on the patient, the environment in which each is disposed, the location of each in the hardware (e.g., separate from other devices or integrated with the processor device 104, for example), etc.

The optional therapeutic device 255 may be a device that provides therapeutic support to the patient to treat or mitigate the effects of the patient's condition or of events related to the patient's condition. For example, the therapeutic device 255 may administer a therapy on a regular basis to help treat the underlying condition, or in response to a detected event (e.g., after a seizure) to facilitate or accelerate the dissipation of after effects of the event. The therapeutic device 255 may, in some embodiments, be a drug pump that delivers timed, measured doses of a pharmacological agent (i.e., a drug) to the patient, while in other embodiments the therapeutic device 255 may be an oxygen generator configured to increase (or, potentially, decrease) the patient's oxygen levels according to predicted or determined need. In still other embodiments, the therapeutic device 255 may be a continuous positive airway pressure (CPAP) device or an adaptive servo ventilation device, each of which may be employed for mitigating obstructive sleep apnea, which may increase of decrease pressure according to detected. In further embodiments, the therapeutic device 255 may be a neurostimulator device (e.g., a vagal nerve stimulation device, a hypoglossal nerve stimulation device, an epicranial and/or transcranial electrical stimulation device, an intracranial electrical stimulation device, a phrenic nerve stimulator, a cardiac pacemaker, etc.) configured to apply or adjust (e.g., amplitude, frequency of the signal, frequency of the stimulus application, etc.) a neurostimulation signal. Cardiac pacemakers and phrenic nerve stimulators, respectively, may be used to ensure proper cardiac and diaphragmatic function, ensuring that the patient continues to have adequate cardiac and/or respiratory function.

Referring again to Figs. 6A and 6B, the microphone 250 detects sound related to the patient and the patient's environment. The microphone 250 may be any type of microphone suitable for disposal on the patient and suitable for operation from a portable power source such as a battery. In particular, the microphone 250 may be a piezoelectric microphone, a MEMS microphone, or a fiber optic microphone. In embodiments, an accelerometer device may be adapted to measure vibrations and, accordingly, to detect sound, rendering the accelerometer device suitable for use as the microphone 250. The microphone 250 may be disposed at any of a variety of positions on the patient including, but not limited to, the patient's head, arm, torso, leg, hand, or neck. In some embodiments, the microphone 250 may be integrated with the sensor array 102 and placed on or beneath the scalp of the patient with the sensor array 102, while in others the microphone 250 may be integrated with the processor device 104, and still in others the microphone 250 may be distinct from both the sensor array 102 and the processor device 104. In embodiments, especially those in which the patient's voice is the primary sensing target for the microphone 250, the microphone 250 senses sound via bone conduction. In some embodiments, the microphone 250 may be integrated with a hearing or vestibular prosthesis. Of course, while depicted in the accompanying figures as a single microphone, the microphone 250 may be one or more microphones, disposed as an array in a particular position on the patient, or as distinct units on a variety of positions on the patient. In embodiments implementing multiple microphones, the multiple microphones may be of the same type, or may be different, depending on the location of each on the patient, the environment in which each is disposed (e.g., sub-scalp vs. not), the location of each in the hardware (e.g., separate from other devices or integrated within the processor device 104, for example), etc. Each may have the same or different directionality and/or sensitivity characteristics as the others, depending on the placement of the microphone and the noises or vibrations the microphone is intended to detect.

The microphone 250 may detect the patient's voice, in embodiments, with the goal of determining one or more of: pauses in vocalization; stutters; periods of extended silence; abnormal vocalization; and/or other vocal abnormalities that, individually or in combination with data from the sensor array 102, the accelerometer 252, and/or self-reported data received via the user interface 106, may assist algorithms executing within the processor device 104 in determining whether the patient has experienced an event of interest and, if so, classifying the event as described herein. In embodiments, the microphone 250 may also detect other noises in the patient's environment that may be indicative that the patient experienced an event of interest. For example, the microphone 250 may detect the sound of glass breaking, which may indicate that the patient has dropped a glass. Such an indication, in conjunction with electrical signals detected by the sensor array 102, may provide corroboration that the patient has, in fact, experienced an event of interest.

In embodiments, such as that of Fig. 6B, the microphone 250 may detect other sounds, such as snoring, ambient noise, or other acoustic signals. For example, the microphone 250 may detect that the patient is snoring. Such information may be useful, for example, when analyzed in concert with other biomarker data such as blood oxygen saturation levels detected by the PPG sensor 108. (A drop in blood oxygen saturation level, coupled with a cessation of snoring may indicate an obstructive sleep apnea condition, for instance.) As another non-limiting example, the microphone 250 may detect that there are acoustic signals (e.g., a voice) present. When analyzed in concert with other biomarker data, this could provide information about a cochlear disorder. (Detection of an voice by the microphone 250 that does not have corresponding electrical activity detected by the sensor array 102 indicating processing of the signal by the brain may indicate that the patient cannot hear the voice, for instance.)

In the embodiments of Figs. 6A and 6B, the accelerometer 252 detects movement and/or orientation of the patient. The accelerometer 252 may be any type of accelerometer suitable for disposal on the patient and suitable for operation from a portable power source such as a battery. In particular, and by way of example, the accelerometer 252 may be a chip-type accelerometer employing MEMS technology, and may include accelerometers employing capacitive, piezoelectric resistive, or magnetic induction technologies. Like the microphone 250, the accelerometer 252 may be in any of a variety of positions on the patient including, but not limited to, the patient's head, arm, torso, leg, hand, or neck. In some embodiments, there may be multiple accelerometers, to detect motions in different parts of the body. In some embodiments, an accelerometer 252 may be integrated with the sensor array 102 and placed on or beneath the scalp of the patient with the sensor array 102, while in others an accelerometer 252 may be integrated with the processor device 104 and still in others the accelerometer 252 may be distinct from both the sensor array 102 and the processor device 104. In some embodiments, the accelerometer 252 may be integrated with a hearing or vestibular prosthesis. Of course, while depicted in the accompanying figures as a single accelerometer, the accelerometer 252 may be one or more accelerometers, disposed as an array in a particular position on the patient, or as distinct units on a variety of positions on the patient. In embodiments implementing multiple accelerometers, the multiple accelerometers may be of the same type, or may be different, depending on the location of each on the patient, the environment in which each is disposed (e.g., sub-scalp vs. not), the location of each in the hardware (e.g., separate from other devices or integrated within the processor device 104, for example), etc. Each may have the same or different sensitivity characteristics and/or number of detectable axes as the others, depending on the placement of the accelerometer and the motions and/or vibrations the accelerometer is intended to detect.

The accelerometer 252 may detect tremors, pauses in movement, gross motor movement (e.g., during a tonic-clonic seizure), falls (e.g., during an atonic or drop seizure or a tonic seizure), repeated movements (e.g., during clonic seizures), twitches (e.g., during myoclonic seizures), and other motions or movements that, in combination with data from the sensor array 102, the microphone 250, and/or self-reported data received via the user interface 106, may assist algorithms executing with the processor device 104 in determining whether the patient has experienced an invent of interest and, if so, classifying the event. In embodiments, the accelerometer 252 may act as an additional microphone 250 or may act as the only microphone 250.

Like the microphone 250, the accelerometer 252 may be in any of a variety of positions on the patient including, but not limited to, the patient's head, arm, torso, leg, hand, or neck. In some embodiments, there may be multiple accelerometers, to detect motions in different parts of the body. In some embodiments, an accelerometer 252 may be integrated with the sensor array 102 and placed on or beneath the scalp of the patient with the sensor array 102, while in others an accelerometer 252 may be integrated with the processor device 104.

Together, the sensor array 102 and, if present, the microphone(s) 250 and/or accelerometer(s) 252 may provide data from which biomarker data related to the patient(s) may be extracted. The system 100 may be configured to determine a variety of biomarkers depending on the inclusion and/or placement of the various sensor devices (i.e., the sensor array 102 and, if present, the microphone(s) 250 and/or accelerometer(s) 252). By way of example, and not limitation, muscle tone biomarker data may be determined from a combination of electromyography data (i.e., from the electrode devices 110 in the sensor array 102) and accelerometer data collected by one or more accelerometers 252 disposed on the head and/or arms of the patient; unsteadiness biomarker data may be determined from accelerometer data collected by one or more accelerometers 252 disposed on the head and/or arms of the patient; posture biomarker data may be determined from accelerometer data collected by one or more accelerometers 252 disposed on the head and/or arms of the patient; mood disruption biomarker data may be determined from microphone data collected by one or more microphones 250; loss of coordination biomarker data may be determined from accelerometer data collected by one or more accelerometers 252 disposed on the head and/or arms of the patient; speech production biomarker data may be determined from microphone data collected by one or more microphones 250; epileptiform activity biomarker data may be determined from EEG data received from one or more electrode devices 110 in the sensor array 102; jaw movement biomarker data may be determined from a combination of electromyography data and microphone data collected by one or more devices (e.g., electrode devices 110 and/or accelerometers 252) disposed on the patient; fatigue biomarker data may be determined from accelerometer data collected by one or more accelerometers 252 disposed on the head of the patient; dizziness biomarker data may be determined from accelerometer data collected by one or more accelerometers 252 disposed on the head and/or arms of the patient; vomiting biomarker data may be determined from a combination of electromyography data, microphone data, and/or accelerometer data collected by one or more devices (e.g., electrode devices 110, microphones 250, accelerometers 252) disposed on the patient; sleep biomarker data may be determined from EEG data received from one or more electrode devices 110 in the sensor array 102, etc.

The processor device 104 receives data from the sensor array 102, the PPG sensor 108 (in embodiments related to Fig. 6B), the microphone 250, the accelerometer 252, and the user interface 106 and, using the received data, may detect and classify events of interest. The processor device 104 includes communication circuitry 256, a microprocessor 258, and a memory device 260. The microprocessor 258 may be any known microprocessor configurable to execute the routines necessary for detecting and classifying events of interest, including, by way of example and not limitation, general purpose microprocessors (GPUs), RISC microprocessors, digital signal processors (DSPs), application specific integrated circuits (ASICs), and field-programmable gate arrays (FPGAs).

The communication circuitry 256 may be any transceiver and/or receiver/transmitter pair that facilitates communication with the various devices from which the processor device 104 receives data and/or transmits data. The communication circuitry 256 is communicatively coupled, in a wired or wireless manner, to each of the sensor array 102, the microphone 250, the accelerometer 252, and the user interface 106. Additionally, the communication circuitry 256 is coupled to the microprocessor 258, which, in addition to executing various routines and instructions for performing analysis, may also facilitate storage in the memory 260 of data received, via the communication circuity 256, from the sensor array 102, the microphone 250, the accelerometer 252, and the user interface 106.

The memory 260 may include both volatile memory (e.g., random access memory (RAM)) and non-volatile memory, in the form of either or both of magnetic or solid state media. In addition to an operating system (not shown), the memory 260 may store sensor array data 262 received from the sensor array 102, PPG data 267 received from the PPG sensor 108 (in embodiments related to Fig. 6B), accelerometer data 264 received from the accelerometer(s) 252, microphone data 266 received from the microphone(s) 250, and user report data 268 received from the user (and/or other person such as a caregiver) via the user interface 106. In particular, the user report data 268 may include reports from the user, received via the user interface 106, of various types of symptoms. By way of non-limiting examples, the symptoms reported via the user interface 106 may include: perceived seizures/epileptic events; characteristics or features of perceived seizures/epileptic events such as severity and/or duration, perceived effects on memory, or other effects on the individual's wellbeing (such as their ability to hold a cup or operate a vehicle); other types of physiological symptoms (e.g., headaches, impaired or altered vision, involuntary movements, disorientation, falling to the ground, repeated jaw movements or lip smacking, etc.); characteristics or features of other symptoms (e.g., severity and/or duration); medication ingestion information (e.g., medication types, dosages, and/or frequencies/timing); perceived medication side-effects; characteristics or features of medication side-effects (e.g., severity and/or duration), and other user reported information (e.g., food and/or drink ingested, activities performed (e.g., showering, exercising, working, brushing hair, etc.), tiredness, stress levels, etc.), as well as the timing of each. With reference to embodiments of Fig. 6B, specifically, non-limiting examples of the symptoms reported via the user interface 106, in addition to those above, may include: perceived sleep apnea events; characteristics or features of perceived sleep apnea events such as severity and/or duration, perceived effects on memory, or other effects on the individual's wellbeing (such as their wakefulness); perceived vestibular and/or cochlear events; characteristics or features of perceived vestibular cochlear events such as severity and/or duration, perceived effects on balance or hearing, or other effects on the individual's wellbeing (such as their ability to hold a conversation or their ability to stand and/or ambulate).

In embodiments related to Fig. 6B, the memory 260 may also include treatment preference data 269. The treatment preference data 269 may indicate specific therapeutic goal data that may be used (e.g., by a treatment strategy routine) to adjust a target therapeutic effect and/or an acceptable level/amount/severity of side-effects. The treatment preference data 269 may be received, in embodiments, from the patient via the user interface 106. In other embodiments, the treatment preference data 269 may be received from an external device (e.g., from a physician device communicatively coupled to the system).

As will be described in greater detail below, the memory 260 may also store a model 270 for detecting and classifying events of interest according to a set of feature values 272 extracted from the sensor array data 262, the accelerometer data 264, the microphone data 266, and the user report data 268. Classification results 274 (and, by extension, detected events) output by the model 270 may be stored in the memory 260. A data pre-processing routine 271 may provide pre-processing of the sensor array data 262, the user report data 268 and, if present, the accelerometer data 264 and/or microphone data 266. As will be understood (and, in part, described below), the data pre-processing routine 271 may provide a range of pre-processing steps including, for example, filtering and extraction from the data of the feature values 272. Of course, it should be understood that wherever a routine, model, or other element stored in memory is referred to as receiving an input, producing or storing an output, or executing, the routine, model, or other element is, in fact, executing as instructions on the microprocessor 258. Further, those of skill in the art will appreciate that the model or routine or other instructions would be stored in the memory 260 as executable instructions, which instructions the microprocessor 258 would retrieve from the memory 260 and execute. Further, the microprocessor 258 should be understood to retrieve from the memory 260 any data necessary to perform the executed instructions (e.g., data required as an input to the routine or model), and to store in the memory 260 the intermediate results and/or output of any executed instructions.

In embodiments, the data pre-processing routine 271 may also extract from the sensor array data 262, the PPG data 267 (in embodiments related to Fig. 6B), the accelerometer data 264, and the microphone data 266, one or more biomarkers. The one or more biomarkers may be included among the feature values that are provided as inputs to the model 270, in embodiments, in order for the model 270 to output detected and/or classified events to the classification results 274.

The data stored in the sensor array data 262, the PPG data 267 (in embodiments related to Fig. 6B), the accelerometer data 264, the microphone data 266, and the user report data 268 is stored with corresponding time stamps such that the data may be correlated between data types. For example, each value in the sensor array data 262 should have a corresponding time stamp such that the microphone data 266, accelerometer data 264, and user report data 268 for the same time (and/or different times) can be compared, allowing the various types of data to be lined up and analyzed for any given time period. With respect to the user report data 268, there may be multiple time stamps for any particular user report, including, for example, the time that the user filled out the user report and the time of the event that the user was reporting (as reported by the user).

Events need not be contemporaneous to be relevant or related, or to be feature values input into the model. Put another way, the model 270 may consider temporal relationships between non-contemporaneous events in detecting and/or classifying an event. By way of example and not limitation, an electrical activity event (e.g., EEG signals) indicating a seizure may be classified as a particular type of event if preceded by the ingestion of medication, and as a different type of event if not preceded by the ingestion of the medication. Other examples of non-contemporaneous events preceding a seizure that are precursors are patient subjective reports of auras or optical lights, shortness of breath or increased cardiac pulse rate, and acoustic biomarkers suggesting the alteration of speech patterns. Additionally, the system 100 and, in particular, the model 270, may identify pre- and/or post-seizure events, such as unsteady balance, falls, slurred speech, or brain activity patterns that are indicative of a pre- and/or post-seizure event.

Of course, contemporaneous events may also be relevant. For example, accelerometer data indicative of a generalized tonic-clonic (i.e., grand mal) seizure may be classified as such if it is accompanied by contemporaneous electrical activity indicative of such a seizure.

The memory 260 may also store a treatment strategy routine 273, in embodiments depicted in Fig. 6B. The treatment strategy routine 273 may include pre-programmed treatment strategies recommended or implemented according to the biomarkers extracted from the EEG data 262, the PPG data 267, the accelerometer data 264, the microphone data 266, the feature values 272, the user reports 268, and/or the classification results 274. For example, the treatment strategy routine 273 may be programmed to recommend to the patient or a caregiver, or to implement (e.g., via the therapeutic device 108), increased supplemental oxygen for the patient if the PPG data 267 show decreased blood oxygen levels, or if the classification results 274 produced by the model 270 include that the patient has just suffered a seizure and that the likely effects of that seizure are decreased blood oxygen levels. As another example, the model 270 may, based on feature values 272 extracted from the EEG data 262, the PPG data 267, the accelerometer data 264, and the microphone data 266, output classification results 274 indicating that the patient is having frequent sleep apnea episodes. The treatment strategy routine 273 may be programmed to recommend to the patient that the patient increase the pressure on a CPAP device or adjust the settings on a hypoglossal nerve stimulation device or, in embodiments in which the processor device 104 is communicatively coupled to the therapeutic device 255 (e.g., the CPAP device, adaptive servo ventilation device, or the hypoglossal nerve stimulation device), to adjust the settings on the therapeutic device 255 directly to decrease the frequency or severity of the sleep apnea events.

Fig. 6B also depicts optional external processor devices 105, which may include, in various embodiments one or more caregiver devices 107A and one or more physician devices 107B. As will be described in greater detail below, the external devices 105 may receive alerts or alarms from the processor device 104 about occurring or recently occurred events (e.g. seizures, sleep apnea desaturations, etc.), and may receive, in some embodiments, proposed treatment recommendations or requests for approval to implement adjustments to one or more settings of the therapeutic device 255. The external devices 105 and, in particular, the caregiver device 107A, may include an instance of the user interface 106, allowing the caregiver to provide information about the state of the patient.

As described above and throughout this specification, the interplay between biomarkers derived from the EEG data 262, the PPG data 267 (where present), the accelerometer data 264, and the microphone data 266, may provide insight into neurological, cardiac, respiratory, and even inflammatory function in the patient. Measurement of these functions can improve the detection and classification of events and conditions. Measurement of these functions can also improve understanding of patient-specific physiological changes that result from the condition or the events associated with the condition.

Specifically, biomarkers that can be extracted from the PPG data 267 may improve clinical or sub-clinical seizure detection, as changes in biomarkers in the PPG data 267 may coincide or have specific temporal relationships with biomarkers in the EEG data 262 and with events detected in the accelerometer data 264 and/or the microphone data 266. At the same time, biomarkers in the PPG data 267 may be used to determine if changes to blood oxygen levels, and cardiac and respiratory function are related to seizure activity or drug side-effects, which can assist in the optimization of treatment dose and timing to maximize therapeutic effect while minimizing side-effects. In addition, while biomarkers in the EEG data 262 may provide sufficient data, in some instances, to determine whether a seizure (or an event related to another condition, such as sleep apnea) is occurring or has occurred, the additional cardiac-related biomarker information extracted from the PPG data 267 may inform whether the seizure is cardiac induced or, instead, is causing cardiac changes (i.e., may determine a cause-effect relationship between seizure events and cardiac function). PPG-related biomarkers may also help sub-classify clinical and sub-clinical seizures as those that are ictal hypoxemic and those that are not.

Biomarkers extracted from the PPG data 267 may also be used to characterize blood oxygenation, cardiac, and respiratory changes before, at the onset of, during, and after seizures. These seizure-related effects on the patient can include respiratory changes that include obstructive apnea, tachypnea, bradypnea, and hypoxemia.

Additionally, the combination of biomarkers extracted from the PPG data 267 and the EEG data 262 may facilitate detection of SUDEP (sudden unexplained death in epilepsy) or SUDEP-precipitating events. That is, by monitoring the patient's heart-rate, blood pressure, and/or blood oxygenation, in combination with EEG data 262, the system 100 may detect a SUDEP or SUDEP-precipitating event. In so doing, the system 100 may generate alerts or alarms for the patient, for the caregivers or physicians of the patient, or for bystanders. The system 100 may also activate connected therapeutic devices such as neurostimulators (vagal, transcranial, epicranial, intracranial, etc.) or cardiac defibrillators to counter or prevent SUDEP events when they are detected.

Patients, particularly those suffering from epilepsy and/or sleep disorders, can also benefit from characterization of sleep quality. The systems and methods described herein utilize biomarkers extracted from the PPG data 267, alone or with the EEG data 262, to characterize sleep quality (e.g., capture a sleep quality score). The scoring can be combined with indicators of sleep cycle data in the EEG data 262. A more holistic representation of the sleep quality for the individual can be developed by including information from the user report data 268 entered by the patient via the user interface 106 after the patient wakes. The sleep quality score for the patient can be used, for example by the treatment strategy routine 273, to make recommendations to caregivers or physicians regarding the adjustment of dosage and timing of medication or other treatments (e.g., VNS) such that treatment is titrated to reach clinical efficacy but move away from the dosage impacting sleep quality. In some embodiments in which the processor 104 is communicatively coupled to a therapeutic device 255, the treatment strategy routine 273 may implement adjustments to the therapeutic device. Such implementation may, in some embodiments, require the processor device 104 to communicate first with a physician (e.g., sending a request or alert to a device in the possession of the physician) to receive confirmation of the adjustment.

In view of these considerations, it is considered that while some objectives of the system 100 may be achieved using the model 270 according to known data about the patient and/or the condition, other objectives of the system 100 must necessarily implement a trained artificial intelligence (AI) model to achieve maximum benefit.

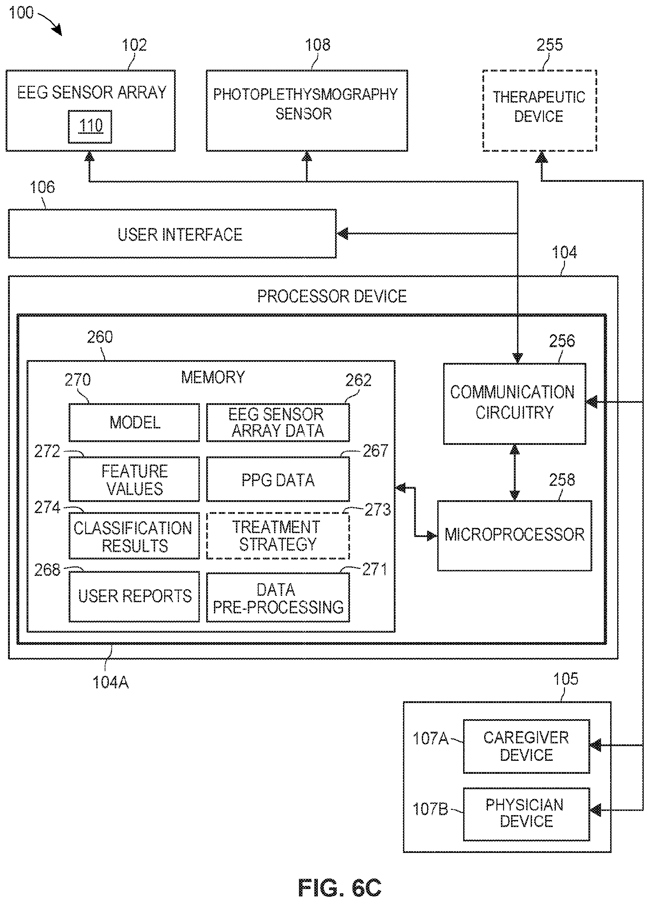

Turning now to Fig. 6C, the system 100 is presented as a block diagram with respect to the first subsystem in greater detail. As depicted in Fig. 6C, the system 100 includes, in embodiments, a therapeutic device 255, in addition to the sensor array 102, the PPG sensor 108, the processor device 104, and the user interface 106. Each of the sensor array 102 and the PPG sensor 108 may sense or collect respective data and communicate the respective data to the processor device 104. As should be understood at this point, in embodiments, the sensor array 102 may include an array of electrode devices 110 that provide electrical signal data and, in particular, provide electrical signal data indicative of brain activity of the patient (e.g., EEG signal data). As should also be understood in view of the description above, the sensor array 102 may be disposed beneath the scalp of the patient - on and/or extending into the cranium 204 - so as to facilitate accurate sensing of brain activity. However, in embodiments, it is also contemplated that the sensor array 102 need not be placed beneath the scalp.

The PPG sensor 108 detects, using a photodetector circuit, light that is transmitted through or reflected from the patient after the light interacts with the blood just beneath the surface of the patient's skin. The PPG sensor 108 may be any type of PPG sensor suitable for disposal on the patient and, in particular, suitable for operation from a portable power source such as a battery. The PPG sensor 108 may be disposed at any of a variety of positions on the patient including, but not limited to, the patient's finger, toe, forehead, earlobes, nasal septum, wrist, ankle, arm, torso, leg, hand, or neck. In some embodiments, the PPG sensor 108 may be integrated with the sensor array 102 and placed on or beneath the scalp of the patient with the sensor array 102, while in others the PPG sensor 108 may be integrated with the processor device 104, and still in others the PPG sensor 108 may be distinct from both the sensor array 102 and the processor device 104. Of course, while depicted in the accompanying figures as a single PPG sensor, the PPG sensor 108 may be one or more PPG sensors, disposed as connected or distinct units on a variety of positions on the patient (so-called multi-site photoplethysmography). In embodiments implementing multiple PPG sensors, the multiple PPG sensors may be of the same type, or may be different, depending on the location of each on the patient, the environment in which each is disposed, the location of each in the hardware (e.g., separate from other devices or integrated with the processor device 104, for example), etc.

The optional therapeutic device 255 may be a device that provides therapeutic support to the patient to treat or mitigate the effects of the patient's condition or of events related to the patient's condition. For example, the therapeutic device may administer a therapy prior to a predicted event (e.g., prior to a predicted seizure), or in response to a detected event (e.g., after a seizure) to facilitate or accelerate the dissipation of after effects of the event. The therapeutic device 255 may, in some embodiments, be a drug pump that delivers timed, measured doses of a pharmacological agent (i.e., a drug) to the patient, while in other embodiments the therapeutic device 255 may be an oxygen generator configured to increase (or, potentially, decrease) the patient's oxygen levels according to predicted or determined need. In still other embodiments, the therapeutic device 255 may be a continuous positive airway pressure (CPAP) device or an adaptive servo ventilation device, each of which may be employed for mitigating obstructive sleep apnea, which may increase of decrease pressure according to detected or predicted events. In further embodiments, the therapeutic device 255 may be a neurostimulator device (e.g., a vagus nerve stimulation device, a hypoglossal nerve stimulation device, an epicranial and/or transcranial electrical stimulation device, an intracranial electrical stimulation device, a phrenic nerve stimulator, a cardiac pacemaker, etc.) configured to apply or adjust (e.g., amplitude, frequency of the signal, frequency of the stimulus application, etc.) a neurostimulation signal. Cardiac pacemakers and phrenic nerve stimulators, respectively, may be used to ensure proper cardiac and diaphragmatic function, ensuring that the patient continues to have adequate cardiac and/or respiratory function.

The processor device 104 receives data from the sensor array 102, the PPG sensor 108, and the user interface 106 and, using the received data, may detect, classify, monitor, and/or predict events of interest. The processor device 104 includes communication circuitry 256, a microprocessor 258, and a memory device 260. The microprocessor 258 may be any known microprocessor configurable to execute the routines necessary for detecting, classifying, monitoring, and/or predicting events of interest, including, by way of example and not limitation, general purpose microprocessors (GPUs), RISC microprocessors, digital signal processors (DSPs), application specific integrated circuits (ASICs), and field-programmable gate arrays (FPGAs).

The communication circuitry 256 may be any transceiver and/or receiver/transmitter pair that facilitates communication with the various devices from which the processor device 104 receives data and/or transmits data. The communication circuitry 256 is communicatively coupled, in a wired or wireless manner, to each of the sensor array 102, the PPG sensor 108, the therapeutic device 255 (in embodiments implementing it), and the user interface 106. Additionally, the communication circuitry 256 is coupled to the microprocessor 258, which, in addition to executing various routines and instructions for performing analysis, may also facilitate storage in the memory 260 of data received, via the communication circuity 256, from the sensor array 102, the PPG sensor 108, the therapeutic device 255, and the user interface 106. In embodiments, the communication circuitry 256 may also communicate with other processors or devices, as will be described elsewhere in this specification.

The memory 260 may include both volatile memory (e.g., random access memory (RAM)) and non-volatile memory, in the form of either or both of magnetic or solid state media. In addition to an operating system (not shown), the memory 260 may store sensor array data 262 (i.e., EEG data) received from the sensor array 102, PPG data 267 received from the PPG sensor 108, and user report data 268 received from the user (e.g., patient, caregiver, etc.) via the user interface 106. In particular, where the condition and events relate to epilepsy, the user report data 268 may include reports from the user, received via the user interface 106, of: perceived seizures/epileptic events; characteristics or features of perceived seizures/epileptic events such as severity and/or duration, perceived effects on memory, or other effects on the individual's well-being (such as their ability to hold a cup or operate a vehicle); other types of physiological symptoms (e.g., headaches, impaired or altered vision, involuntary movements, disorientation, falling to the ground, repeated jaw movements or lip smacking, etc.); characteristics or features of other symptoms (e.g., severity and/or duration); medication ingestion information (e.g., medication types, dosages, and/or frequencies/timing); perceived medication side-effects; characteristics or features of medication side effects (e.g., severity and/or duration), and other user reported information (e.g., food and/or drink ingested, activities performed (e.g., showering, exercising, working, brushing hair, etc.), tiredness, stress levels, etc.), as well as the timing of each. Where the condition relates to a sleep disorder, the user report data 268 may include reports from the user, received via the user interface 106, of: perceived tiredness or lethargy, perceived wakefulness (e.g., at night), perceived sleep apnea events such as waking up gasping for breath, perceived sleep quality, perceived shortness of breath, cognitive decrement or slowness after poor sleep, as well as the severity, speed of onset, and other factors related to each of these. Where the condition relates to a vestibular or cochlear disorder, the user report data 268 may include reports from the user, received via the user interface 106, of: perceived changes in hearing threshold, perceived cognitive effort required to hear, and perceived dizziness or vertigo, as well as the severity, speed of onset, and other factors related to each of these.

As will be described in greater detail below, in the sub-system 104A, the memory 260 may also store a model 270 for detecting and predicting both events and the effects of those events, according to a set of feature values 272 extracted from the sensor array data 262, the PPG data 267, and the user report data 268. Classification results 274 (and, by extension, detected and predicted events and associated effects) output by the model 270 may be stored in the memory 260. A data pre-processing routine 271 may provide pre-processing of the sensor array data 262, the user report data 268, and the PPG data 267. As will be understood (and, in part, described below), the data pre-processing routine 271 may provide a range of pre-processing steps including, for example, filtering and extraction from the data of the feature values 272. Of course, it should be understood that wherever a routine, model, or other element stored in memory is referred to as receiving an input, producing or storing an output, or executing, the routine, model, or other element is, in fact, executing as instructions on the microprocessor 258. Further, those of skill in the art will appreciate that the model or routine or other instructions would be stored in the memory 260 as executable instructions, which instructions the microprocessor 258 would retrieve from the memory 260 and execute. Further, the microprocessor 258 should be understood to retrieve from the memory 260 any data necessary to perform the executed instructions (e.g., data required as an input to the routine or model), and to store in the memory 260 the intermediate results and/or output of any executed instructions.

In embodiments, the data pre-processing routine 271 may also extract from the sensor array data 262 and the PPG data 267, one or more biomarkers. The one or more biomarkers may be included among the feature values that are provided as inputs to the model 270, in embodiments, in order for the model 270 to output detected and/or classified events and associated effects to the classification results 274.

The data stored in the sensor array data 262, the PPG data 267, and the user report data 268 is stored with corresponding time stamps such that the data may be correlated between data types. For example, each value in the sensor array data 262 should have a corresponding time stamp such that the PPG data 266 and user report data 268 for the same time can be compared, allowing the various types of data to be lined up and analyzed for any given time period, and so that time relationships between events occurring and biomarkers present in the various types of data may be analyzed to look for relationships between them whether temporally concurrent or merely temporally related. With respect to the user report data 268, there may be multiple time stamps for any particular user report, including, for example, the time that the user filled out the user report and the time of the event or information (e.g., drug ingestion) that the user was reporting (as reported by the user).

Events need not be contemporaneous to be relevant or related, or to be feature values input into the model. Put another way, the model 270 may consider temporal relationships between non-contemporaneously recorded data in detecting, classifying, or predicting an event or the effects of an event. By way of example and not limitation, an electrical activity event (e.g., EEG signals) indicating a seizure may be classified as a particular type of event if preceded by the ingestion of medication, and as a different type of event if not preceded by the ingestion of the medication. Other examples of non-contemporaneous events preceding a seizure that are precursors are patient subjective reports of auras or optical lights, shortness of breath or increased cardiac pulse rate. Additionally, the system 100 and, in particular, the model 270, may identify pre- and/or post-event conditions, such as decreased blood oxygenation, dizziness, or other symptoms that are likely to occur according to patient history or other biomarkers present in the EEG data 262, the PPG data 267, and/or the user reports 268.

Of course, contemporaneous events may also be relevant. For example, EEG data indicative of a generalized tonic-clonic (i.e., grand mal) seizure, when accompanied contemporaneously by a drop in blood oxygenation as detected by the PPG sensor may indicate the immediate presence of an after-effect of the seizure or even of seizure-induced apnea.

Fig. 6C also depicts optional external processor devices 105, which may include, in various embodiments one or more caregiver devices 107A and one or more physician devices 107B. As will be described in greater detail below, the external devices 105 may receive alerts or alarms from the processor device 104 about predicted, occurring, or recently occurred events (e.g. seizures, sleep apnea desaturations, etc.), and may receive, in some embodiments, proposed treatment recommendations or requests for approval to implement adjustments to one or more settings of the therapeutic device 255.

The memory 260 may also store a treatment strategy routine 273, in embodiments. The treatment strategy routine 273 may include pre-programmed treatment strategies recommended or implemented according to the biomarkers extracted from the EEG data 262, the PPG data 267, the feature values 272, the user reports 268, and/or the classification results 274. For example, the treatment strategy routine 273 may be programmed to recommend to the patient or a caregiver, or to implement (e.g., via the treatment device 255), increased supplemental oxygen for the patient if the PPG data 267 show decreased blood oxygen levels, if the classification results 274 produced by the model 270 include that the patient has just suffered a seizure and that the likely effects of that seizure are decreased blood oxygen levels, or if the classification results 274 include a prediction that the patient is about to have a seizure that is likely to result in decreased blood oxygen levels. As another example, the biomarkers extracted as feature values 272 from the EEG data 262 and the PPG data 267 may result in classification results 274 indicative of an impending seizure. The treatment strategy routine 273 may be programmed to adjust the parameters of a vagus nerve stimulator (VNS) system (e.g., treatment device 255) in order to prevent the seizure or lessen the severity of the seizure. In still another example, the model 270 may, based on feature values 272 extracted from the EEG data 262 and the PPG data 267, output classification results 274 indicating that the patient is having frequent sleep apnea episodes. The treatment strategy routine 273 may be programmed to recommend to the patient that the patient increase the pressure on a CPAP device or adjust the settings on a hypoglossal nerve stimulation device or, in embodiments in which the processor device 104 is communicatively coupled to the therapeutic device 255 (e.g., the CPAP device, adaptive servo ventilation device, or the hypoglossal nerve stimulation device), to adjust the settings on the therapeutic device 255 directly to decrease the frequency or severity of the sleep apnea events.

As described above and throughout this specification, the interplay between biomarkers derived from the EEG data 262 and the PPG data 267 may provide insight into neurological, cardiac, respiratory, and even inflammatory function in the patient. Measurement of these functions can improve the detection of events and conditions and, through understanding temporal relationships between biomarkers that might presage certain events, can improve the prediction of these events and conditions. Measurement of these functions can also improve understanding of patient-specific physiological changes that result from the condition or the events associated with the condition.

Specifically, biomarkers that can be extracted from the PPG data 267 may improve clinical or sub-clinical seizure detection, as changes in biomarkers in the PPG data 267 may coincide or have specific temporal relationships with biomarkers in the EEG data 262. At the same time, biomarkers in the PPG data 267 may be used to determine if changes to blood oxygen levels, and cardiac and respiratory function are related to seizure activity or drug side effects, which can assist in the optimization of treatment dose and timing to maximize therapeutic effect while minimizing side-effects. In addition, while biomarkers in the EEG data 262 may provide sufficient data, in some instances, to determine whether a seizure is occurring or has occurred, the additional cardiac-related biomarker information extracted from the PPG data 267 may inform whether the seizure is cardiac induced or, instead, is causing cardiac changes (i.e., may determine a cause-effect relationship between seizure events and cardiac function). PPG-related biomarkers may also help sub-classify clinical and sub-clinical seizures as those that are ictal hypoxemic and those that are not.

Biomarkers extracted from the PPG data 267 may also be used to characterize blood oxygenation, cardiac, and respiratory changes before, at the onset of, during, and after seizures. Characterizing these changes and, in particular, changes before or at the onset of seizure events in a particular patient or group of patients can facilitate or improve prediction of seizure events, potentially giving patients time to prepare (e.g., situate themselves in safer positions or surroundings, alert caregivers or bystanders, etc.) or even to take action that might prevent or lessen the severity of an impending seizure event, while characterizing changes before, during, and after events may allow patients and caregivers to take action to prevent or lessen the severity of the effects of a seizure event on short- and long-term patient well-being. These seizure-related effects on the patient can include respiratory changes that include obstructive apnea, tachypnea, bradypnea, and hypoxemia.

Quantifying the impact of events (seizure events, apnea events, vestibular events, etc.) on vital functions such as respiration and cardiac functions, as well as on recovery and long-term impact to patient health, especially paired with prediction (pre-ictal detection) and characterization of events, can allow patients, caregivers, and physicians to mitigate these impacts. In particular, qualitative and quantitative detection and characterization of post-ictal state (for seizures) or after-effects of events related to other conditions (e.g., sleep apnea events), when combined with prediction and/or detection of the events themselves can lead to therapies and strategies for reducing the clinical impact of the events and improving the overall well-being of the patients.

Additionally, the combination of biomarkers extracted from the PPG data 267 and the EEG data 262 may facilitate detection of SUDEP (sudden unexplained death in epilepsy) or SUDEP-precipitating events. That is, by monitoring the patient's heart-rate, blood pressure, and/or blood oxygenation, in combination with EEG data 262, the system 100 may detect and/or predict a SUDEP or SUDEP-precipitating event. In so doing, the system 100 may generate alerts or alarms for the patient, for the caregivers or physicians of the patient, or for bystanders. The system 100 may also activate connected therapeutic devices such as neurostimulators or cardiac defibrillators to counter or prevent SUDEP events when they are detected or predicted.

Patients, particularly those suffering from epilepsy and/or sleep disorders, can also benefit from characterization of sleep quality. The systems and methods described herein utilize biomarkers extracted from the PPG data 267, alone or with the EEG data 262, to characterize sleep quality (e.g., capture a sleep quality score). The scoring can be combined with indicators of sleep cycle data in the EEG data 262. A more holistic representation of the sleep quality for the individual can be developed by including information from the user report data 268 entered by the patient via the user interface 106 after the patient wakes. The sleep quality score for the patient can be used, for example by the treatment strategy routine 273, to make recommendations to caregivers or physicians regarding the adjustment of dosage and timing of medication or other treatments (e.g., VNS) such that treatment is titrated to reach clinical efficacy but move away from the dosage impacting sleep quality. In some embodiments in which the processor 104 is communicatively coupled to a therapeutic device 255, the treatment strategy routine 273 may implement adjustments to the therapeutic device. Such implementation may, in some embodiments, require the processor device 104 to communicate first with a physician (e.g., sending a request or alert to a device in the possession of the physician) to receive confirmation of the adjustment.

The systems and methods described herein may utilize the novel combinations of biomarkers derived from the EEG data 262 and the PPG data 267 to create forecasting models that provide outputs that forecast not only particular events (e.g., seizures, apnea desaturations, etc.), but also forecast the severity of the event, ictal cardiac and respiratory changes, types of ictal respiratory changes (e.g., central apnea, hypoxemia, etc.), likely impact to post-ictal well-being of the individual, clustering of events, systemic inflammatory markers (such as those that can lead to middle or inner ear inflammation, cochlear or vestibular dysfunction, etc.), and sleep apnea events, among others. As alluded to, the forecasting of these events and effects can allow the system 100 to recommend and/or implement interventions and treatments that can reduce the severity of the event or its effects, reduce the clinical impact of the event or effects on the patient's well-being, or hasten the patient's recovery from the event or its effects.

In view of these considerations, it is considered that while some objectives of the system 100 may be achieved using the model 270 according to known data about the patient and/or the condition, other objectives of the system 100 must necessarily implement a trained artificial intelligence (AI) model to achieve maximum benefit.

Throughout the remainder of this specification, the phrase "evaluative functions" will be used to refer to the collective potential outputs of the various embodiments including at least: detecting and/or classifying events that are occurring; detecting and/or classifying events that have occurred; predicting and/or classifying events that are about to occur; detecting and/or classifying measures of pre-event patient well-being related to events that are occurring, have occurred, or are predicted to occur; detecting and/or classifying measures of intra-event patient well-being related to events that are occurring, have occurred, or are predicted to occur; detecting and/or classifying measures of post-event patient well-being related to events that are occurring, have occurred, or are predicted to occur.

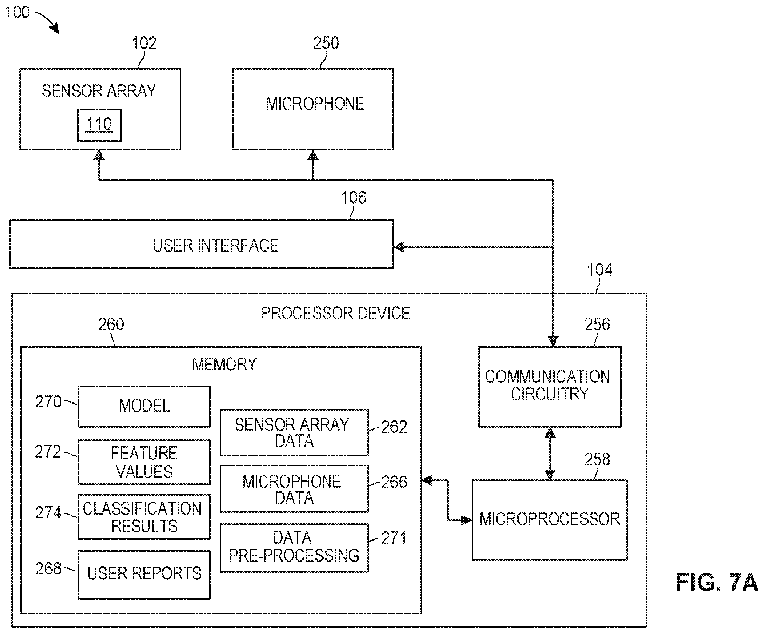

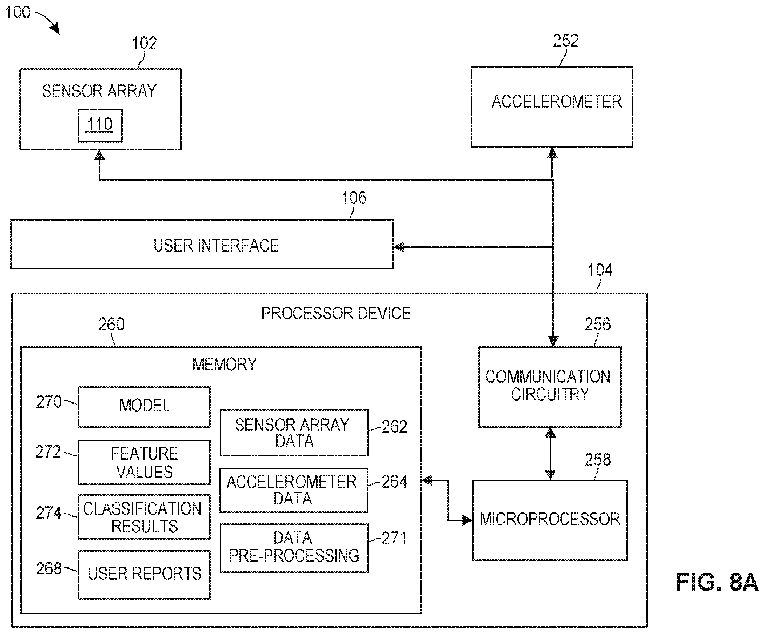

Figs. 7A-7B and 8A-8B are block diagrams depicting exemplary alternative embodiments to that depicted in Figs. 6A-6B. In Figs. 7A-7B, the system 100 includes all of the same components as depicted in Figs. 6A-6B, with the exception of the accelerometer 252 and the corresponding accelerometer data 264 stored in the memory 260. That is, in embodiments such as those depicted in Figs. 7A-7B, the accelerometer data 264 may not be necessary or required in order to detect or classify events, or to do so with sufficient accuracy for diagnostic and/or treatment purposes. Of course, the model 270 depicted in Figs. 7A-7B would be modified relative to the model 270 depicted in Figs. 6A-6B, to account for the lack of accelerometer data 264. Similarly, in Figs. 8A-8B, the system 100 includes all of the same components as depicted in Figs. 6A-6B, with the exception of the microphone 250 and the corresponding microphone data 266 stored in the memory 260. That is, in embodiments such as those depicted in Figs. 8A-8B, the microphone data 266 may not be necessary or required in order to detect or classify events, or to do so with sufficient accuracy for diagnostic and/or treatment purposes. Of course, the model 270 depicted in Figs. 8A-8B would be modified relative to the model 270 depicted in Figs. 6A-6B, to account for the lack of microphone data 266.

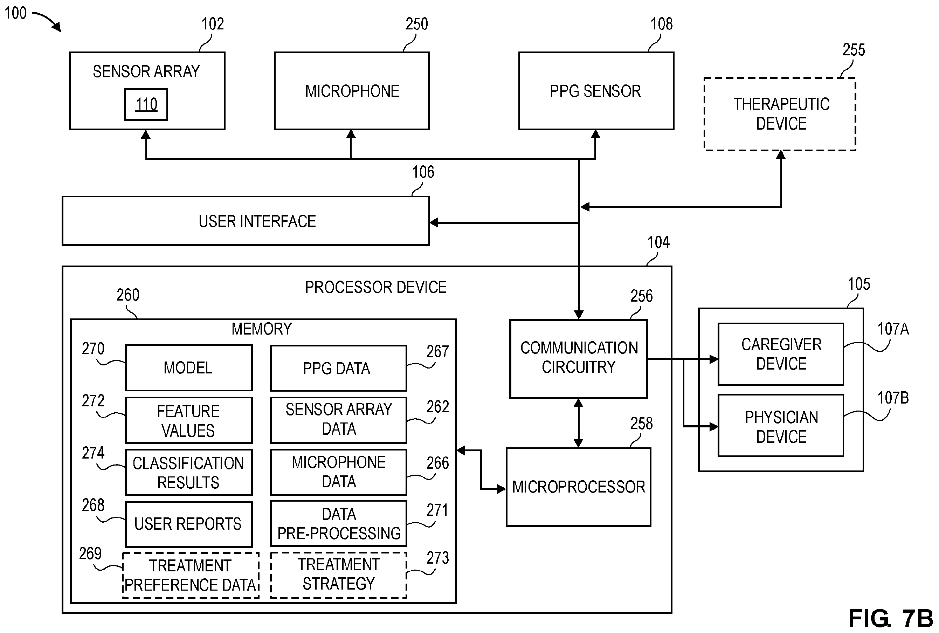

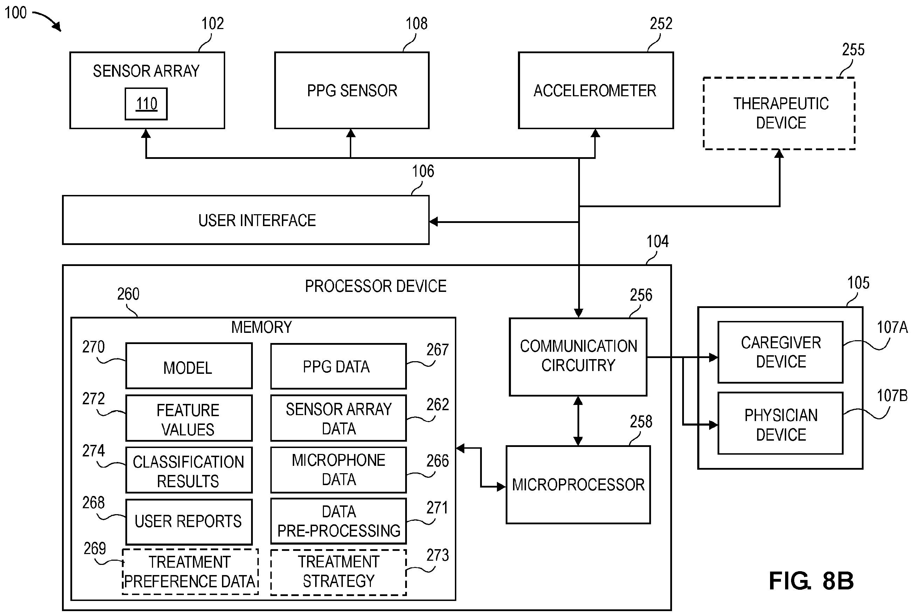

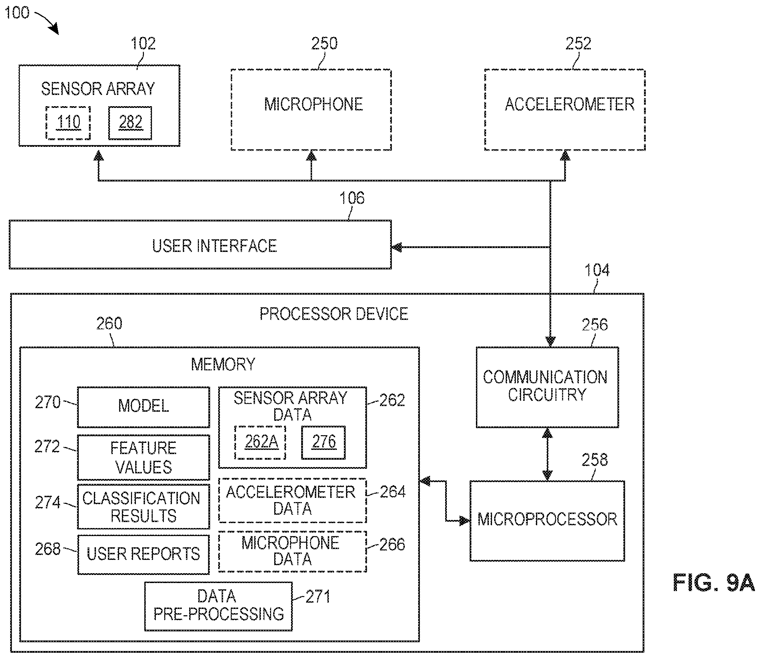

In embodiments, one or more chemical biomarkers may be detected within the system 100, in addition to or instead of other biomarkers determined by the sensor array 102, the PPG sensor 108 (in Figs. 7B and 8B), the microphone 250, and/or the accelerometer 252. Figs. 9A-9B are block diagrams of such embodiments. In Figs. 9A-9B, the microphone 250, the accelerometer 252, and the data 266 and 264 in memory 260 associated, respectively, with each, are depicted in dotted lines to denote that they are optional (e.g., corresponding to Figs. 7A-7B, 8A-8B, and/or an embodiment that includes only the sensor array 102). In the embodiments depicted in Figs. 9A-9B, the sensor array 102, in addition to or instead of electrode devices 110 detecting electrical activity of the brain, includes one or more biochemical sensors 282 that produce an electrical signal in response to detected chemical activity. The biochemical sensors convert a chemical or biological quantity into an electrical signal that can be provided from the sensor array 102 to the processor device 104 for storage in the memory 260 as chemical biomarker data 276. As a person of skill in the art would appreciate, the biochemical sensors 282 include a chemical sensitive layer that responds to an analyte molecule to cause an electrical signal to be generated by a transducer. The biochemical sensors 282 may include any combination of one or more sensor types including of conductimetric, potentiometric, amperometric, or calorimetric sensors. The biochemical sensors 282 may also or alternatively include one or more "gene chips," configured to measure activity associated with various biochemical or genetic "probes" to determine presence and/or concentration of molecules of interest. Of course, the model 270 depicted in Figs. 9A-9B would be modified relative to the model 270 depicted in Figs. 6A-6C, 7A-7B, and 8A-8B, to account for the biochemical sensors 282 and the chemical biomarker data 276 (in addition to or instead of the electrode devices 110 and associated electrode data 262A).

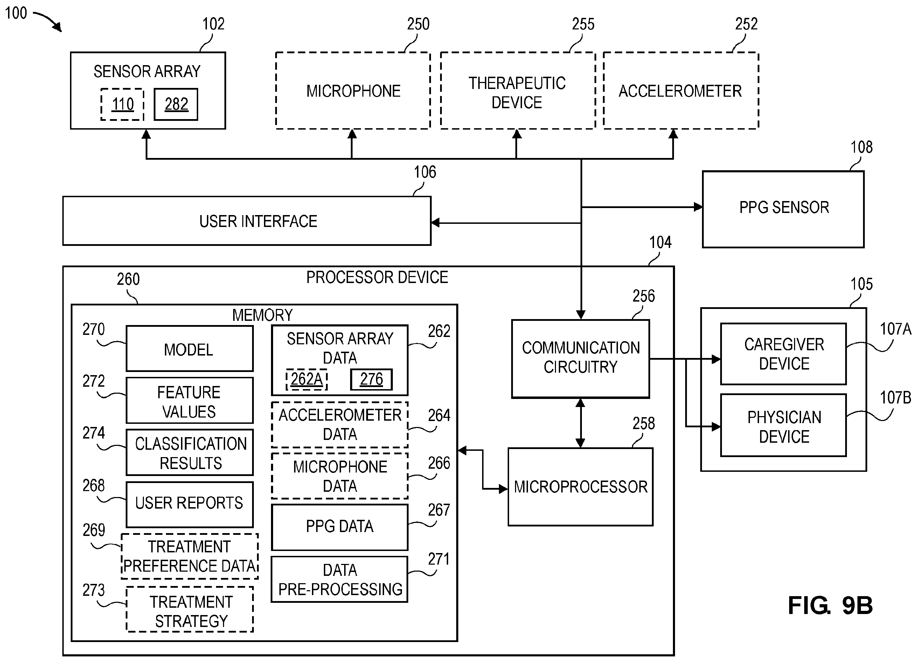

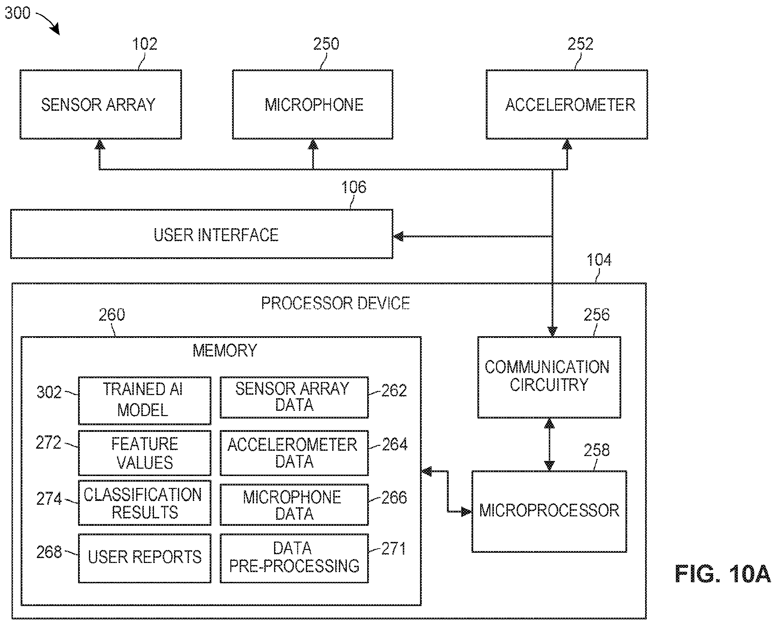

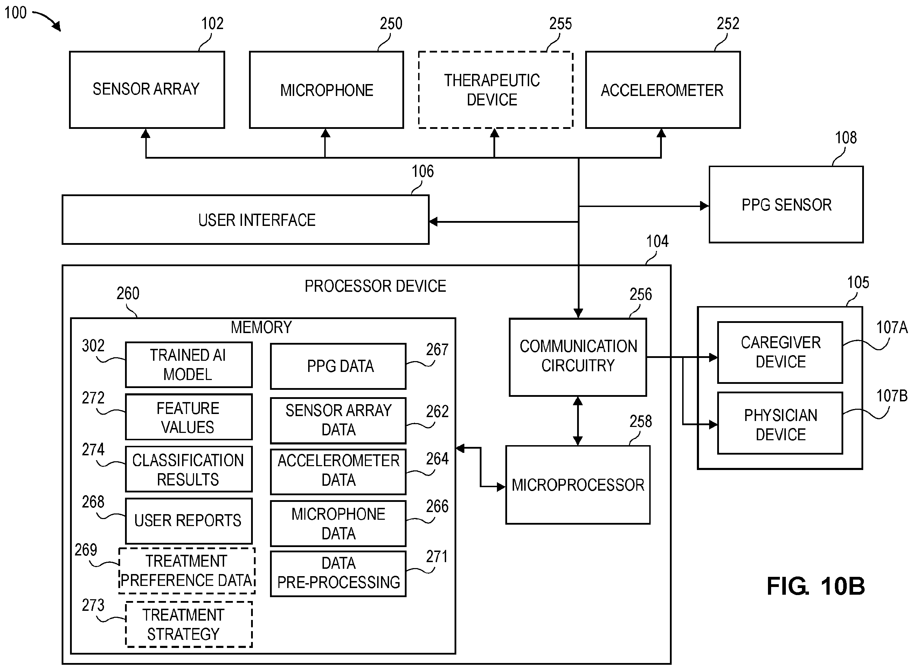

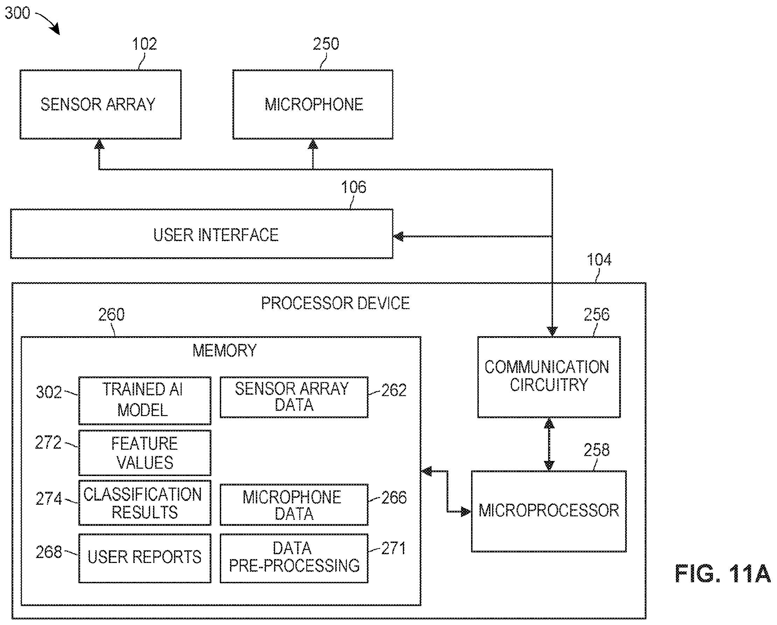

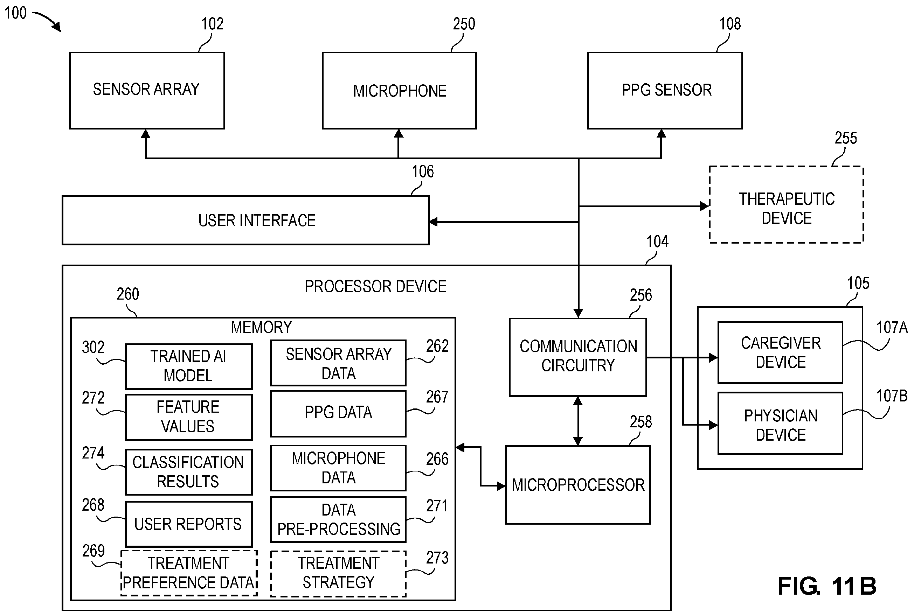

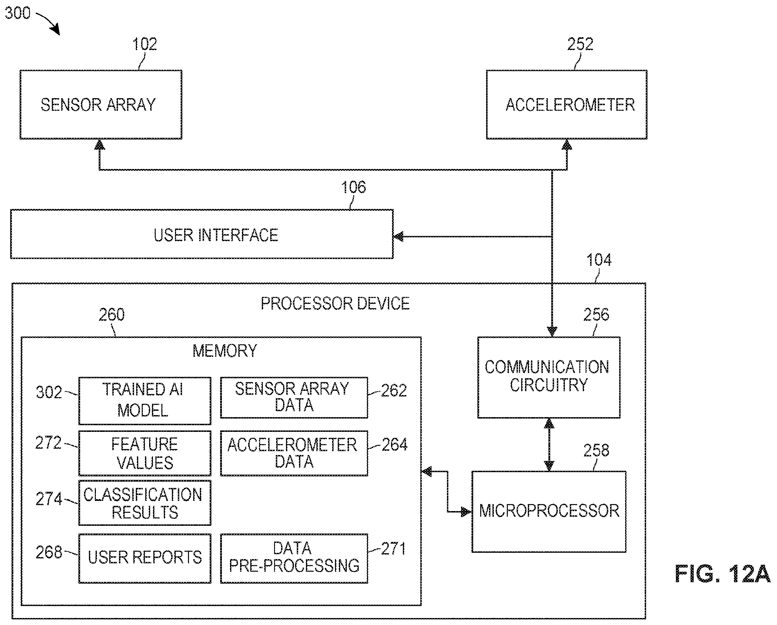

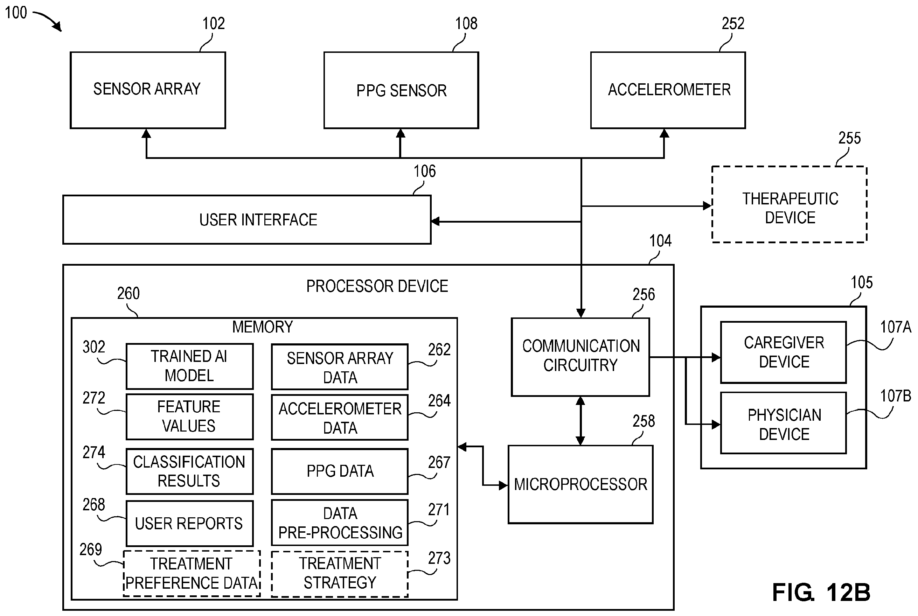

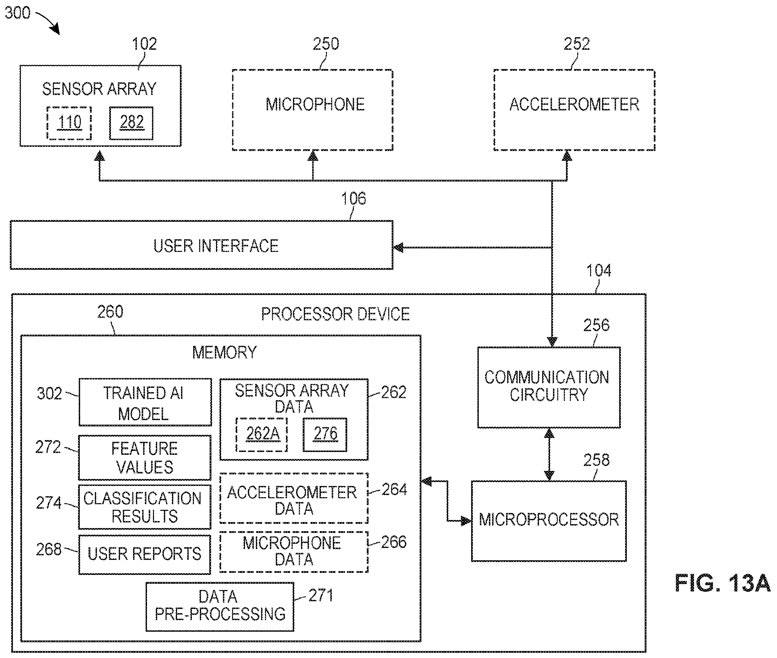

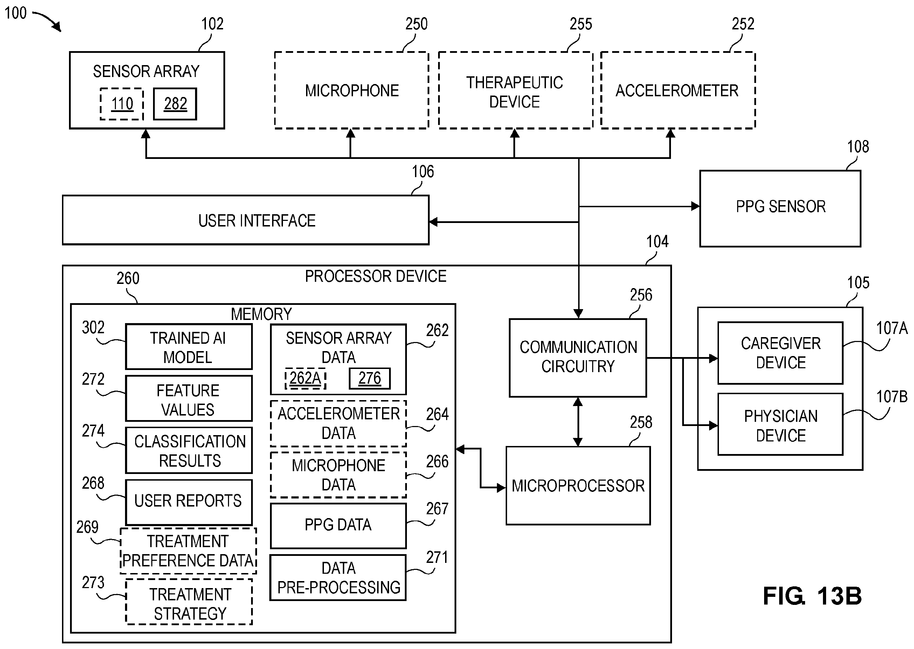

Figs. 10A-13D are block diagrams of an example system 300 similar to the system 100 of Figs. 6A-9B, but which include a trained artificial intelligence (AI) model 302 instead of the model 270 based on a static algorithm. That is, Figs. 10A and 10B correspond generally to Figs. 6A and 6B, respectively; Figs. 11A and 11B correspond generally to Figs. 7A and 7B, respectively; Figs. 12A and 12B correspond generally to Figs. 8A and 8B, respectively, and Figs. 13A and 13B corresponds generally to Figs. 9A and 9B, respectively; with the only difference between the system 100 and the system 300 in respective figures being the inclusion of the trained Al model 302 rather than the model 270 based on a static algorithm. The system 300, as depicted in Figs. 10A-13B is the same in all respects as in Figs. 6A-9B (respectively), above, except that the trained AI model 302 is created using Al algorithms to search for patterns in training data and, upon implementation in the processor device 104, to receive the sensor array data 262 and the PPG data 267 (in the embodiments of Figs. 10B, 11B, 12B, and 13B), and the accelerometer data 264 and/or microphone data 266 and/or user reports 268 and to determine from those data feature values 272 from which the trained Al model 302 may detect events and classify events to provide the classification results 274. Like the static model 270, the trained Al model 302 may consider temporal relationships between non-contemporaneous events and/or biomarkers in detecting and/or classifying an event. The trained Al model 302 may also identify clustering of events, or the cyclical nature of events, in embodiments.

The trained Al model 302 may be created by an adaptive learning component configured to "train" an Al model (e.g., create the trained Al model 302) to detect and classify events of interest using as inputs raw or pre-processed (e.g., by the data pre-processing routine 271) data from the sensor array data 262 and the PPG data 267 (in the embodiments of Figs. 10B, 11B, 12B, and 13B) and, optionally, the user reports 268 and/or accelerometer data 264 and/or microphone data 266. As described herein, the adaptive learning component may use a supervised or unsupervised machine learning program or algorithm. The machine learning program or algorithm may employ a neural network, which may be a convolutional neural network (CNN), a deep learning neural network, or a combined learning module or program that learns in two or more features or feature datasets in a particular area of interest. The machine learning programs or algorithms may also include natural language processing, semantic analysis, automatic reasoning, regression analysis, support vector machine (SVM) analysis, decision tree analysis, random forest analysis, K-Nearest neighbor analysis, naïve Bayes analysis, clustering, reinforcement learning, and/or other machine learning algorithms and/or techniques. Machine learning may involve identifying and recognizing patterns in existing data (i.e., training data) such as epileptiform activity in the EEG signal be this a clinical relevant epileptic seizure or inter-ictal activity such as spiking, in order to facilitate making predictions for subsequent data, such as epileptic seizure events, inter-ictal spiking clusters, or drug side-effect responses and magnitudes.

The trained Al model 302 may be created and trained based upon example (e.g., "training data") inputs or data (which may be termed "features" and "labels") in order to make valid and reliable predictions for new inputs, such as testing level or production level data or inputs. In supervised machine learning, a machine learning program operating on a server, computing device, or other processor(s), may be provided with example inputs (e.g., "features") and their associated, or observed, outputs (e.g., "labels") in order for the machine learning program or algorithm to determine or discover rules, relationships, or other machine learning "models" that map such inputs (e.g., "features") to the outputs (e.g., "labels"), for example, by determining and/or assigning weights or other metrics to the model across its various feature categories. Such rules, relationships, or other models may then be provided subsequent inputs in order for the model, executing on the server, computing device, or other processor(s), to predict, based on the discovered rules, relationships, or model, an expected output.

In unsupervised learning, the server, computing device, or other processor(s), may be required to find its own structure in unlabeled example inputs, where, for example, multiple training iterations are executed by the server, computing device, or other processor(s) to train multiple generations of models until a satisfactory model (e.g., a model that provides sufficient prediction accuracy when given test level or production level data or inputs) is generated. The disclosures herein may use one or both of such supervised or unsupervised machine learning techniques.

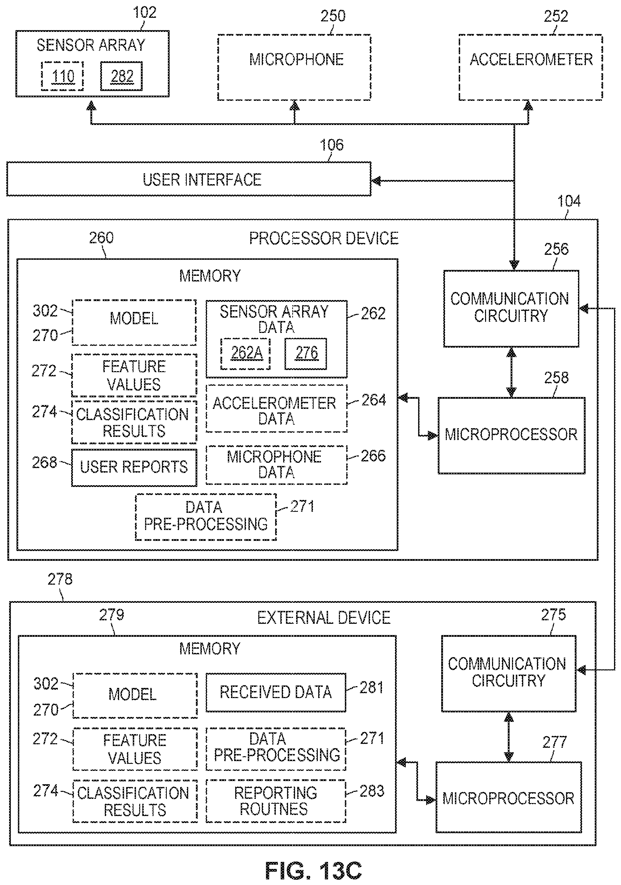

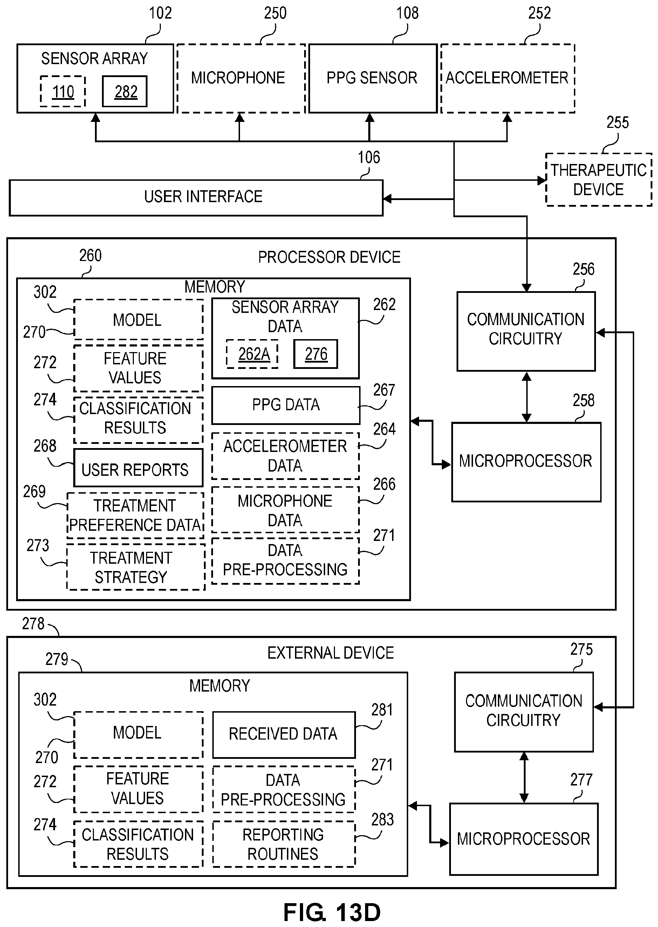

Figs. 13C and 13D are block diagrams depicting additional example embodiments, in which the detection and classification of events take place on a device other than the processor device 104 and, specifically, on an external device 278. In the embodiments depicted in Figs. 13C and 13D, it is contemplated that the models detecting and classifying the events of interest may be either the static model 270 or the trained Al model 302 and, as a result, Figs. 13C and 13D illustrate an alternate embodiments of Figs. 6A-9B and of Figs. 10A-13B. In the embodiments contemplated within Figs. 13C and 13D, the processor device 104 generally collects the data from the sensor array 102, the PPG sensor 108 (in the embodiments of Fig. 13D), the user interface 106 and, if present, the microphones 250 and/or accelerometers 252. These data are stored in the memory 260 of the processor device 104 as the sensor array data 262, the PPG data 267 (in the embodiments of Fig. 13D), the user report data 268, the microphone data 266, and the accelerometer data 264, respectively. While the processor device 104 may be equipped to perform the modeling - that is may have stored in the memory 260 the model 270 or 302 and the data pre-processing routine(s) 271, and be configured to analyze the various data to output feature values 272 and classification results 274 - in the embodiments contemplated by Figs. 13C and 13D, this functionality is optional. Instead, the microprocessor 258 may be configured to communicate with the external device 278 such that the external device 278 may perform the analysis.

The external device 278 may be a workstation, a server, a cloud computing platform, or the like, configured to receive data from one or more processor devices 104 associated with one or more respective patients. The external device 278 may include communication circuitry 275, coupled to a microprocessor 277 that, in turn, is coupled to a memory 279. The microprocessor 277 may be any known microprocessor configurable to execute the routines necessary for detecting and classifying events of interest, including, by way of example and not limitation, general purpose microprocessors (GPUs), RISC microprocessors, digital signal processors (DSPs), application specific integrated circuits (ASICs), and field-programmable gate arrays (FPGAs).

The communication circuitry 275 may be any transceiver and/or receiver/transmitter pair that facilitates communication with the various devices from or to which the external device 278 receives data and/or transmits data. The communication circuitry 256 is coupled to the microprocessor 277, which, in addition to executing various routines and instructions for performing analysis, may also facilitate storage in the memory 279 of data received, via the communication circuity 275, from the processor devices 104 of the one or more patients.

The memory 279 may include both volatile memory (e.g., random access memory (RAM)) and non-volatile memory, in the form of either or both of magnetic or solid state media. In addition to an operating system (not shown), the memory 279 may store received data 281 received from the processor devices 104, including the sensor array data 262, the accelerometer data 264 received from the accelerometer(s) 252, the microphone data 266 received from the microphone(s) 250, and user report data 268 received from the user via the user interface 106.

Like the processor device 104, the external device 278 may have, stored in its memory 279, the static model 270 or the trained Al model 302, as well as data pre-processing routines 271. The microprocessor 277 may execute the data pre-processing routines 271 to refine, filter, extract biomarkers from, etc. the received data 281 and to output feature values 272 (which, in embodiments, include biomarkers or relationships between biomarkers). The microprocessor 277 may also execute the model 270, 302, receiving as inputs the feature values 272 and outputting classification results 274. One or more reporting routines 283 stored on the memory 279, when executed by the microprocessor 277, may facilitate outputting reports for use by the patient(s) or by medical personnel, such as physicians, to review the data and or treat the patient(s).

The embodiments depicted in Figs. 13C and 13D also contemplate that, even in embodiments in which the processor device 104 executes the model 270 or 302 to produce classification results, the processor device 104 may communicate the classification results 274, as well as the data 262, 264, 266, 267, 268 upon which the classification results are based, to the external device 278. The external device 278 may receive such data for one or more patients, and may store the data for those patients for later viewing or analysis by the patient(s), physicians, or others, as necessary. In embodiments in which the external device 278 performs analysis for multiple patients, or for which the external device 278 receives from multiple processor devices 104 data of multiple patients, the external device 278 may store the received data 281, the classification results 274, and the feature values 272 for each patient separately in the memory.

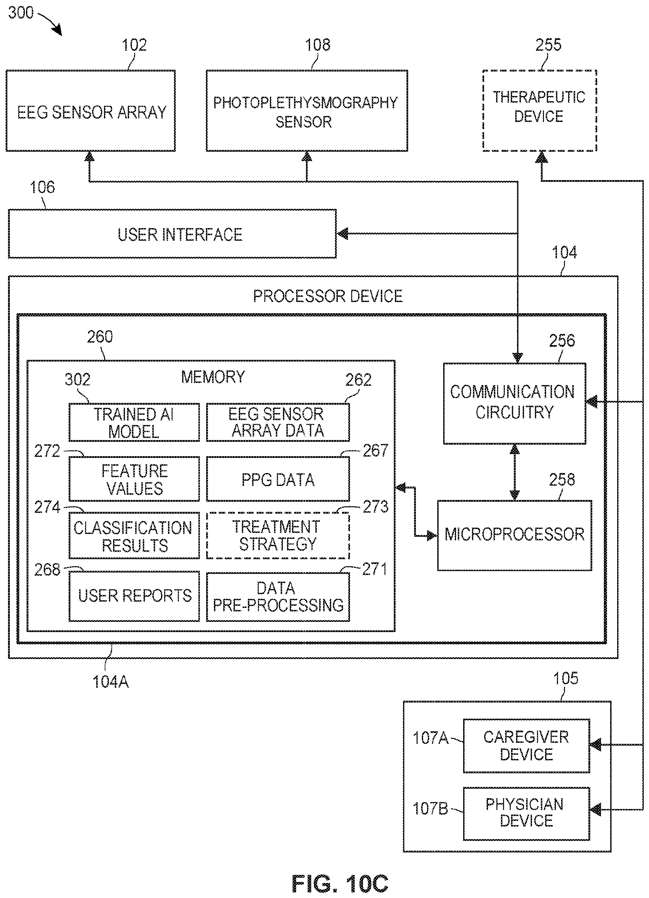

Fig. 10C is a block diagram of an example system 300 similar to the system 100 of Fig. 6C, but which includes a trained artificial intelligence (AI) model 302 instead of the model 270 based on a static algorithm. That is, Fig. 10C corresponds generally to Fig. 6C, with the only difference between the system 100 and the system 300 in the respective figures being the inclusion of the trained Al model 302 rather than the model 270 based on a static algorithm. The system 300, as depicted in Fig. 10C is the same in all respects as in Fig. 6C, above, except that the trained Al model 302 is created using Al algorithms to search for and identify patterns in training data and, upon implementation in the processor device 104, to receive the sensor array data 262 and the PPG data 267 and/or user reports 268 and to determine from those data feature values 272 from which the trained Al model 302 may perform the evaluative functions to determine the classification results 274, the output of which may be used by the treatment strategy routine 273 to recommend or implement treatments. Like the static model 270, the trained Al model 302 may consider temporal relationships between non-contemporaneous events and/or biomarkers in performing the evaluative functions.

The trained Al model 302 may be created by an adaptive learning component configured to "train" an Al model (e.g., create the trained Al model 302) to detect and classify events of interest (i.e., perform the evaluative functions) using as inputs raw or pre-processed (e.g., by the data pre-processing routine 271) data from the sensor array data 262 and, optionally, the user reports 268, and PPG data 267. As described herein, the adaptive learning component may use a supervised or unsupervised machine learning program or algorithm. The machine learning program or algorithm may employ a neural network, which may be a convolutional neural network (CNN), a deep learning neural network, or a combined learning module or program that learns in two or more features or feature datasets in a particular area of interest. The machine learning programs or algorithms may also include natural language processing, semantic analysis, automatic reasoning, regression analysis, support vector machine (SVM) analysis, decision tree analysis, random forest analysis, K-Nearest neighbor analysis, naïve Bayes analysis, clustering, reinforcement learning, and/or other machine learning algorithms and/or techniques. Machine learning may involve identifying and recognizing patterns in existing data (i.e., training data) such as temporal correlations between biomarkers in the EEG data 262 and the PPG data 267, in order to facilitate making predictions for subsequent data.

The trained Al model 302 may be created and trained based upon example (e.g., "training data") inputs or data (which may be termed "features" and "labels") in order to make valid and reliable predictions for new inputs, such as testing level or production level data or inputs. In supervised machine learning, a machine learning program operating on a server, computing device, or other processor(s), may be provided with example inputs (e.g., "features") and their associated, or observed, outputs (e.g., "labels") in order for the machine learning program or algorithm to determine or discover rules, relationships, or other machine learning "models" that map such inputs (e.g., "features") to the outputs (e.g., "labels"), for example, by determining and/or assigning weights or other metrics to the model across its various feature categories. Such rules, relationships, or other models may then be provided subsequent inputs in order for the model, executing on the server, computing device, or other processor(s), to predict, based on the discovered rules, relationships, or model, an expected output.

In unsupervised learning, the server, computing device, or other processor(s), may be required to find its own structure in unlabeled example inputs, where, for example, multiple training iterations are executed by the server, computing device, or other processor(s) to train multiple generations of models until a satisfactory model (e.g., a model that provides sufficient prediction accuracy when given test level or production level data or inputs) is generated. The disclosures herein may use one or both of such supervised or unsupervised machine learning techniques.

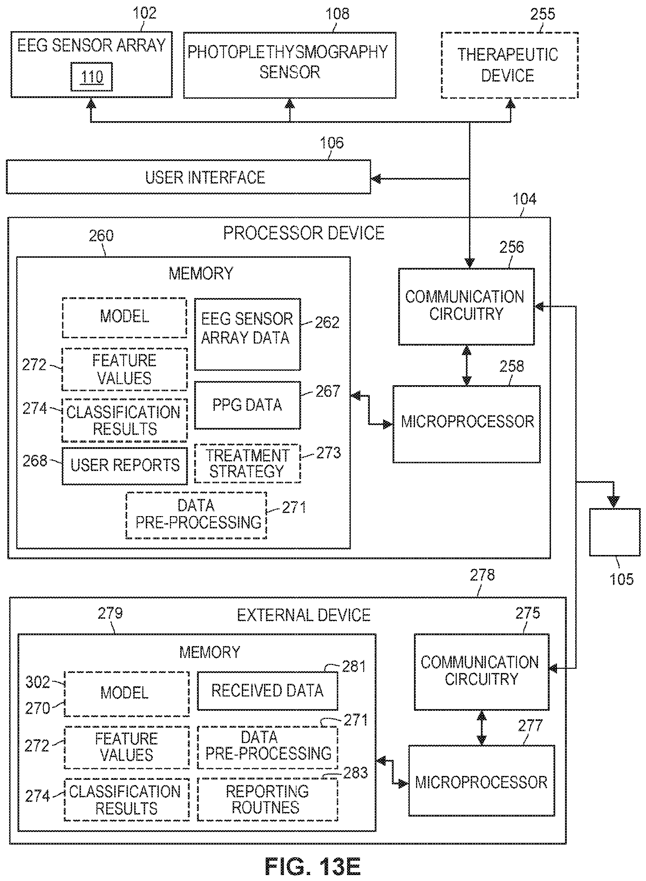

Fig. 13E is a block diagram depicting another example embodiment, in which the evaluative functions take place on a device other than the processor device 104 and, specifically, on an external device 278. In the embodiments depicted in Fig. 13E, it is contemplated that the models performing the evaluative functions may be either the static model 270 or the trained Al model 302 and, as a result, Fig. 13E illustrates an alternate embodiment of Figs. 6C and 10C. In the embodiments contemplated within Fig. 13E, the processor device 104 generally collects the data from the sensor array 102, the user interface 106, and the PPG sensor 108. These data are stored in the memory 260 of the processor device 104 as the sensor array data 262, the user report data 268, the PPG data 267, respectively. While the processor device 104 may be equipped to perform the modeling - that is, may have stored in the memory 260 the model 270 or 302 and the data pre-processing routine(s) 271, and be configured to perform the evaluative functions to output feature values 272 and classification results 274 - in the embodiments contemplated by Fig. 13E, this functionality is optional. Instead, the microprocessor 258 may be configured to communicate with the external device 278 such that the external device 278 may perform the evaluative functions.

The external device 278 may be a workstation, a server, a cloud computing platform, or the like, configured to receive data from one or more processor devices 104 associated with one or more respective patients. The external device 278 may include communication circuitry 275, coupled to a microprocessor 277 that, in turn, is coupled to a memory 279. The microprocessor 277 may be any known microprocessor configurable to execute the routines necessary for producing the evaluative results, including, by way of example and not limitation, general purpose microprocessors (GPUs), RISC microprocessors, digital signal processors (DSPs), application specific integrated circuits (ASICs), and field-programmable gate arrays (FPGAs).

The communication circuitry 275 may be any transceiver and/or receiver/transmitter pair that facilitates communication with the various devices from or to which the external device 278 receives data and/or transmits data. The communication circuitry 256 is coupled to the microprocessor 277, which, in addition to executing various routines and instructions for performing analysis, may also facilitate storage in the memory 279 of data received, via the communication circuity 275, from the processor devices 104 of the one or more patients.

The memory 279 may include both volatile memory (e.g., random access memory (RAM)) and non-volatile memory, in the form of either or both of magnetic or solid state media. In addition to an operating system (not shown), the memory 279 may store received data 281 received from the processor devices 104, including the sensor array data 262 received from the sensor array 102, the PPG data 267 received from the PPG sensor 108, and user report data 268 received from the user via the user interface 106.

Like the processor device 104, the external device 278 may have, stored in its memory 279, the static model 270 or the trained Al model 302, as well as data pre-processing routines 271. The microprocessor 277 may execute the data pre-processing routines 271 to refine, filter, extract biomarkers from, etc. the received data 281 and to output feature values 272 (which, in embodiments, include biomarkers or relationships between biomarkers). The microprocessor 277 may also execute the model 270, 302, receiving as inputs the feature values 272 and outputting classification results 274. One or more reporting routines 283 stored on the memory 279, when executed by the microprocessor 277, may facilitate outputting reports for use by the patient(s) or by medical personnel, such as physicians, to review the data and or treat the patient(s).

The embodiments depicted in Fig. 13E also contemplate that, even in embodiments in which the processor device 104 executes the model 270 or 302 to produce classification results, the processor device 104 may communicate the classification results 274, as well as the data 262, 267, 266, 268 upon which the classification results are based, to the external device 278. The external device 278 may receive such data for one or more patients, and may store the data for those patients for later viewing or analysis by the patient(s), physicians, or others, as necessary. In embodiments in which the external device 278 performs analysis for multiple patients, or for which the external device 278 receives from multiple processor devices 104 data of multiple patients, the external device 278 may store the received data 281, the classification results 274, and the feature values 272 for each patient separately in the memory.

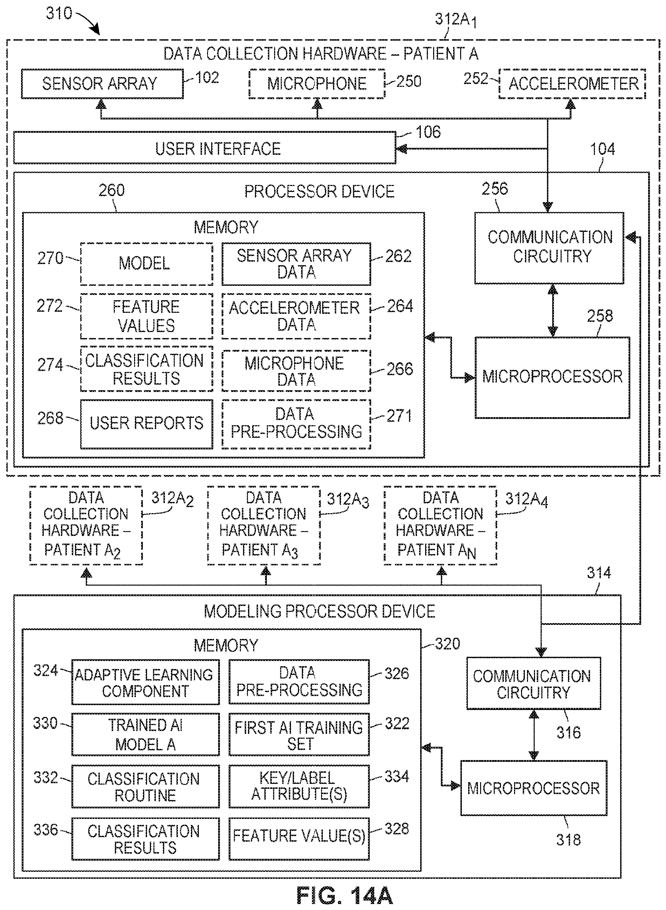

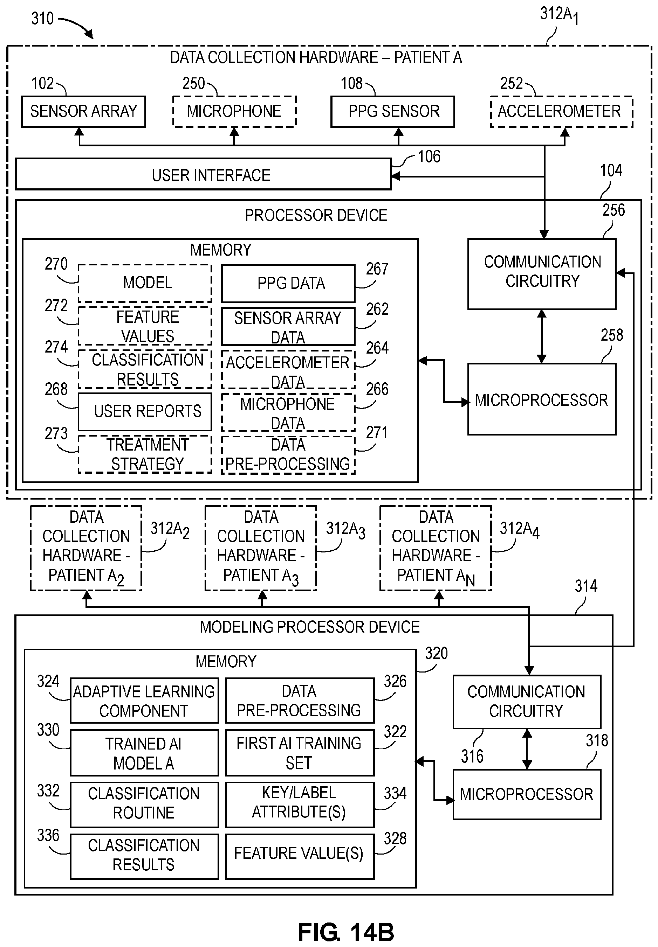

Figs. 14A-14B are block diagrams of example systems 310 for use in creating a trained Al model (e.g., the trained Al model 302). The systems 310 include one or more sets 312A₁-312A_{N} of data collection hardware similar to the system 100 of Figs. 6A-9B. That is, each set of data collection hardware 312A₁-312A_{N} includes a corresponding sensor array 102 (including electrode devices 110 and/or biochemical sensors 282, in various embodiments), a PPG sensor 108 (e.g., as in Figs. 6B, 6C, 7B, 8B, and 9B), and may include one or more microphones 250 and/or one or more accelerometers 252, and an interface 106. Each of the sets 312A₁-312A_{N} of data collection hardware also includes a respective processor device 104, including communication circuitry 256, a microprocessor 258, and a memory 260. As in the systems 100, the memory 260 of each set 312A₁-312A_{N} of data collection hardware stores at least the sensor array data 262, the PPG data 267 (for embodiments implementing the PPG sensor 108), and the user reports 268, and may also store accelerometer data 264 and/or microphone data 266, in embodiments in which a microphone 250 and/or an accelerometer 252 are implemented. Each of the sets 312A₁-312A_{N} of data collection hardware is associated with a corresponding patient A₁-A_{N} and, accordingly, each of the sets 312A₁-312A_{N} of data collection hardware collects data for a corresponding patient.

Unlike the systems 100 depicted in Figs. 6A-9B, however, the sets 312A₁-312A_{N} of data collection hardware in the system 310 need not necessarily include the model 270 stored in the memory 260, and the memory 260 need not necessarily store feature values 272 or classification results 274. That is, the sets 312A₁-312A_{N} of data collection hardware in the system 310 need not necessarily be capable of detecting and classifying events of interest, but may, in embodiments, merely act as collectors of, and conduits for, information to be used as "training data" for to create the trained AI model 302.

The data collected by the sets 312A₁-312A_{N} of data collection hardware may be communicated to a modeling processor device 314. The modeling processor device 314 may be any computer workstation, laptop computer, mobile computing device, server, cloud computing environment, etc. that is configured to receive the data from the sets 312A₁-312A_{N} of data collection hardware and to use the data from the sets 312A₁-312A_{N} of data collection hardware to create the trained Al model 302. The modeling processor device 314 may receive the data from the sets 312A₁-312A_{N} of data collection hardware via wired connection (e.g., Ethernet, serial connection, etc.) or wireless connection (e.g., mobile telephony, IEEE 802.11 protocol, etc.), directly (e.g., a connection with no intervening devices) or indirectly (e.g., a connection through one or more intermediary switches, access points, and/or the Internet), between the communication circuitry 256 of the processor device 104 and the communication circuitry 316 of the modeling processor device 314. Additionally, though not depicted in Figs. 14A-14B, the data may be communicated from one or more of the sets 312A₁-312A_{N} of data collection hardware to the modeling processor device 314 via storage media, rather than by respective communication circuitry. The storage media may include any known storage memory type including, by way of example and not limitation, magnetic storage media, solid state storage media, secure digital (SD) memory cards, USB drives, and the like.

The modeling processor device 314 includes communication circuitry 316, in embodiments in which it is necessary, a microprocessor 318, and a memory device 320. Though it should be understood, the microprocessor 318 may be one or more stand-alone microprocessors, one or more shared computing resources or processor arrays (e.g., a bank of processors in a cloud computing device), one or more multi-core processors, one or more DSPs, one or more FPGAs, etc. Similarly, the memory device 320 may be volatile or non-volatile memory, and may be memory dedicated solely to the modeling processor device 314 or shared among a variety of users, such as in a cloud computing environment.

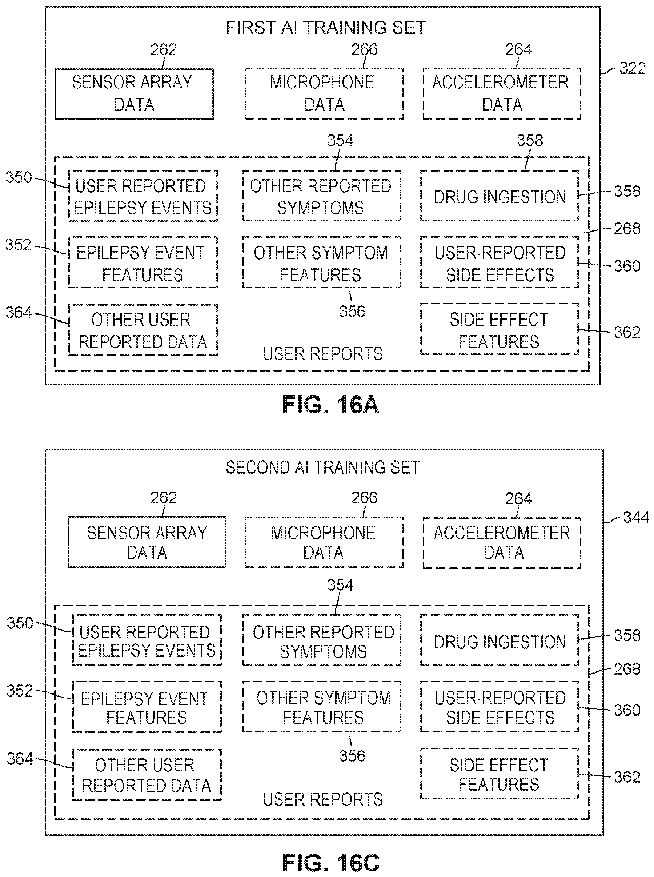

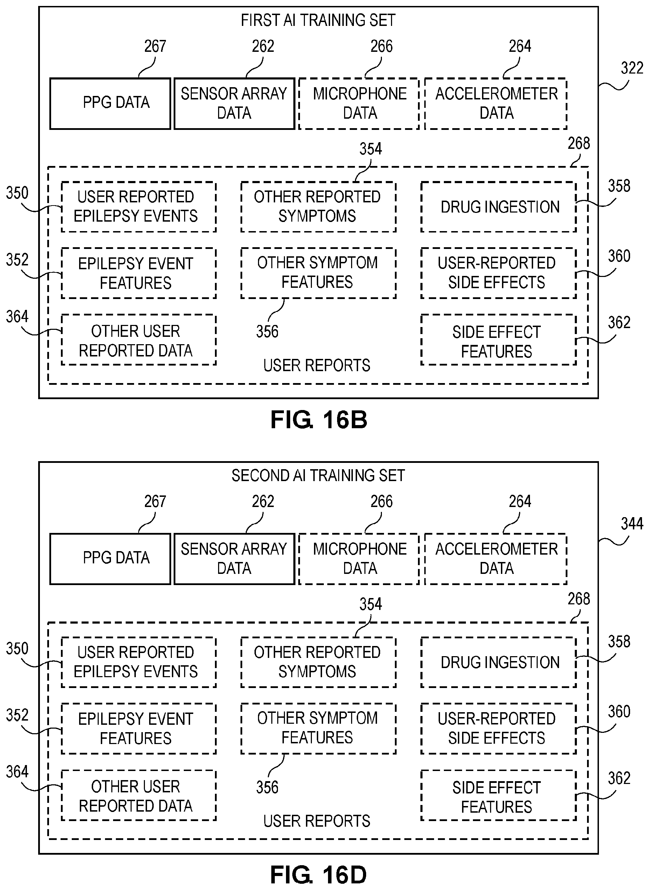

The memory 320 of the modeling processor device 314 may store as a first Al training set 322 (depicted in Figs. 16A, 16B) the sensor array data 262, the PPG data 267 (in embodiments implementing the PPG sensor 108), user report data 268 and optional accelerometer data 264 and/or microphone data 266 received from each of the sets 312A₁-312A_{N} of data collection hardware. As depicted in Figs. 16A, 16B, and by way of non-limiting example, the user report data 268 may include: perceived events 350; characteristics or features of perceived events 352 such as severity and/or duration, perceived effects on memory, or other effects on the individual's wellbeing (such as their ability to hold a cup or operate a vehicle); other types of physiological symptoms (e.g., headaches, impaired or altered vision, involuntary movements, disorientation, falling to the ground, repeated jaw movements or lip smacking, etc.) 354; characteristics or features of other symptoms 356 (e.g., severity and/or duration); medication ingestion information 358 (e.g., medication types, dosages, and/or frequencies/timing); perceived medication side-effects 360; characteristics or features of medication side-effects 362 (e.g., severity and/or duration), and other user reported information 364 (e.g., food and/or drink ingested, activities performed (e.g., showering, exercising, working, brushing hair, etc.), tiredness, stress levels, etc.), as well as the timing of each. An adaptive learning component 324 may comprise instructions that are executable by the microprocessor 318 to implement a supervised or unsupervised machine learning program or algorithm, as described above. One or more data pre-processing routines 326, when executed by the microprocessor 318, may retrieve the data in the first Al training set 322, which may be raw recorded data, and may perform various pre-processing algorithms on the data in preparation for use of the data as training data by the adaptive learning component 324. The pre-processing routines 326 may include routines for removing noisy data, cleaning data, reducing or removing irrelevant and/or redundant data, normalization, transformation, and extraction of biomarkers and other features. The pre-processing routines 326 may also include routines for detection of muscle activity in the electrical activity data and particularly in the EEG data 262 and/or PPG data 267 (in embodiments implementing the PPG sensor 108) by analyzing the spectral content of the signal and/or routines for selection of the channel or channels of the electrical activity data that have the best (or at least better, relatively) signal to noise ratios. The output of the pre-processing routines 326 is a final training set stored in the memory 320 as a set 328 of feature values.

In embodiments in which the adaptive learning component 324 implements unsupervised learning algorithms, the adaptive learning component 324, executed by the microprocessor 318, finds its own structure in the unlabeled feature values 328 and, therefrom, generates a first trained Al model 330.

In embodiments in which the adaptive learning component 324 implements supervised learning algorithms, the memory 320 may also store one or more classification routines 332 that facilitate the labeling of the feature values (e.g., by an expert, such as a neurologist, reviewing the feature values 328 and/or the first Al training set 322) to create a set of key or label attributes 334. The adaptive learning component 324, executed by the microprocessor 318, may use both the feature values 328 and the key or label attributes 334 to discover rules, relationships, or other "models" that map the features to the labels by, for example, determining and/or assigning weights or other metrics. The adaptive learning component 324 may output the set of rules, relationships, or other models as a first trained Al model 330.

Regardless of the manner in which the adaptive learning component 324 creates the first trained Al model 330, the microprocessor 318 may use the first trained Al model 330 with the first Al training set 322 and/or the feature values 328 extracted therefrom, or on a portion of the first Al training set 322 and/or a portion of the feature values 328 extracted therefrom that were reserved for validating the first trained Al model 330, in order to provide classification results 336 for comparison and/or analysis by a trained professional in order to validate the output of the model.

As should be apparent, the first Al training set 322 may include data from one or more of the sets 312A₁-312A_{N} of data collection hardware and, as a result, from one or more patients. Thus, the adaptive learning component 324 may use data from a single patient, from multiple patients, or from a multiplicity of patients when creating the first trained Al model 330. The population from which the patient or patients are selected may be tailored according to particular demographic (e.g., a particular type of suspected epilepsy, a particular age group, etc.), in some instances, or may be non-selective. In embodiments, at least some of the patients associated with the sets 312A₁-312A_{N} of data collection hardware from which the first Al training set 322 is created may be patients without any symptoms of the underlying condition(s) (e.g., epilepsy, sleep apnea, vestibular or cochlear disorders) and, as such, may serve to provide additional control data to the first Al training set 322.

In embodiments, the first trained Al model 330 may be transmitted to (or otherwise received - e.g., via portable storage media) to another set of data collection hardware (e.g., the system 300 depicted in any of Figs. 10A-13E). The set of data collection hardware may implement the first trained Al model 330 to provide classification results 274 based on data that was not part of the first Al training set 322 collected by the sets 312A₁-312A_{N} of data collection hardware or, alternatively, may simply collect additional data for use by the modeling processor device 314 to iterate the first trained Al model 330.

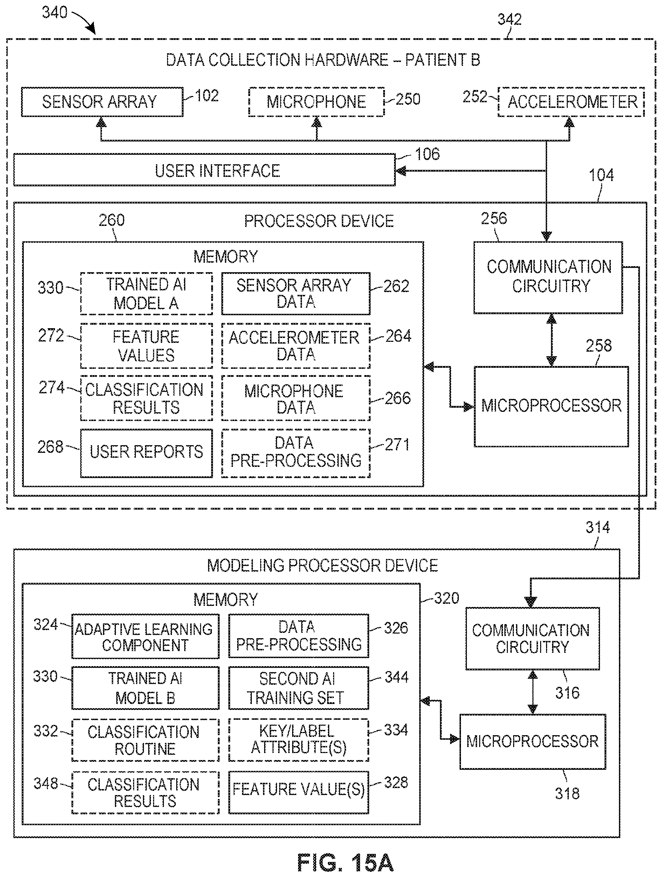

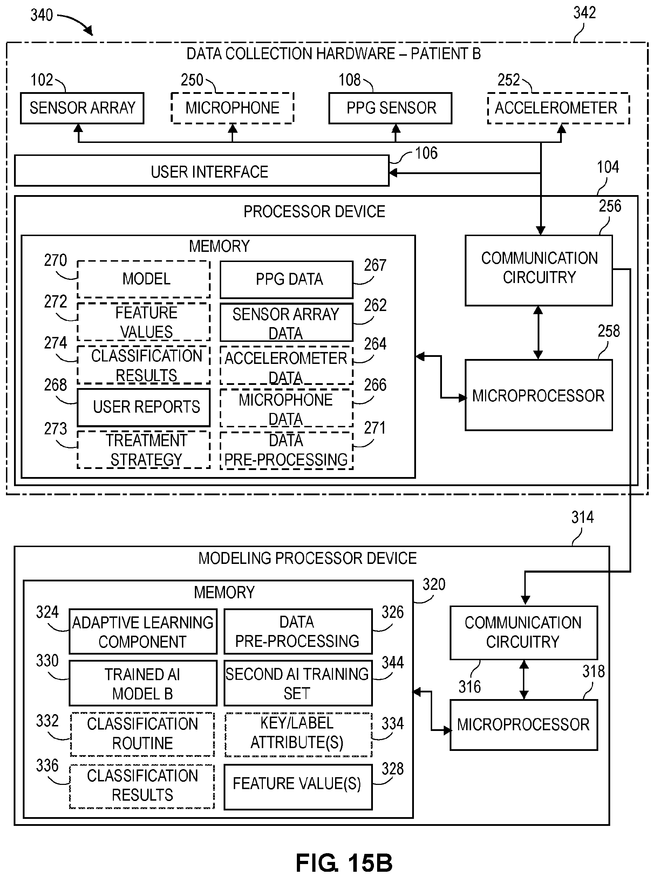

Figs. 15A and 15B depict such embodiments. In Figs. 15A and 15B, a system 340 includes a set 342 of data collection hardware for a patient. Like hardware previously described, the set of 342 of data collection hardware includes the sensor array 102, the PPG sensor 108 (in the embodiments of Fig. 15B), optionally the microphone 250, optionally the accelerometer 252, the user interface 106, and the processor device 104. The processor device 104 includes the communication circuitry 256, the microprocessor 258, and the memory device 260. The memory device 260 has stored thereon the sensor array data 262, the PPG data 267 (in the embodiments of Fig. 15B), optionally the accelerometer data 264, optionally the microphone data 266, and the user report data 268. However, the memory 260 of the processor device 104 in the set 342 of data collection hardware optionally has stored thereon the first trained Al model 330 and optionally (e.g. in the embodiments of Fig. 15B) has stored thereon the treatment strategy routine 273. In such embodiments, the processor device 104 of the set 342 of data collection hardware may implement the data pre-processing routine 271 to extract feature values 272 and provide associated classification results 274.

Any or all of the data stored in the memory device 260 of the set 342 of data collection hardware may be communicated from the set 342 of data collection hardware to the modeling processor device 314. As above, the modeling processor device 314 may receive the data from the set 342 of data collection hardware via wired connection (e.g., Ethernet, serial connection, etc.) or wireless connection (e.g., mobile telephony, IEEE 802.11 protocol, etc.), directly (e.g., a connection with no intervening devices) or indirectly (e.g., a connection through one or more intermediary switches, access points, and/or the Internet), between the communication circuitry 256 of the processor device 104 and the communication circuitry 316 of the modeling processor device 314. Additionally, though not depicted in Figs. 15A and 15B, the data may be communicated from the set 342 of data collection hardware to the modeling processor device 314 via storage media, rather than by respective communication circuitry. The storage media may include any known storage memory type including, by way of example and not limitation, magnetic storage media, solid state storage media, secure digital (SD) memory cards, USB drives, and the like.

The received data may be stored in the memory 320 as a second AI training set 344 (depicted in Figs. 16C and 16D). The second Al training set 344 may include the sensor array data 262, the PPG data 267 (e.g., in the embodiments of Fig. 15B), user report data 268 and optional accelerometer data 264 and/or microphone data 266 received from the set 342 of data collection hardware. As depicted in Figs. 16C and 16D, the user report data 268 may include perceived events 350; characteristics or features of perceived events 352 such as severity and/or duration, perceived effects on memory, or other effects on the individual's wellbeing (such as their ability to hold a cup or operate a vehicle); other types of physiological symptoms (e.g., headaches, impaired or altered vision, involuntary movements, disorientation, falling to the ground, repeated jaw movements or lip smacking, etc.) 354; characteristics or features of other symptoms 356 (e.g., severity and/or duration); medication ingestion information 358 (e.g., medication types, dosages, and/or frequencies/timing); perceived medication side-effects 360; characteristics or features of medication side-effects 362 (e.g., severity and/or duration), and other user reported information 364 (e.g., food and/or drink ingested, activities performed (e.g., showering, exercising, working, brushing hair, etc.) tiredness, stress levels, etc.), as well as the timing of each. The adaptive learning component 324 may comprise instructions that are executable by the microprocessor 318 to implement a supervised or unsupervised machine learning program or algorithm, as described above, for iterating the first trained Al model 330, which may have a first error rate associated with its detection and/or classification results 336, to create a second trained AI model 346, which may have a second error rate, reduced from the first error rate, associated its detection and/or classification results 348. The data pre-processing routines 326, when executed by the microprocessor 318, may retrieve the data in the second AI training set 344, which may be raw recorded data, and may perform various pre-processing algorithms on the data in preparation for use of the data as training data by the adaptive learning component 324. The pre-processing routines 326 may include routines for removing noisy data, cleaning data, reducing or removing irrelevant and/or redundant data, normalization, transformation, and extraction of biomarkers and other features. The pre-processing routines 326 may also include routines for detection of muscle activity in the electrical activity data and particularly in the EEG data and/or the PPG data by analyzing the spectral content of the signal and/or routines for selection of the channel or channels of the electrical activity data that have the best (or at least better, relatively) signal to noise ratios. The output of the pre-processing routines 326 is a final training set stored in the memory 320 as a set 328 of feature values.

In embodiments in which the adaptive learning component 324 implements unsupervised learning algorithms, the adaptive learning component 324, executed by the microprocessor 318, finds its own structure in the unlabeled feature values 328 and, therefrom, generates a second trained Al model 346.

In embodiments in which the adaptive learning component 324 implements supervised learning algorithms, the memory 320 may also store one or more classification routines 332 that facilitate the labeling of the feature values (e.g., by an expert, such as a neurologist, reviewing the feature values 328 and/or the second Al training set 344) to create a set of key or label attributes 334. The adaptive learning component 324, executed by the microprocessor 318, may use both the feature values 328 and the key or label attributes 334 to discover rules, relationships, or other "models" that map the features to the labels by, for example, determining and/or assigning weights or other metrics. The adaptive learning component 324 may output an updated set of rules, relationships, or other models as a second trained Al model 346.

Regardless of the manner in which the adaptive learning component 324 iterates and/or updates the first trained Al model 330 to be the second trained Al model 346, the microprocessor 318 may use the second trained Al model 346 with the second Al training set 344 and/or the feature values 328 extracted therefrom, or on a portion of the second Al training set 344 and/or a portion of the feature values 328 extracted therefrom that were reserved for validating the second trained Al model 346, in order to provide classification results 348 for comparison and/or analysis by a trained professional in order to validate the output of the model. An error rate of the classification results 348 output by the second trained Al model 346 will be reduced relative to an error rate of the classification results 336 output by the first trained Al model 330. The second trained Al model 346 may be programmed into or communicated to the systems depicted, for example, in Figs 10A-13E, for use detecting and classifying events of interest among patients.

The static model 270, and the trained AI model 302, may each be programmed to facilitate a determination of whether an individual is experiencing epileptic or other types of events, by detecting within the received data (e.g., the sensor array data 262, the PPG data 267 (in embodiments implementing the PPG sensor 108), the user report data 268 and, optionally, the accelerometer data 264 and/or microphone data 266) events of interest, extracting from the received data relevant biomarkers for seizure activity (or sleep apnea activity or cochlear or vestibular disorders, if PPG data are available), and classifying or categorizing the relevant biomarkers as one of several different types of events.

In an embodiment, the models 270 and 302 may be programmed or trained to classify detected events of interest as one of the following types:
(1) a clinical manifestation of epilepsy, in which the respective patient exhibits outward signs of a seizure, and for which the detected events indicate a seizure;
(2) a sub-clinical manifestation of epilepsy, in which the respective patient exhibits no outward signs of a seizure, but for which the detected events would indicate a seizure;
(3) a non-clinical event, in which the respective patient exhibits no outward signs of a seizure, and for which the detected events include abnormal activity that is not suggestive of a seizure, but indicates abnormal activity relative to baseline sensor activity;
(4) a non-event, in which the respective patient either reports a seizure but that sensors do not suggest either a type 1, 2, or 3 event, or detected events closely resemble a seizure, but can be ruled out as noisy data or data artifacts; or
(5) a medication side-effect.

In embodiments, particularly those including the PPG sensor 108 and corresponding PPG data 267) the models 270 and 302 may be programmed or trained to classify detected events of interest as sleep apnea events, epilepsy events, cochlear events, vestibular events, etc., and may also classify an origin and/or type of the event, a severity of the event, a duration of the event, etc.

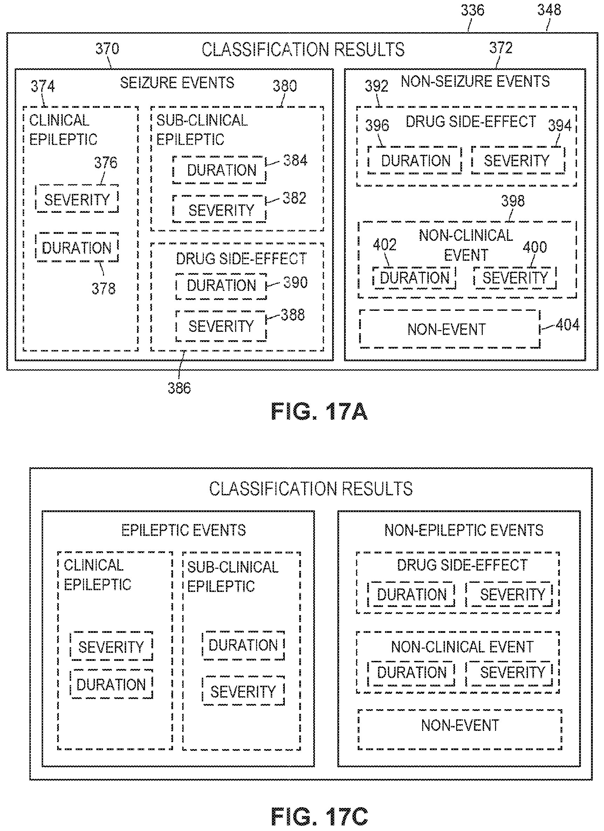

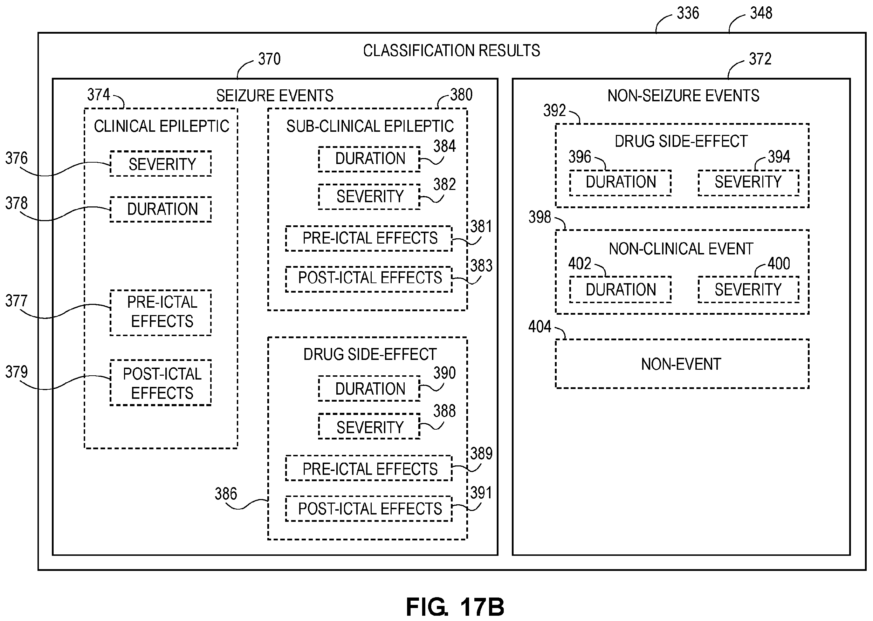

Figs. 17A and 17B depict the first set of classification results 336 and the second set of classification results 348, resulting respectively from the output of the first trained Al model 330 and the second trained Al model 346. (The classification results 274 output by the static model 270 may be similar.) The classification results 336 and 248 may each include a set of events 370 classified as seizure events and a set of events 372 classified as non-seizure events. In some embodiments, the detection and classification of events of interest may cease upon the classification of each detected event as a seizure event 270 or a non-seizure event 372. The detected events classified as seizure events 370 may include type 1 events (clinical manifestation of epilepsy) and type 2 events (sub-clinical manifestation of epilepsy). In embodiments, the seizure events 370 may also include certain type 5 events (medication side-effects), where the side-effect of the medication causes a seizure event. The detected events classified as non-seizure events 372 may include type 3 events (non-clinical) and type 4 events (non-events). In embodiments, the non-seizure events 372 may also include certain detected non-seizure events that are type 5 events (medication side-effects).

In embodiments, the detected events are further classified within each of the seizure events 370 and the non-seizure events 372. Specifically, the classification results may indicate the type of event and/or the severity of the event and/or the duration of the event. Figs. 17A and 17B illustrate that the seizure events 370 may further be categorized as having a first set of events 374 that are classified by the static model 270 or by the trained Al model 330 or 346 as type 1 events (clinical epileptic seizures), and may optionally include for each event a severity 376 and/or a duration 378. The seizure events 370 may also have a second set of events 380 that are classified by the static model 270 or by the trained Al model 330 or 346 as type 2 events (sub-clinical epileptic seizures), and may optionally include for each event a severity 382 and/or a duration 384. In some embodiments (e.g., some embodiments depicted in Fig. 17B), the seizure events 370 may optionally include for each event (whether one of the clinical epileptic events 374 or the sub-clinical epileptic events 380) one or more pre-ictal effects 377 and/or one or more post-ictal effects 379, which indicate, respectively, effects of the seizure events such as hypoxemia, changes in respiration or heart function, changes in mental status, and the like. The pre- and post-ictal effects may be determined from any one or more of the sensor array data 262, the PPG data 267, the microphone data 266 (if present), the accelerometer data 264 (if present), and the user reports 268. In embodiments, the seizure events 370 may also have a third set of events 386 that are classified by the static model 270 or by the trained Al model 330 or 346 as type 5 events (caused by medication side-effects), and may optionally include for each event a severity 388, a duration 390, pre-ictal effects 389, and/or post-ictal effects 391.

The non-seizure events 372 may similarly have a first set of events 392 that are classified by the static model 270 or by the trained Al model 330 or 346 as type 5 events (non-seizure events caused by medication side-effects), and may optionally include for each event a severity 394 and/or a duration 396. The non-seizure events 372 may also have a second set of events 398 that are classified by the static model 270 or by the trained Al model 330 or 346 as type 3 events (non-clinical), and may optionally include for each event a severity 400 and/or a duration 402. The non-seizure events 372 may also have a third set of events 404 that are classified by the static model 270 or by the trained Al model 330 or 346 as type 4 events (non-events).

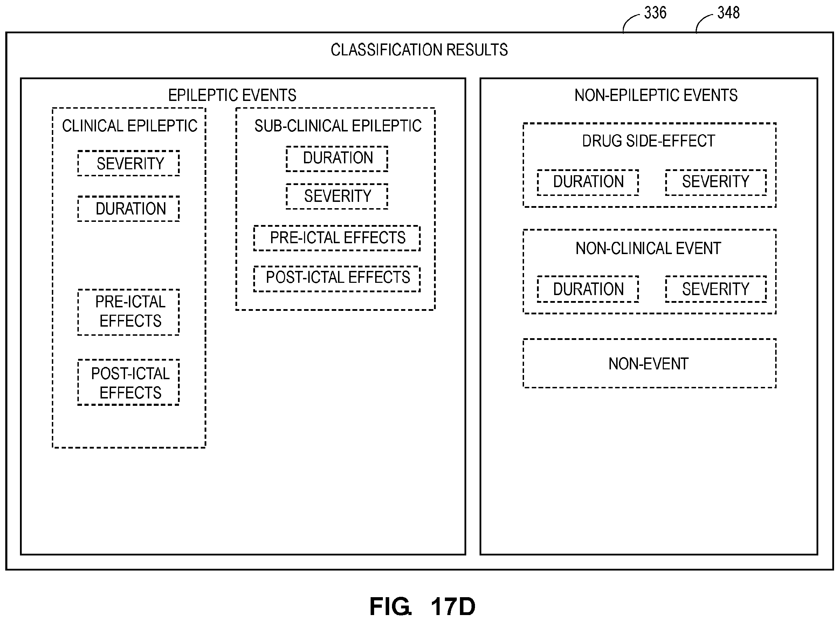

Figs. 17C and 17D depict alternate examples of the first set of classification results 336 and the second set of classification results 348, resulting respectively from the output of the first trained Al model 330 and the second trained Al model 346, in which events are categorized not as seizure and non-seizure events, but as epileptic and non-epileptic events. (The classification results 274 output by the static model 270 may be similar.) While the data may be largely identical, Figs. 17C and 17D make the point that drug side-effect events, while they may include seizures, are not epileptic events. That is, the data for each of the events may be the same in Figs. 17A-17D, but may be presented and/or stored differently depending on the embodiment. In essence, each of the detected events may be classified as one of types 1, 2, ,3, 4, or 5, in embodiments, and may further include severity and duration information. In embodiments, the classification results 336, 348 may include an indication of the feature values for each detected event that were heavily weighted in determining the classification type of the event.

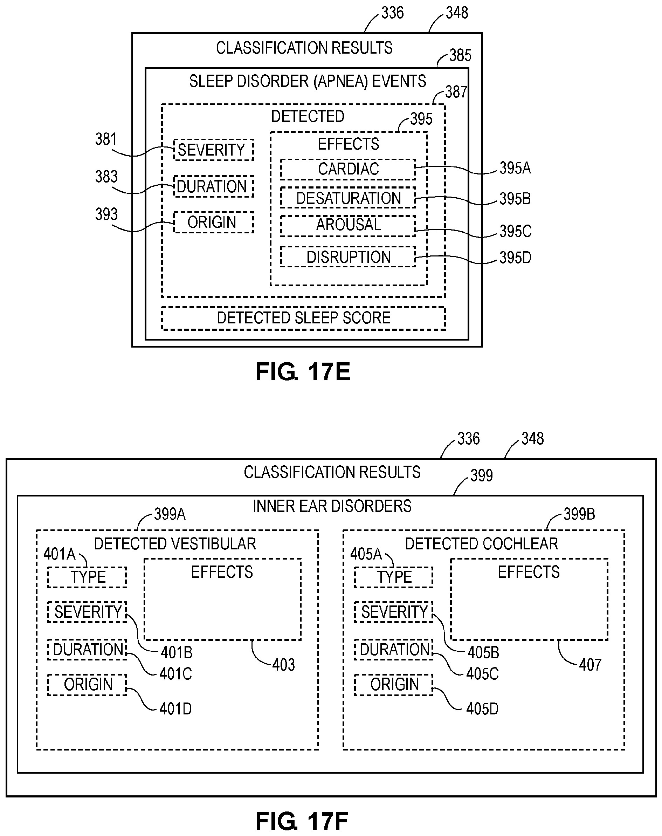

Fig. 17E depicts the first set of classification results 336 and the second set of classification results 348, resulting respectively from the output of the first trained Al model 330 and the second trained Al model 346, for embodiments in which the first and second trained Al models 330 and 346 are trained to perform evaluative functions related to sleep disorder events, such as apnea. (The classification results 274 output by the static model 270 may be similar.) The classification results 336 and 348 may each include a set 385 of data related to sleep disorder events. The set 385 of data may include data 387 related to detected sleep disorder events (e.g., apnea events). The classification results 336 and 348 may, in various embodiments, include any number of combination of the information depicted in Fig. 17E and, accordingly, all of the data are depicted in Fig. 17E as optional. However, it should be understood that certain data would be pre-requisite to other data. The data 387 related to detected sleep disorder events may include data for each detected event including severity 381 of the event, duration 383 of the event, and the origin 393 (e.g., obstructive apnea or central apnea) of the event. The data 387 for each detected event may also include data 395 on the effects of the event on patient well-being, including cardiac effects 395A (e.g., how severe, the duration, the recovery time), data 395B on desaturation experienced by the patient (e.g., how severe, the duration, the recovery time), data 395C on the arousal experienced by the patient (e.g., did the patient wake, how long was the patient awake, etc.), and data 395D related to the general disruption to the patient's normal well-being (e.g., how well the patient is able to function the following day). In embodiments, the data 385 may also include a detected sleep score 397 that takes into account all of the various factors described captured by the data 387.

Fig. 17F depicts the first set of classification results 336 and the second set of classification results 348, resulting respectively from the output of the first trained Al model 330 and the second trained Al model 346, when the first and second trained Al models 330 and 346 are trained to perform evaluative functions related to inner ear disorders, such as vestibular disorders and cochlear disorders. (The classification results 274 output by the static model 270 may be similar.) The classification results 336 and 348 may each include a set 399 of data related to inner ear disorder events. The set 399 of data may include data 399A related to detected vestibular disorder events (e.g., dizziness spells) and data 399B related to detected cochlear disorder events. The classification results 336 and 348 may, in various embodiments, include any number of combinations of the information depicted in Fig. 17F and, accordingly, all of the data are depicted in Fig. 17F as optional. However, it should be understood that certain data would be pre-requisite to other data - for example, if the data 399 do not include the data 399A related to vestibular events, then other data for vestibular events such as severity, duration, etc. would not be included either. The data 399A related to detected vestibular disorder events may include data for each event including a type 401A (e.g., dizziness, blurred vision, etc.) of the detected event, a severity 401B of the detected event, a duration 401C of the detected event, and an origin 401D (e.g., systemic infection, structural damage, neurological, etc.) of the detected event. The data 399A for each detected vestibular disorder event may also include data 403 on the effects of the detected event on patient well-being (e.g., how severe, the duration, the recovery time). Similarly, the data 399B related to detected cochlear disorder events may include data for each detected event including a type 405A (e.g., tinnitus, change in hearing threshold, etc.) of the detected event, a severity 405B of the detected event, a duration 405C of the detected event, and an origin 405D (e.g., systemic infection, structural damage, neurological, etc.) of the detected event. The data 399B for each detected cochlear disorder event may also include data 407 on the effects of the detected event on patient well-being (e.g., how severe, the duration, the recovery time).

Of course, in each of Figs. 17A-17F, the detected events may be associated with a time at which the detected event was detected to have occurred.

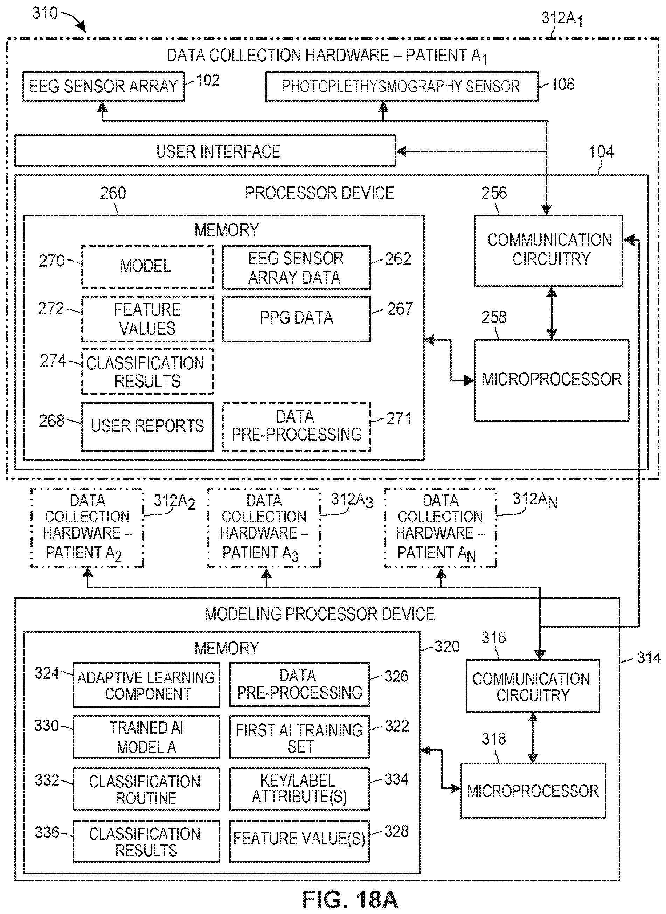

Figs. 18A-18G depict aspects of a set of embodiments related to Figs. 6C, 10C, and 13E. Fig. 18A is a block diagram of an example system 310 for use in creating a trained Al model (e.g., the trained Al model 302). The system 310 includes one or more sets 312A₁-312A_{N} of data collection hardware similar to the system 100 of Fig. 6C. That is, each set of data collection hardware 312A₁-312A_{N} includes a corresponding sensor array 102 (including electrode devices 110), one or more PPG sensors 108, and a user interface 106. Each of the sets 312A₁-312A_{N} of data collection hardware also includes a respective processor device 104, including communication circuitry 256, a microprocessor 258, and a memory 260. As in the systems 100, the memory 260 of each set 312A₁-312A_{N} of data collection hardware stores at least the sensor array data 262, the user reports 268, and the PPG data 267. Each of the sets 312A₁-312A_{N} of data collection hardware is associated with a corresponding patient A₁-A_{N} and, accordingly, each of the sets 312A₁-312A_{N} of data collection hardware collects data for a corresponding patient.

Unlike the systems 100 depicted in Fig. 6C, however, the sets 312A₁-312A_{N} of data collection hardware in the system 310 need not necessarily include the model 270 stored in the memory 260, and the memory 260 need not necessarily store feature values 272 or classification results 274. That is, the sets 312A₁-312A_{N} of data collection hardware in the system 310 need not necessarily be capable of performing the evaluative functions, but may, in embodiments, merely act as collectors of, and conduits for, information to be used as "training data" for to create the trained Al model 302.

The data collected by the sets 312A₁-312A_{N} of data collection hardware may be communicated to a modeling processor device 314. The modeling processor device 314 may be any computer workstation, laptop computer, mobile computing device, server, cloud computing environment, etc. that is configured to receive the data from the sets 312A₁-312A_{N} of data collection hardware and to use the data from the sets 312A₁-312A_{N} of data collection hardware to create the trained Al model 302. The modeling processor device 314 may receive the data from the sets 312A₁-312A_{N} of data collection hardware via wired connection (e.g., Ethernet, serial connection, etc.) or wireless connection (e.g., mobile telephony, IEEE 802.11 protocol, etc.), directly (e.g., a connection with no intervening devices) or indirectly (e.g., a connection through one or more intermediary switches, access points, and/or the Internet), between the communication circuitry 256 of the processor device 104 and communication circuitry 316 of the modeling processor device 314. Additionally, though not depicted in Fig. 18A, the data may be communicated from one or more of the sets 312A₁-312A_{N} of data collection hardware to the modeling processor device 314 via storage media, rather than by respective communication circuitry. The storage media may include any known storage memory type including, by way of example and not limitation, magnetic storage media, solid state storage media, secure digital (SD) memory cards, USB drives, and the like.

The modeling processor device 314 includes the communication circuitry 316, in embodiments in which it is necessary, a microprocessor 318, and a memory device 320. Though it should be understood, the microprocessor 318 may be one or more stand-alone microprocessors, one or more shared computing resources or processor arrays (e.g., a bank of processors in a cloud computing device), one or more multi-core processors, one or more DSPs, one or more FPGAs, etc. Similarly, the memory device 320 may be volatile or non-volatile memory, and may be memory dedicated solely to the modeling processor device 314 or shared among a variety of users, such as in a cloud computing environment.

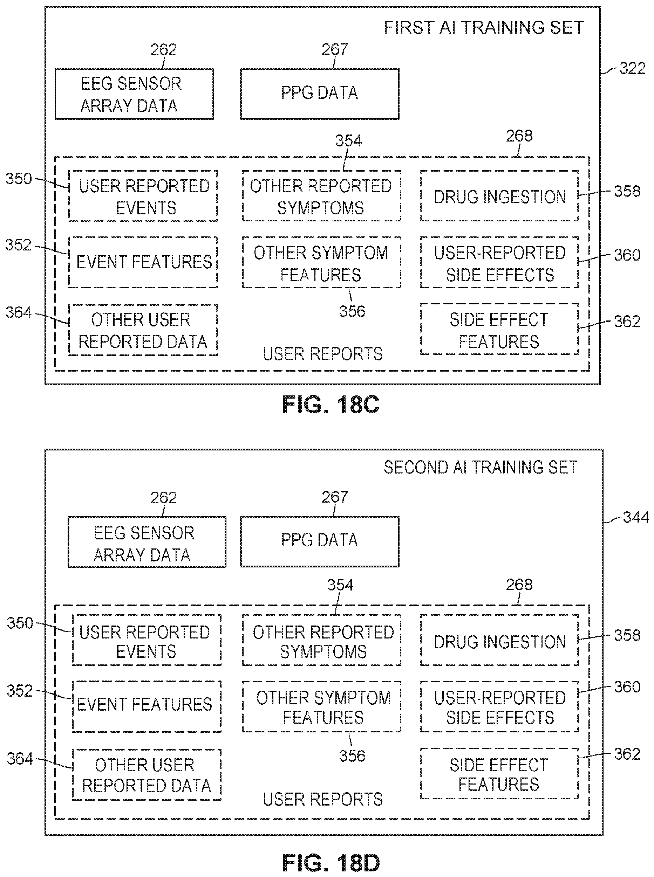

The memory 320 of the modeling processor device 314 may store as a first Al training set 322 (depicted in Fig. 18C) the sensor array data 262, user report data 268, and the PPG data 267 received from each of the sets 312A₁-312A_{N} of data collection hardware. As depicted in Fig. 18C, the user report data 268 may include perceived events (e.g., epileptic/seizure events; respiratory events such as apnea, tachnypnea, bradypnea; vestibular dysfunction events such as dizziness; cochlear dysfunction events such as hearing issues, etc.) 350; characteristics or features of perceived events 352 such as severity and/or duration, perceived effects on memory, or other effects on the individual's well-being (such as their ability to hold a cup or operate a vehicle, their ability to sleep, their ability to hear or balance, etc.); other types of physiological symptoms (e.g., headaches, impaired or altered vision, involuntary movements, disorientation, falling to the ground, repeated jaw movements or lip smacking, etc.) 354; characteristics or features of other symptoms 356 (e.g., severity and/or duration); medication ingestion information 358 (e.g., medication types, dosages, and/or frequencies/timing); perceived medication side-effects 360; characteristics or features of medication side effects 362 (e.g., severity and/or duration), and other user reported information 364 (e.g., food and/or drink ingested, activities performed (e.g., showering, exercising, working, brushing hair, etc.), tiredness, stress levels, etc.), as well as the timing of each. An adaptive learning component 324 may comprise instructions that are executable by the microprocessor 318 to implement a supervised or unsupervised machine learning program or algorithm, as described above. One or more data pre-processing routines 326, when executed by the microprocessor 318, may retrieve the data in the first Al training set 322, which may be raw recorded data, and may perform various pre-processing algorithms on the data in preparation for use of the data as training data by the adaptive learning component 324. The pre-processing routines 326 may include routines for removing noisy data, cleaning data, reducing or removing irrelevant and/or redundant data, normalization, transformation, and extraction of biomarkers and other features. The pre-processing routines 326 may also include routines for detection of muscle activity in the electrical activity data and particularly in the EEG data by analyzing the spectral content of the signal and/or routines for selection of the channel or channels of the electrical activity data that have the best (or at least better, relatively) signal to noise ratios. Still further, the pre-processing routines 326 may include routines for detecting biomarker signals from the raw data produced by the PPG sensor 108. The output of the pre-processing routines 326 is a final training set stored in the memory 320 as a set 328 of feature values.

In embodiments in which the adaptive learning component 324 implements unsupervised learning algorithms, the adaptive learning component 324, executed by the microprocessor 318, finds its own structure in the unlabeled feature values 328 and, therefrom, generates a first trained Al model 330.

In embodiments in which the adaptive learning component 324 implements supervised learning algorithms, the memory 320 may also store one or more classification routines 332 that facilitate the labeling of the feature values (e.g., by an expert, such as a neurologist, reviewing the feature values 328 and/or the first Al training set 322) to create a set of key or label attributes 334. The adaptive learning component 324, executed by the microprocessor 318, may use both the feature values 328 and the key or label attributes 334 to discover rules, relationships, or other "models" that map the features to the labels by, for example, determining and/or assigning weights or other metrics. The adaptive learning component 324 may output the set of rules, relationships, or other models as a first trained Al model 330.

Regardless of the manner in which the adaptive learning component 324 creates the first trained Al model 330, the microprocessor 318 may use the first trained Al model 330 with the first Al training set 322 and/or the feature values 328 extracted therefrom, or on a portion of the first Al training set 322 and/or a portion of the feature values 328 extracted therefrom that were reserved for validating the first trained Al model 330, in order to provide classification results 336 for comparison and/or analysis by a trained professional in order to validate the output of the model.

As should be apparent, the first Al training set 322 may include data from one or more of the sets 312A₁-312A_{N} of data collection hardware and, as a result, from one or more patients. Thus, the adaptive learning component 324 may use data from a single patient, from multiple patients, or from a multiplicity of patients when creating the first trained Al model 330. The population from which the patient or patients are selected may be tailored according to particular demographic (e.g., a particular type of epilepsy, a particular age group, etc.), in some instances, or may be non-selective. In embodiments, at least some of the patients associated with the sets 312A₁-312A_{N} of data collection hardware from which the first Al training set 322 is created may be patients without any symptoms of the condition(s) in question and, as such, may serve to provide additional control data to the first Al training set 322.

In embodiments, the first trained Al model 330 may be transmitted to (or otherwise received - e.g., via portable storage media) to another set of data collection hardware (e.g., the system 300 depicted in of Fig. 10C). The set of data collection hardware may implement the first trained Al model 330 to provide classification results 274 based on data that was not part of the first Al training set 322 collected by the sets 312A₁-312A_{N} of data collection hardware or, alternatively, may simply collect additional data for use by the modeling processor device 314 to iterate the first trained Al model 330.

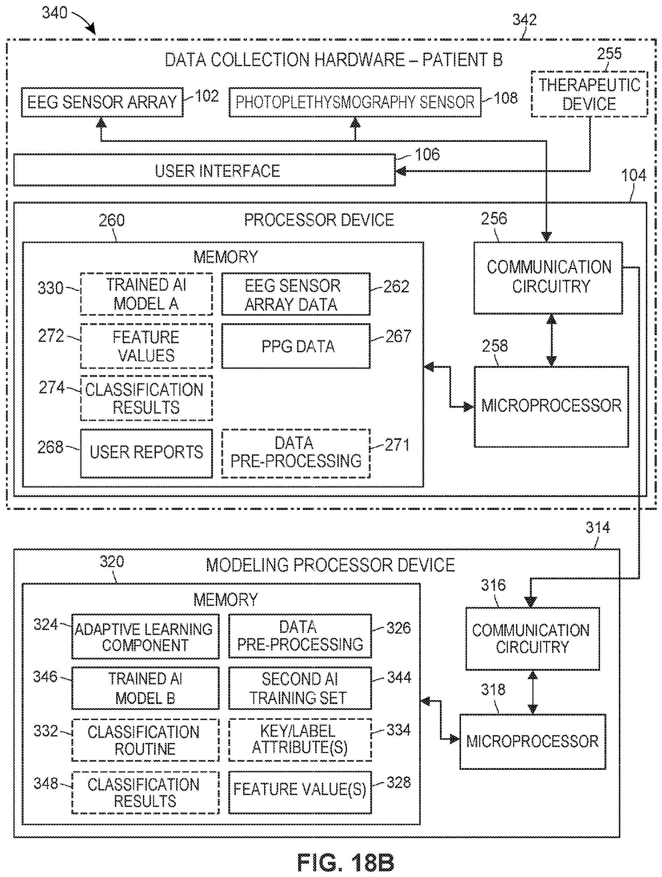

Fig. 18B depicts such an embodiment. In Fig. 18B, a system 340 includes a set 342 of data collection hardware for a patient. Like hardware previously described, the set of 342 of data collection hardware includes the sensor array 102, the PPG sensor 108, the user interface 106, the processor device 104 and, optionally, the therapeutic device 255. The processor device 104 includes the communication circuitry 256, the microprocessor 258, and the memory device 260. The memory device 260 has stored thereon the sensor array data 262, the PPG data 267, and the user report data 268. However, the memory 260 of the processor device 104 in the set 342 of data collection hardware optionally has stored thereon the first trained Al model 330. In such embodiments, the processor device 104 of the set 342 of data collection hardware may implement the data pre-processing routine 271 to extract feature values 272 and provide associated classification results 274.

Any or all of the data stored in the memory device 260 of the set 342 of data collection hardware may be communicated from the set 342 of data collection hardware to the modeling processor device 314. As above, the modeling processor device 314 may receive the data from the set 342 of data collection hardware via wired connection (e.g., Ethernet, serial connection, etc.) or wireless connection (e.g., mobile telephony, IEEE 802.11 protocol, etc.), directly (e.g., a connection with no intervening devices) or indirectly (e.g., a connection through one or more intermediary switches, access points, and/or the Internet), between the communication circuitry 256 of the processor device 104 and the communication circuitry 316 of the modeling processor device 314. Additionally, though not depicted in Fig. 18B, the data may be communicated from the set 342 of data collection hardware to the modeling processor device 314 via storage media, rather than by respective communication circuitry. The storage media may include any known storage memory type including, by way of example and not limitation, magnetic storage media, solid state storage media, secure digital (SD) memory cards, USB drives, and the like.

The received data may be stored in the memory 320 as a second Al training set 344 (depicted in Fig. 18D). The second Al training set 344 may include the sensor array data 262, user report data 268, and the PPG data 267 received from the set 342 of data collection hardware. As depicted in Fig. 18C, the user report data 268 may include perceived events (e.g., epileptic/seizure events; respiratory events such as apnea, tachnypnea, bradypnea; vestibular dysfunction events such as dizziness; cochlear dysfunction events such as hearing issues, etc.) 350; characteristics or features of perceived events 352 such as severity and/or duration, perceived effects on memory, or other effects on the individual's well-being (such as their ability to hold a cup or operate a vehicle, their ability to sleep, their ability to hear or balance, etc.); other types of physiological symptoms (e.g., headaches, impaired or altered vision, involuntary movements, disorientation, falling to the ground, repeated jaw movements or lip smacking, etc.) 354; characteristics or features of other symptoms 356 (e.g., severity and/or duration); medication ingestion information 358 (e.g., medication types, dosages, and/or frequencies/timing); perceived medication side-effects 360; characteristics or features of medication side effects 362 (e.g., severity and/or duration), and other user reported information 364 (e.g., food and/or drink ingested, activities performed (e.g., showering, exercising, working, brushing hair, etc.), tiredness, stress levels, etc.), as well as the timing of each. The adaptive learning component 324 may comprise instructions that are executable by the microprocessor 318 to implement a supervised or unsupervised machine learning program or algorithm, as described above, for iterating the first trained Al model 330, which may have a first error rate associated with its classification results 336 (e.g., the results of the evaluative functions), to create a second trained Al model 346, which may have a second error rate, reduced from the first error rate, associated its classification results 348 (e.g., the results of the evaluative functions). The data pre-processing routines 326, when executed by the microprocessor 318, may retrieve the data in the second Al training set 344, which may be raw recorded data, and may perform various pre-processing algorithms on the data in preparation for use of the data as training data by the adaptive learning component 324. The pre-processing routines 326 may include routines for removing noisy data, cleaning data, reducing or removing irrelevant and/or redundant data, normalization, transformation, and extraction of biomarkers and other features. The pre-processing routines 326 may also include routines for detection of muscle activity in the electrical activity data and particularly in the EEG data by analyzing the spectral content of the signal and/or routines for selection of the channel or channels of the electrical activity data that have the best (or at least better, relatively) signal to noise ratios. Still further, the pre-processing routines 326 may include routines for detecting biomarker signals from the raw data produced by the PPG sensor 108. The output of the pre-processing routines 326 is a final training set stored in the memory 320 as a set 328 of feature values.

In embodiments in which the adaptive learning component 324 implements unsupervised learning algorithms, the adaptive learning component 324, executed by the microprocessor 318, finds its own structure in the unlabeled feature values 328 and, therefrom, generates a second trained Al model 346.

In embodiments in which the adaptive learning component 324 implements supervised learning algorithms, the memory 320 may also store one or more classification routines 332 that facilitate the labeling of the feature values (e.g., by an expert, such as a neurologist, reviewing the feature values 328 and/or the second Al training set 344) to create a set of key or label attributes 334. The adaptive learning component 324, executed by the microprocessor 318, may use both the feature values 328 and the key or label attributes 334 to discover rules, relationships, or other "models" that map the features to the labels by, for example, determining and/or assigning weights or other metrics. The adaptive learning component 324 may output an updated set of rules, relationships, or other models as a second trained Al model 346.

Regardless of the manner in which the adaptive learning component 324 iterates and/or updates the first trained Al model 330 to be the second trained Al model 346, the microprocessor 318 may use the second trained Al model 346 with the second Al training set 344 and/or the feature values 328 extracted therefrom, or on a portion of the second Al training set 344 and/or a portion of the feature values 328 extracted therefrom that were reserved for validating the second trained Al model 346, in order to provide classification results 348 for comparison and/or analysis by a trained professional in order to validate the output of the model. An error rate of the classification results 348 output by the second trained Al model 346 will be reduced relative to an error rate of the classification results 336 output by the first trained Al model 330. The second trained Al model 346 may be programmed into or communicated to the system depicted, for example, in Fig. 10C, for use performing evaluative functions for patients.

The static model 270, and the trained Al model 302, may each be programmed to perform the evaluative functions by detecting within the received data (e.g., the sensor array data 262, the user report data 268, and the PPG data 267) relevant biomarkers for the condition(s) of interest (e.g., epilepsy/seizure activity, signs of vestibular or cochlear dysfunction, sleep disorder/apnea activity) and performing the evaluative functions based on the presence, absence, and/or temporal relationships between the relevant biomarkers. In various embodiments, the models 270 and 302 may be programmed or trained to perform one or more of the following evaluative functions:
(1) detecting a seizure;
(2) classifying a seizure as epileptic or cardiac in origin;
(3) classifying a seizure as ictal hypoxemic or not;
(4) predicting a seizure event;
(5) classifying a severity of a seizure event;
(6) determining a pre- or post-ictal impact of a seizure event on patient well-being;
(7) predicting a pre- or post-ictal impact of a seizure event on patient well-being (severity of the event, ictal cardiac changes; types of ictal respiratory changes);
(8) predicting a recovery time from post-ictal impacts of a seizure event on patient well-being;
(9) detecting an apnea event;
(10) classifying an apnea event as central or obstructive;
(11) detecting a tachypnea or a bradypnea event;
(12) predicting an apnea, tachypnea, or bradypnea event;
(13) determining an impact of an apnea, tachypnea, or bradypnea event on patient well-being;
(14) predicting a pre- or post-ictal impact of an apnea, tachypnea, or bradypnea event event on patient well-being;
(15) predicting a recovery time from post-ictal impacts of an apnea, tachypnea, or bradypnea event event on patient well-being;
(16) detecting a SUDEP event;
(17) predicting a SUDEP event;
(18) detecting a vestibular dysfunction;
(19) detecting a cochlear dysfunction;
(20) detecting inflammatory markers to predict systemic infection.

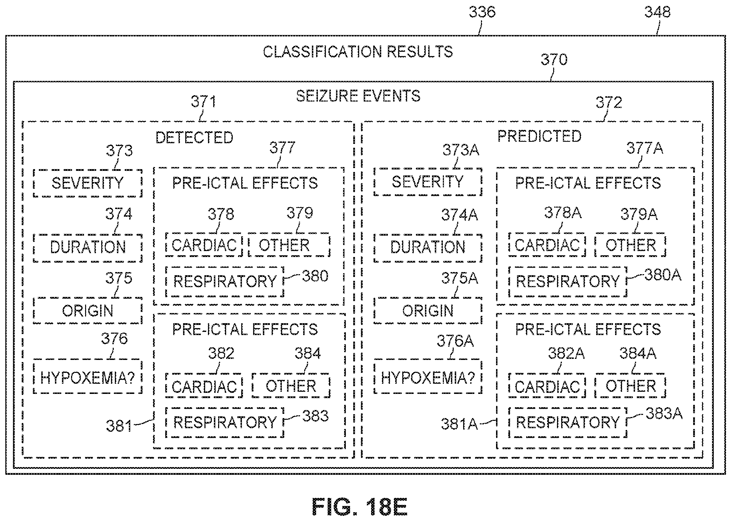

Fig. 18E depicts the first set of classification results 336 and the second set of classification results 348, resulting respectively from the output of the first trained Al model 330 and the second trained Al model 346, when the first and second trained Al models 330 and 346 are trained to perform evaluative functions related to epilepsy events. (The classification results 274 output by the static model 270 may be similar.) The classification results 336 and 348 may each include a set 370 of data related to seizure events. The set 370 of data may include data 371 related to detected seizure events and data 372 related to predicted seizure events. The classification results 336 and 348 may, in various embodiments, include any number of combinations of the information depicted in Fig. 18E and, accordingly, all of the data are depicted in Fig. 18E as optional. However, it should be understood that certain data would be pre-requisite to other data - for example, if the data 370 do not include the data 371 of detected events, then other data for detected events such as severity, duration, etc. would not be included either. In any case, the data 371 related to detected seizure events may include data for each detected event including severity 373 of the event, duration 374 of the event, origin 375 (e.g., epileptic or cardiac) of the event, and whether the event induced hypoxemia 376. The data 371 related to detected events may also include, in embodiments, pre-ictal effects 377 and/or post-ictal effects 381. In embodiments that include, for one or more events, pre-ictal effects 377, the pre-ictal effects may be further categorized as including cardiac effects 378 (e.g., tachycardia, bradycardia, etc.), respiratory effects 380 (e.g., apnea, tachypnea, bradypnea, etc.), and other effects 379 (e.g., effects on memory, balance, or other abilities). Similarly, in embodiments that include, for one or more events, post-ictal effects 381, the post-ictal effects may be further categorized as including cardiac effects 382 (e.g., tachycardia, bradycardia, etc.), respiratory effects 383 (e.g., apnea, tachypnea, bradypnea, etc.), and other effects 384 (e.g., effects on memory, balance, or other abilities). Likewise, the data 372 related to predicted seizure events may include data for each predicted event including predicted severity 373A of the event, predicted duration 374A of the event, predicted origin 375A (e.g., epileptic or cardiac) of the event, and whether the predicted event will induce hypoxemia 376A. The data 372 related to predicted events may also include, in embodiments, predicted pre-ictal effects 377A and/or predicted post-ictal effects 381A. In embodiments that include, for one or more events, predicted pre-ictal effects 377A, the predicted pre-ictal effects may be further categorized as including predicted cardiac effects 378A (e.g., tachycardia, bradycardia, etc.), predicted respiratory effects 380A (e.g., apnea, tachypnea, bradypnea, etc.), and other predicted effects 379A (e.g., effects on memory, balance, or other abilities). Similarly, in embodiments that include, for one or more events, predicted post-ictal effects 381A, the predicted post-ictal effects may be further categorized as including predicted cardiac effects 382A (e.g., tachycardia, bradycardia, etc.), predicted respiratory effects 383A (e.g., apnea, tachypnea, bradypnea, etc.), and other predicted effects 384A (e.g., effects on memory, balance, or other abilities).

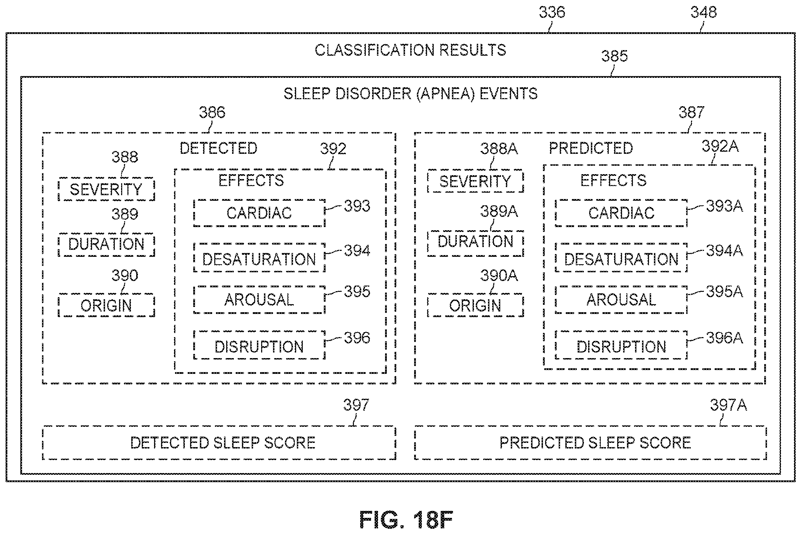

Fig. 18F depicts the first set of classification results 336 and the second set of classification results 348, resulting respectively from the output of the first trained Al model 330 and the second trained Al model 346, when the first and second trained Al models 330 and 346 are trained to perform evaluative functions related to sleep disorder events, such as apnea. (The classification results 274 output by the static model 270 may be similar.) The classification results 336 and 348 may each include a set 385 of data related to sleep disorder events. The set 385 of data may include data 386 related to detected sleep disorder events (e.g., apnea events) and data 387 related to predicted sleep disorder events. The classification results 336 and 348 may, in various embodiments, include any number of combinations of the information depicted in Fig. 18F and, accordingly, all of the data are depicted in Fig. 18F as optional. However, it should be understood that certain data would be pre-requisite to other data - for example, if the data 385 do not include the data 386 of detected events, then other data for detected events such as severity, duration, etc. would not be included either. The data 386 related to detected sleep disorder events may include data for each detected event including severity 388 of the event, duration 389 of the event, and the origin 390 (e.g., obstructive apnea or central apnea) of the event. The data 386 for each detected event may also include data 392 on the effects of the event on patient well-being, including cardiac effects 393 (e.g., how severe, the duration, the recovery time), data 394 on desaturation experienced by the patient (e.g., how severe, the duration, the recovery time), data 395 on the arousal experienced by the patient (e.g., did the patient wake, how long was the patient awake, etc.), and data 396 related to the general disruption to the patient's normal well-being (e.g., how well the patient is able to function the following day). In embodiments, the data 385 may also include a detected sleep score 397 that takes into account all of the various factors described captured by the data 386. Likewise, the data 387 related to predicted sleep disorder events may include data for each predicted event including predicted severity 388A of the event, predicted duration 389A of the event, and the predicted origin 390A (e.g., obstructive apnea or central apnea) of the event. The data 386 for each predicted event may also include data 392A on the predicted effects of the predicted event on patient well-being, including predicted cardiac effects 393A (e.g., predicted severity, predicted duration, predicted recovery time), data 394A on predicted desaturation experienced by the patient (e.g., predicted severity, predicted duration, predicted recovery time), data 395A on the arousal by the patient is predicted to experience (e.g., will the patient wake, how long will the patient remain awake, etc.), and data 396A related to the predicted general disruption to the patient's normal well-being (e.g., how well will the patient be able to function the following day). In embodiments, the data 385 may also include a predicted sleep score 397A that takes into account all of the various factors described captured by the data 387.

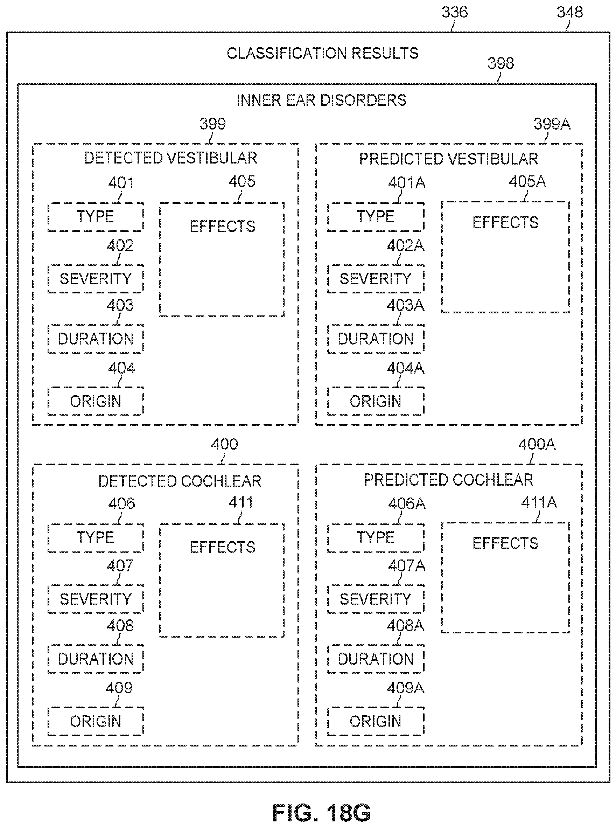

Fig. 18G depicts the first set of classification results 336 and the second set of classification results 348, resulting respectively from the output of the first trained Al model 330 and the second trained Al model 346, when the first and second trained Al models 330 and 346 are trained to perform evaluative functions related to inner ear disorders, such as vestibular disorders and cochlear disorders. (The classification results 274 output by the static model 270 may be similar.) The classification results 336 and 348 may each include a set 398 of data related to inner ear disorder events. The set 398 of data may include data 399 and/or 399A related, respectively, to detected and predicted vestibular disorder events (e.g., dizziness spells) and data 400 and/or 400A related, respectively, to detected and predicted cochlear disorder events. The classification results 336 and 348 may, in various embodiments, include any number of combinations of the information depicted in Fig. 18G and, accordingly, all of the data are depicted in Fig. 18G as optional. However, it should be understood that certain data would be pre-requisite to other data - for example, if the data 398 do not include the data 399 related to vestibular events, then other data for vestibular events such as severity, duration, etc. would not be included either. The data 399 related to detected vestibular disorder events may include data for each event including a type 401 (e.g., dizziness, blurred vision, etc.) of the detected event, a severity 402 of the detected event, a duration 403 of the detected event, and an origin 404 (e.g., systemic infection, structural damage, neurological, etc.) of the detected event. The data 399 for each detected vestibular disorder event may also include data 405 on the effects of the detected event on patient well-being (e.g., how severe, the duration, the recovery time). The data 399A related to predicted vestibular disorder events may include data for each event including a predicted type 401A (e.g., dizziness, blurred vision, etc.) of the predicted event, a predicted severity 402A of the predicted event, a predicted duration 403A of the predicted event, and a predicted origin 404A (e.g., systemic infection, structural damage, neurological, etc.) of the predicted event. The data 399A for each predicted vestibular disorder event may also include data 405A on the predicted effects of the predicted event on patient well-being (e.g., how severe, the duration, the recovery time). Similarly, the data 400 related to detected cochlear disorder events may include data for each detected event including a type 406 (e.g., tinnitus, change in hearing threshold, etc.) of the detected event, a severity 407 of the detected event, a duration 408 of the detected event, and an origin 409 (e.g., systemic infection, structural damage, neurological, etc.) of the detected event. The data 400 for each detected cochlear disorder event may also include data 411 on the effects of the detected event on patient well-being (e.g., how severe, the duration, the recovery time). The data 400A related to predicted cochlear disorder events may include data for each predicted event including a predicted type 406A (e.g., tinnitus, change in hearing threshold, etc.) of the predicted event, a predicted severity 407A of the predicted event, a predicted duration 408A of the predicted event, and a predicted origin 409A (e.g., systemic infection, structural damage, neurological, etc.) of the predicted event. The data 400A for each predicted cochlear disorder event may also include data 411A on the predicted effects of the predicted event on patient well-being (e.g., how severe, the duration, the recovery time).

Of course, in each of Figs. 18E-18G, the detected and/or predicted events may be associated with a time at which the detected event was detected to have occurred or a time at which the predicted event is predicted to occur.

It should be understood that the system and, in particular, the adaptive learning component 324 (whether implemented in the separate modeling processor device 314), the data collection hardware 342, or even in the processor device 104 alongside the trained Al model 302, may be programmed to analyze the predicted event data (e.g., predicted seizure event data 372, predicted sleep disorder event data 387, predicted vestibular disorder event data 399A, predicted cochlear disorder event data 400A) relative to detected event data (e.g., detected seizure event data 371, detected sleep disorder event data 386, detected vestibular disorder event data 399, detected cochlear disorder event data 400) to determine the accuracy of the predictions made by the trained Al model 302. The results of the analysis may be used by the adaptive learning component 324 to further refine the trained Al model 302.

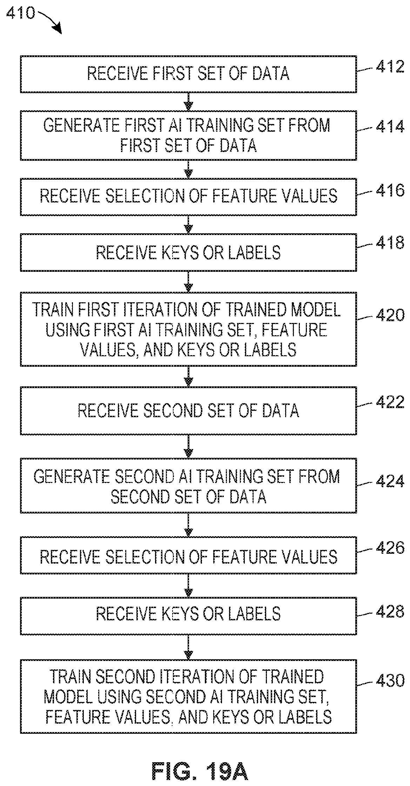

Fig. 19A is a flow chart depicting a method 410 for training an Al model (e.g., the trained Al model 302) to detect, predict, and/or classify events, in various embodiments. The method 410 may include receiving, at a modeling processor device 314, from a first processor device 104 a first set of data (block 412). The first set of data may include sensor array data 262 from one or more first sensor arrays 102 disposed on respective first patients, PPG data 267, in embodiments implementing the PPG sensor 108, and may further include one or both of first microphone data 266 from respective first microphones 250 disposed on the one or more first patients and first accelerometer data 264 received from respective first accelerometers 252 disposed on the one or more first patients. The method 410 may also include generating a first Al training set 322 based on the first set of data and on corresponding user reported data (block 414), including the user reports 268 also received from the first processor device 104. The method may also include receiving a selection of one or more attributes of the first Al training set 322 as feature values 328 (block 416) and receiving one or more keys or labels 334 for the first Al training set 322 (block 418). The feature values 328 and the keys or labels 334 may be received via the classification routine 332. The modeling processor device 314 then trains a first iteration of a trained model 330, using the feature values and the one or more keys or labels for the first Al training set 322 (block 420).

The method 410 may also include receiving, at the modeling processor device 314, from a second processor device 104 a second set of data (block 422). The second set of data may include sensor array data 262 from one or more first sensor arrays 102 disposed on a second patient, PPG data 267, in embodiments implementing the PPG sensor 108, and may further include one or both of second microphone data 266 from a first microphones 250 disposed on the second patient and second accelerometer data 264 received from a second accelerometer 252 disposed on the second patient. The method 410 may also include generating a second AI training set 344 based on the second set of data and on corresponding user reported data (block 424), including the user reports 268 also received from the second processor device 104. The method also include receiving a selection of one or more attributes of the second Al training set 344 as feature values 328 (block 426) and receiving one or more keys or labels 334 for the second Al training set 344 (block 428). The feature values 328 and the keys or labels 334 may be received via the classification routine 332. The modeling processor device 314 then trains a second iteration of a trained model 346, using the feature values and the one or more keys or labels for the second Al training set 344 (block 430).

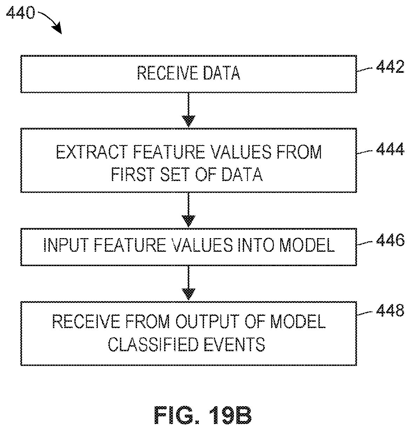

Fig. 19B is a flow chart depicting a method 440 for detecting and classifying events, in embodiments such as those of Figs. 6A, 6B, 7A-10B, and 11A-13D. The method 440 includes receiving, at a processor device 104, a set data (block 442). The set of data may include sub-scalp electrical signal data 262 from a sensor array 102 disposed beneath the scalp of a patient and communicatively coupled, directly or indirectly, to the processor device 104. The set of data may also include one or both of microphone data 266 from a microphone 250 disposed on the patient, and accelerometer data 264 from an accelerometer 252 disposed on the patient. The method 440 also includes extracting from the set of data a plurality of feature values 272 (block 444), the plurality of feature values 272 including each of one or more feature values of the sub-scalp electrical signal data 262 and one or both of one or more feature values of the microphone data 266 and one or more feature values of the accelerometer data 264. The method 440 then includes inputting into a trained model 302 executing on the processor device 104, the plurality of feature values 272 (block 446). The trained model 302 is configured according to an Al algorithm based on a previous plurality of feature values, and the trained model 302 is configured to provide one or more classification results 274 (block 448) based on the plurality of feature values 272, the one or more classification results 274 corresponding to one or more events captured in the biomarker data.

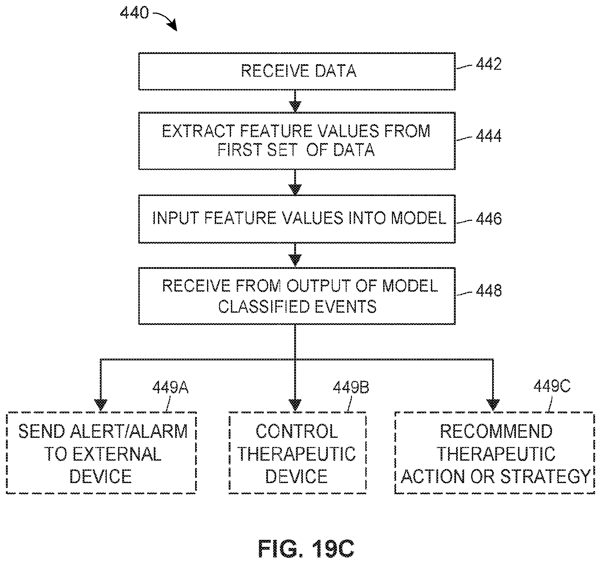

Fig. 19C is a flow chart depicting a method 440 for detecting and classifying events, in embodiments such as those of Figs. 6C, 10C, and 13E. The method 440 includes receiving, at a processor device 104, a set data (block 442). The set of data may include sensor array data 262 from a sensor array 102 disposed on a patient and communicatively coupled, directly or indirectly, to the processor device 104. The set of data may also include PPG data 267 from a PPG sensor 108 disposed on the patient. The method 440 also includes extracting from the set of data a plurality of feature values 272 (block 444), the plurality of feature values 272 including each of one or more feature values of the sensor array data 262 and one or more feature values of the PPG data 267. The method 440 then includes inputting into a trained model 302 executing on the processor device 104, the plurality of feature values 272 (block 446). The trained model 302 is configured according to an Al algorithm based on a previous plurality of feature values, and the trained model 302 is configured to provide one or more classification results 274 (block 448) based on the plurality of feature values 272, the one or more classification results 274 corresponding to one or more events captured in the biomarker data.

The classification results 274 may then optionally be used to perform one or more actions (blocks 449A-C). For example, the classification results 274 may trigger the sending of an alert or alarm to a caregiver, to a physician, and/or to the patient (block 449A). In one specific, non-limiting example, the classification results 274 may indicate that the patient has a blood oxygen saturation level below a threshold - perhaps as the result of a seizure or a sleep apnea event - and may cause the processor device 104 to send an alert to the patient to administer supplemental oxygen. The alert may be delivered via the processor device 104, or via an external device 105. The processor device 104 may also alert a caregiver and/or physician by communicating with one or more external devices 105. In another specific, non-limiting example, the classification results 274 may indicate that the patient may be about to experience a seizure and may cause the processor device 104 to send an alert to the patient so that the patient can prepare (e.g., stop dangerous activities, alert bystanders, get to a safe position, etc.). The alert may be delivered via the processor device 104, or via an external device 105. The processor device 104 may also alert a caregiver and/or physician by communicating with one or more external devices 105.

The classification results 274 may also (or alternatively) trigger the control of the therapeutic device 255, in embodiments (block 449B). In one specific, non-limiting example, the classification results 274 may indicate that the patient is experiencing an obstructive sleep apnea episode and may cause the processor device 104 (e.g., using the treatment strategy routine 273) to communicate with a CPAP machine (e.g., the therapeutic device 255) to increase the airway pressure to relieve the obstruction causing the apnea episode. In another specific, non-limiting example, the classification results 274 may indicate that the patient may be about to experience a seizure and may cause the processor device 104 to communicate with a neurostimulator device (e.g., the therapeutic device 255) to cause the neurostimulator to apply (or adjust the application) of neurostimulation to prevent, or mitigate the effects of, the predicted impending seizure. In still another specific, non-limiting example, the classification results 274 may indicate that the patient may experience a seizure in the coming hours and may cause the processor device 104 to communicate with a drug pump device (e.g., the therapeutic device 255) cause the drug pump device to administer (or change the dose of) a drug to prevent, or mitigate the effects of, the predicted seizure.

Additionally or alternatively, the classification results 274 may trigger the processor device 104 to determine a recommended therapy (e.g., using the treatment strategy routine 273) and to transmit that strategy to the patient (e.g., via the processor device 104 or an external device 105), to a caregiver (e.g., via the external device 105), and/or to a physician (e.g., via the external device 105) (block 449C). In embodiments, the recommended therapy may be transmitted for the purpose of verifying (e.g., by the physician) a treatment prior to causing the processor device 104 to engage or adjust the therapeutic device 255 (e.g., prior to block 449B). In one specific, non-limiting example, the classification results 274 may indicate that the patient is in the early stages of a systemic infection that may jeopardize or have other negative effects on the patient's cochlear well-being. This may cause the processor device 104 to recommend evaluation by the physician, or to recommend a pharmacological intervention (e.g., an antibiotic), and to send the recommendation to the physician or caregiver (or even to the patient) via the external device 105 (or to the patient via the processor device 104 or the external device 105). In another specific, non-limiting example, the classification results 274 may indicate that the patient is likely to experience low blood oxygen saturation levels following a predicted seizure, and may therefore cause the processor 104 to send a recommendation to administer supplemental oxygen before and/or after the seizure event.

The non-limiting examples above should be understood as exemplary only. A person of skill in the art will readily appreciate, in view of the disclosures throughout this specification, that a variety of treatment strategies, alarms, alerts, etc. may be implemented in various situations according to the type of classification results that the system 100 is programmed to generate, and according to the specific therapeutic device(s) 255 that may be coupled thereto.

As may by now be understood, the presently disclosed method and system are amenable to a variety of embodiments, many of which have already been explicitly described, though additional embodiments will now be described with with reference to Figs. 20A-25D.

Figs. 20A-20E depict block diagrams of various example embodiments 450A-E, respectively, of the sensor array 102. In each, electrical activity sensors 452 include either or both of the electrode devices 110 and the biochemical sensors 282. That is, as should by now be understood, the sensor array 102 in any embodiment, includes the one or more electrical activity sensors 452, which may be electrode deices 110, which electrical signals in the brain or elsewhere in the body, depending on placement), biochemical sensors 282, which detect biochemical markers in the patient's body and convert those signals to measurable electrical outputs, or both electrode devices 110 and biochemical sensors 282. In each of the embodiments 450A-E, the sensor array 102 includes the local processing device 144, which includes the amplifier 146, the battery 148, the transceiver or communications circuitry 150, the analog-to-digital converter 152, and the processor 154. Each of the embodiments 450A-E may also optionally include a battery charging circuit 454, for facilitating charging of the battery 148. The battery charging circuit 454 may be any known battery charging technology compatible with the arrangement of the sensor array 102 on the patient. In particular, in embodiments the battery charging circuit 454 may be an inductive charging circuit that facilitates charging through the patient's skin when the sensor array 102 is disposed beneath the scalp of the patient. In other embodiments, the battery charging circuit 454 may draw energy from the movements of the patient throughout the day by, for example, harnessing the movements of the patient to turn a small generator. In still other embodiments, the battery charging circuit 454 may draw power from the environment in the form of RF signals. In further examples still, the battery charging circuit 454 may draw power from chemical reactions taking place in the environment of the sensor array 102. Of course, more traditional charging methods (e.g., using a wired connection to provide power to the battery charging circuit 454) may also be employed.

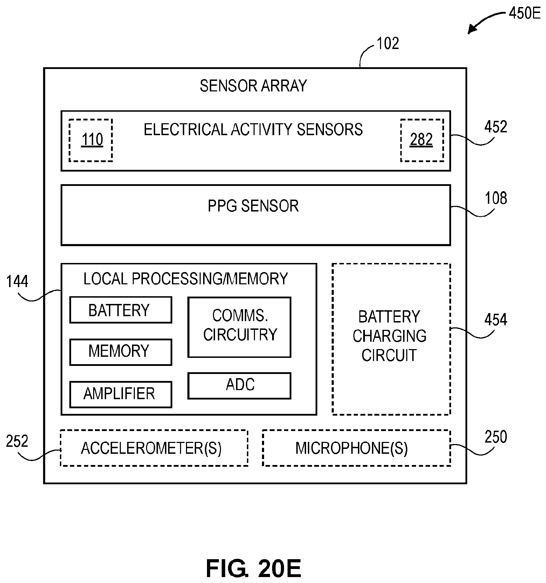

As can be seen in Figs. 20A-20E, the embodiment 450A does not include in the sensor array 102 the microphone 250 or the accelerometer 252. In embodiment 450B, the sensor array 102 includes one or more accelerometers 252. In the embodiment 450C, the sensor array 102 includes one or more microphones 250. In the embodiment 450D, the sensor array 102 includes one or more microphones 250 and one or more accelerometers 252. In the embodiment 450E in Fig. 20E, the sensor array 102 includes the PPG sensor 108, and may optionally include the accelerometers 252 and/or the microphones 250. Of course, each of the embodiments 450A-E of the sensor array 102, may be used with or without additional microphones 250 (i.e., microphones 250 that are not part of the sensor array 102), with or without additional accelerometers 252 (i.e., accelerometers 252 that are not part of the sensor array 102), in various embodiments, and with or without additional PPG sensors 108 (i.e., PPG sensors 108 that are not part of the sensor array 102).

Like the sensor array 102, the processor device 104 is similarly amenable to a variety of embodiments. Figs. 21A-21E are block diagrams of various example embodiments 460A-E, respectively, of the processor device 104. In each of the processor devices 104 depicted in embodiments 460A-E, the processor device 104 includes the communication circuitry 256, the microprocessor 258, and the memory 260. Each of the processor devices 104 in the embodiments 460A-E also includes a battery (or other power source) 462 and battery charging technology 464 (which, obviously, would be omitted in the event that the power source were other than the battery 462). The user interface 106 may also optionally be disposed in the processor device 104.

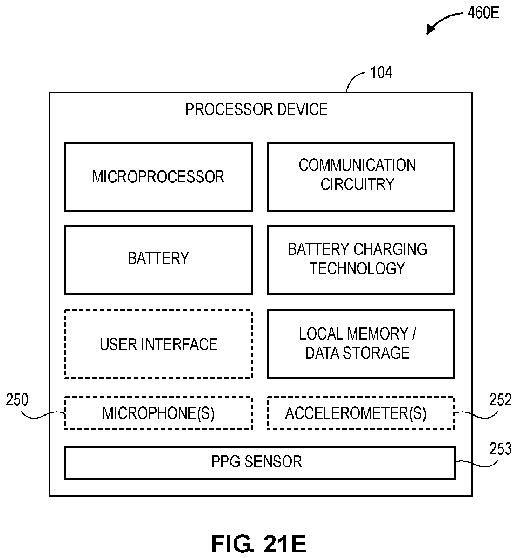

As described throughout the specification, various embodiments of the system 100 may include one or both of microphones 250 and accelerometers 252. In various embodiments, the microphones 250 and/or accelerometers 252 may be separate from, but communicatively coupled to, the processor device 104. In embodiments, such as those described above with respect to Figs. 20B-20E, one or more microphones 250 and/or accelerometers 252 may be disposed in the sensor array 102. Similarly, one or more microphones 250 and/or accelerometers 252 may be disposed in the processor device 104 in various embodiments. Fig. 21A depicts in embodiment 460A a processor device 104 that does not include any microphones 250 or accelerometers 252. Figs. 21B-21D depict, respectively, in embodiments 460B-D, a processor device 104 that includes one or more accelerometers 252, one or more microphones 250, or both one or more accelerometers 252 and one or more microphones 250. Fig. 21E depicts a processor device 104 that includes a PPG sensor 108 and, optionally, one or more microphones 250 and/or one or more accelerometers 252.

Various embodiments are contemplated in which any one of the embodiments 450A-E of the sensor array 102 may be communicatively coupled to any one of the embodiments 460A-E of the processor device 104. For example, Figs. 22A depicts an embodiment 470 in which the sensory array 102, which may take the form of any of the embodiments 450A-E, is communicatively coupled to the processor device 104, which may take the form of any of the embodiments 460A-E. In turn, the processor device 104 may be communicatively coupled to external equipment. The external equipment 472 may be the modeling processor device 314. The external equipment 472 may also be one or more servers that receive and store the data for individual patients and/or communicate the data for the patients to the respective medical personnel or physicians diagnosing and/or treating the patients.

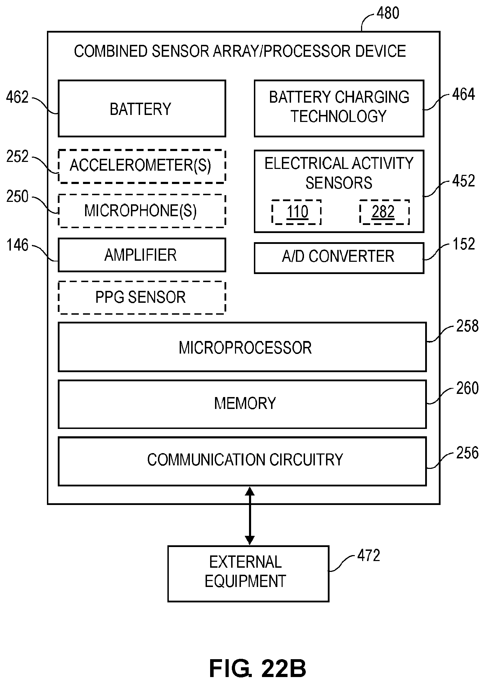

Fig. 22B depicts an alternate embodiment, in which the sensor array 102 and the processor device 104 are integrated into a single unit 480. The combined unit 480 includes the battery 462 and battery charging technology 464 for powering the unit 480. The electrical activity sensors 452 include one or both of the electrode devices 110 and the biochemical sensors 282. As in previously described embodiments, the unit 480 may include one or more PPG sensors 108, one or more accelerometers 252, and/or one or more microphones 250. Additionally, the unit 480 may, as previously described, be communicatively coupled to one or more PPG sensors 108, one or more accelerometers 252, and/or one or more microphones 250, that are external to the unit 480. The amplifier 146 and analog-to-digital converter 152 are also included. The microprocessor 258, memory 260, and communication circuitry 256 function as described throughout.

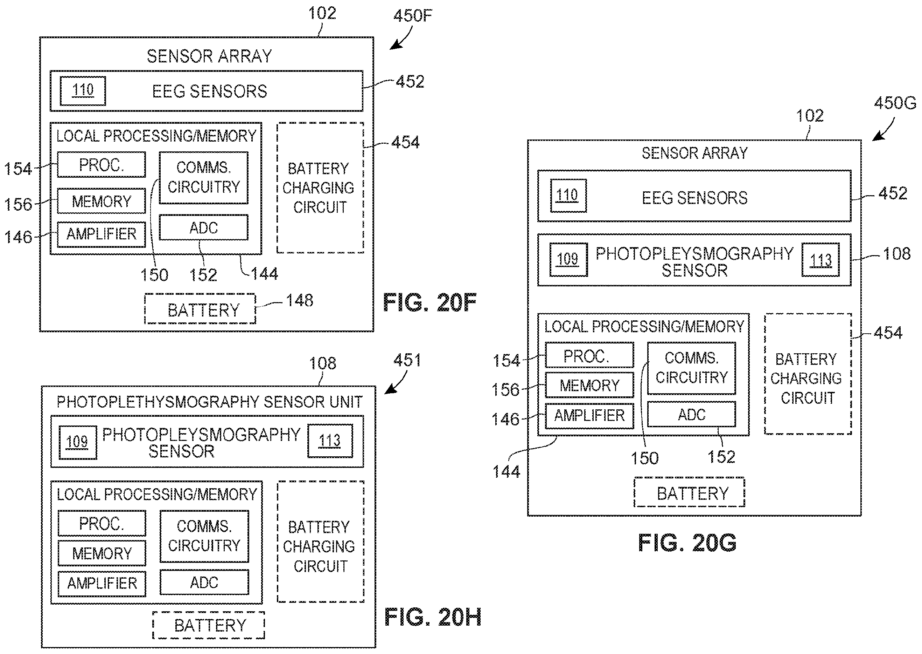

Figs. 20F and 20G depict block diagrams of example embodiments 450F and 450G, respectively, of the sensor array 102, which include the EEG sensors 110 in an array 452. That is, as should by now be understood, the sensor array 102 in any embodiment, includes the one or more electrical activity sensors 452, which may be electrode deices 110, which measure electrical signals in the brain or elsewhere in the body, depending on placement). While the sensor array 102 depicted in Fig. 20F includes only the EEG sensors 542, the sensory array 102 depicted in Fig. 20G also includes the PPG sensor 108. That is, in embodiments such as that depicted in Fig. 20G, the PPG sensor 108 may be integrated with the sensor array 102. In each of the embodiments 450F and 450G, the sensor array 102 includes the local processing device 144, which includes the amplifier 146, the battery 148 (which may be considered part of the local processing unit 144 or external to the local processing unit 144, as depicted), the transceiver or communications circuitry 150, the analog-to-digital converter 152, the processor 154, and the memory 156. Each of the embodiments 450F and 450G may also optionally include a battery charging circuit 454, for facilitating charging of the battery 148. The battery charging circuit 454 may be any known battery charging technology compatible with the arrangement of the sensor array 102 on the patient. In particular, in embodiments the battery charging circuit 454 may be an inductive charging circuit that facilitates charging through the patient's skin when the sensor array 102 is disposed beneath the scalp of the patient. In other embodiments, the battery charging circuit 454 may draw energy from the movements of the patient throughout the day by, for example, harnessing the movements of the patient to turn a small generator. In still other embodiments, the battery charging circuit 454 may draw power from the environment in the form of RF signals. In further examples still, the battery charging circuit 454 may draw power from chemical reactions taking place in the environment of the sensor array 102. Of course, more traditional charging methods (e.g., using a wired connection to provide power to the battery charging circuit 454) may also be employed.

As can be seen in Figs. 20F and 20G, the embodiment 450F does not include in the sensor array 102 the PPG sensor 108. In embodiment 450G, the sensor array 102 includes the PPG sensor 108. Of course, the embodiment 450G of the sensor array 102, may be used with or without additional PPG sensors 108 (i.e., PPG sensors 108 that are not part of the sensor array 102), in various embodiments.

Fig. 20H depicts an embodiment of the PPG sensor 108, illustrating in an embodiment 451 that, like the sensor array 102 depicted in Fig. 20F, the PPG sensor 108 may include local processing and memory elements (similar to the block 144), a battery 148 and, optionally, a battery charging circuit 454.

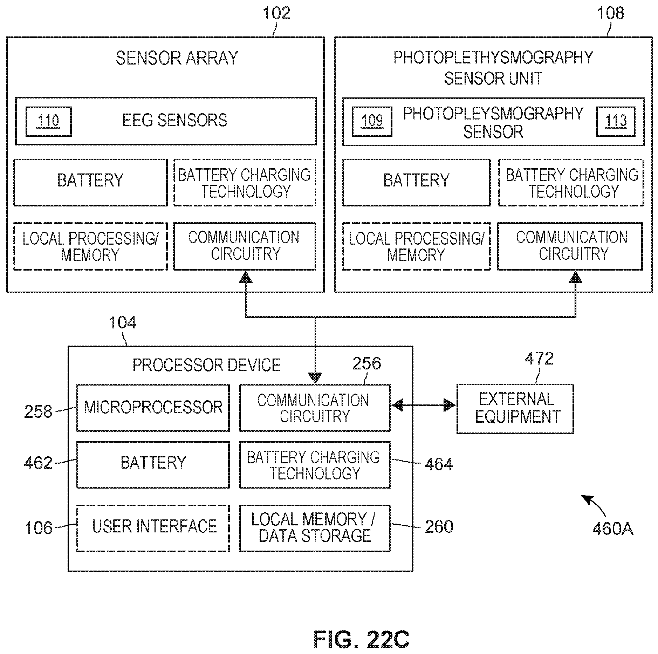

Like the sensor array 102, the processor device 104 is similarly amenable to a variety of embodiments. Figs. 22C-22G are block diagrams of various example embodiments 460A-E, respectively, of the processor device 104. In each of the processor devices 104 depicted in embodiments 460A-D, the processor device 104 includes the communication circuitry 256, the microprocessor 258, and the memory 260. Each of the processor devices 104 in the embodiments 460A-D also includes a battery (or other power source) 462 and battery charging technology 464 (which, obviously, would be omitted in the event that the power source were other than the battery 462). The user interface 106 may also optionally be disposed in the processor device 104.

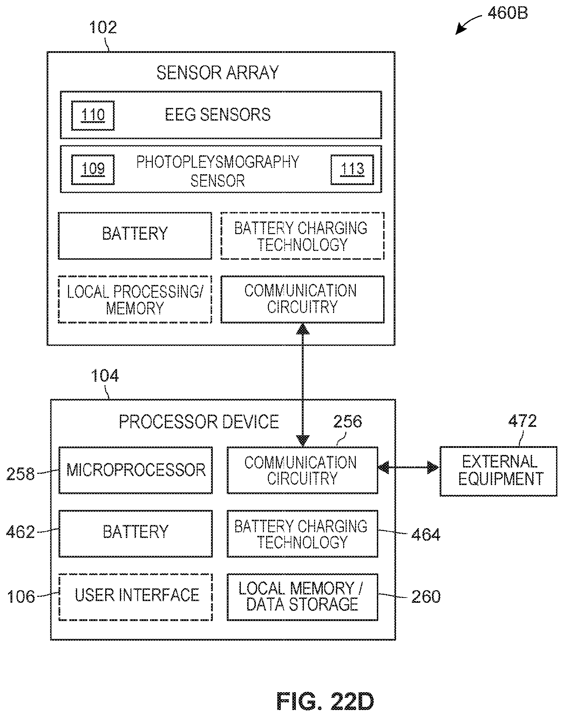

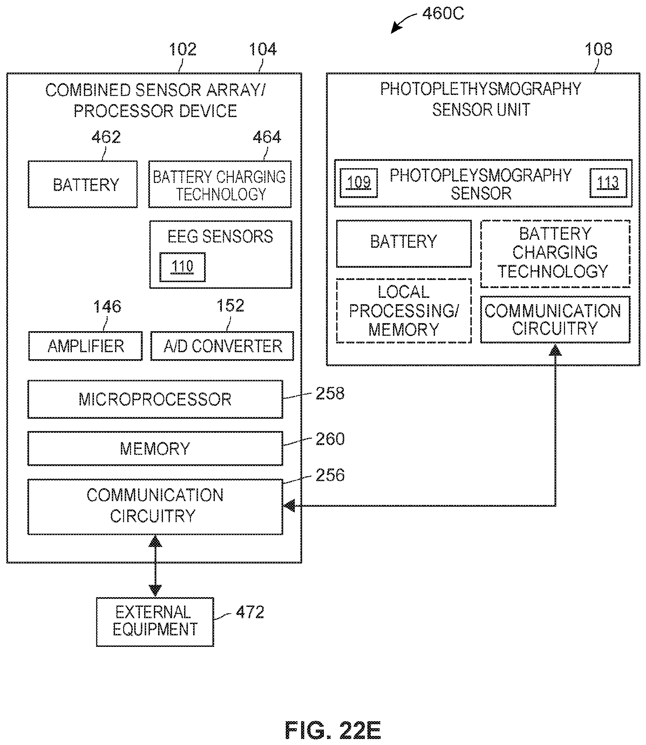

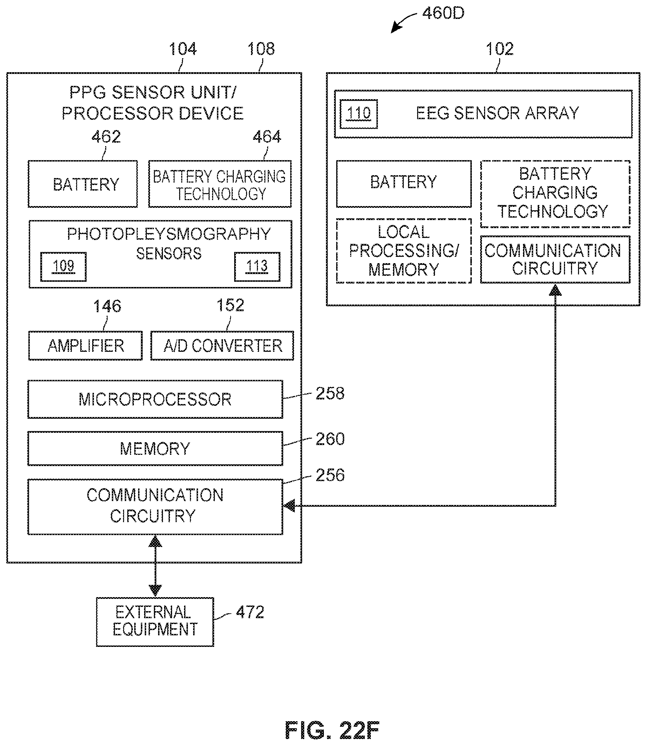

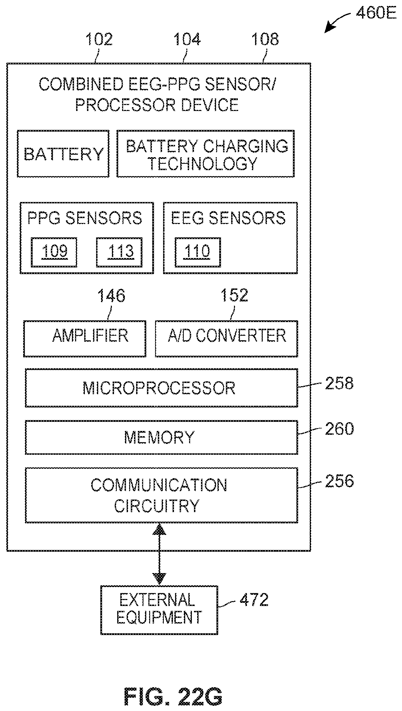

As described throughout the specification, the system 100 includes an EEG sensor array 102 and a PPG sensor 108. In various embodiments, the EEG sensor array 102 and the PPG sensor 108 may be separate from, but communicatively coupled to, the processor device 104, as depicted in Fig. 22C. In other embodiments, one or more PPG sensor 108 may be disposed in the sensor array 102, and coupled to a separate processor device 104, as depicted in Fig. 22D. Figs. 22E and 22F depict respective embodiments in which the processor device 104 is integrated with one or the other of the EEG sensor 102 (Fig. 22E, embodiment 460C) and the PPG sensor 108 (Fig. 22F, embodiment 460D). In a final set of embodiments, the processor device 104 may be integrated with both the EEG sensor 102 and the PPG sensor 108, as depicted in embodiment 460E, depicted in Fig. 22G. In each of the embodiments 460A-460E, the local processor device 104 may be communicatively coupled to external equipment 472, which may be one or more of the modeling processor device 314, the external device 278, the therapeutic device 255, or the external devices 105.

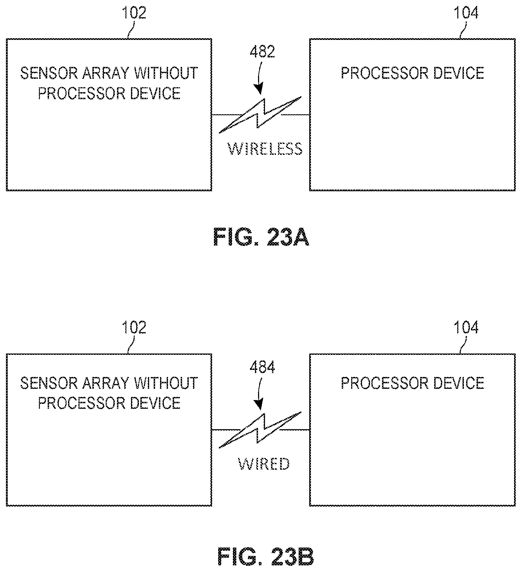

Various communication schemes are contemplated, as well. Figs. 23A and 23B illustrate possible communication schemes between the sensor array 102 and the processor device 104 and, in particular, Fig. 23A illustrates a wireless connection 482 between the sensor array 102 and the processor device 104 (i.e., between the communication circuitry 150 of the sensor array 102 and the communication circuitry 256 of the processor device 104). The wireless connection 482 may be any known type of wireless connection, including a Bluetooth^{®} connection (e.g., low-energy Bluetooth), a wireless internet connect (e.g., IEEE 802.11, known as "WiFi"), a near-field communication connection, or similar. Fig. 23B illustrates a wired connection 484 between the sensor array 102 and the processor device 104. The wired connection may be a serial connection, for example.

The sensor array 102 may communicate data to the processor device 104 as the data are acquired by the sensor array 102 or periodically. For example, the sensor array 102 may store, in the memory 156 of the local processing unit 144, data as it is acquired from the electrode devices 110, biochemical sensors 282, and microphones 250 and/or accelerometers 252 that are part of the sensor array 102 and may periodically (e.g., every second, every minute, every half hour, every hour, every day, when the memory 156 is full, etc.) transmit the data to the processor device 104. In other embodiments, the sensor array 102 may store data until the processor device 104 is coupled to the sensor array 102 (e.g., via wireless or wired connection). The sensor array 102 may also store the data until the processor device 104 requests the transmission of the data from the sensor array 102 to the processor device 104. In these manners, the sensor array 102 may be optimized, for example, to preserve battery life, etc.

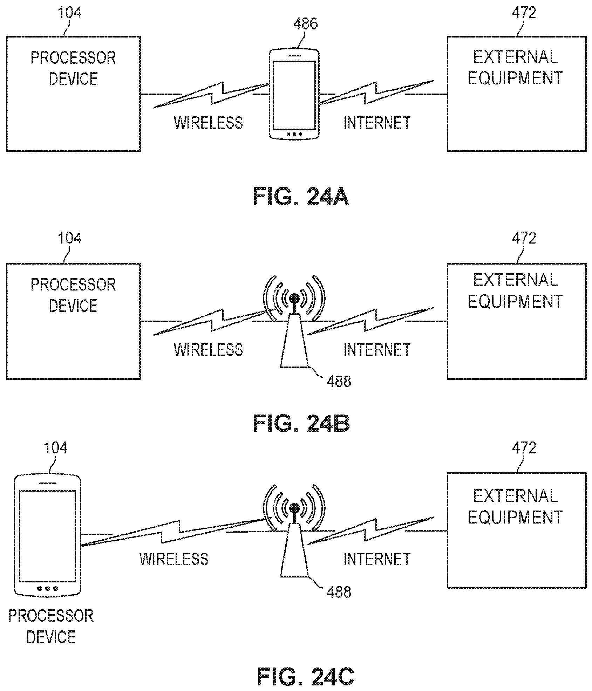

Figs. 24A-24C illustrate possible communication schemes between the processor device 104 and external equipment or servers 472, regardless of whether or not the processor device 104 is integrated with the sensor array 102 (e.g., as in Fig. 23). In Fig. 24A, for example, the processor device 104 may be coupled by a wireless communication connection to a mobile device 486, such as a mobile telephony device, which may, in turn, be coupled to the external equipment 472 by, for example, the Internet. In Fig. 24B, the processor device 104 is coupled to one or more intermediary devices 488 (e.g., a mobile telephony base station, a wireless router, etc.), which in turn provides connectivity to the external equipment 472 via the Internet. In Fig. 24C, the processor device 104 is itself a mobile device, such as a mobile telephony device, which may be coupled by one or more intermediary devices 488 to the external equipment 472 by way of the Internet.

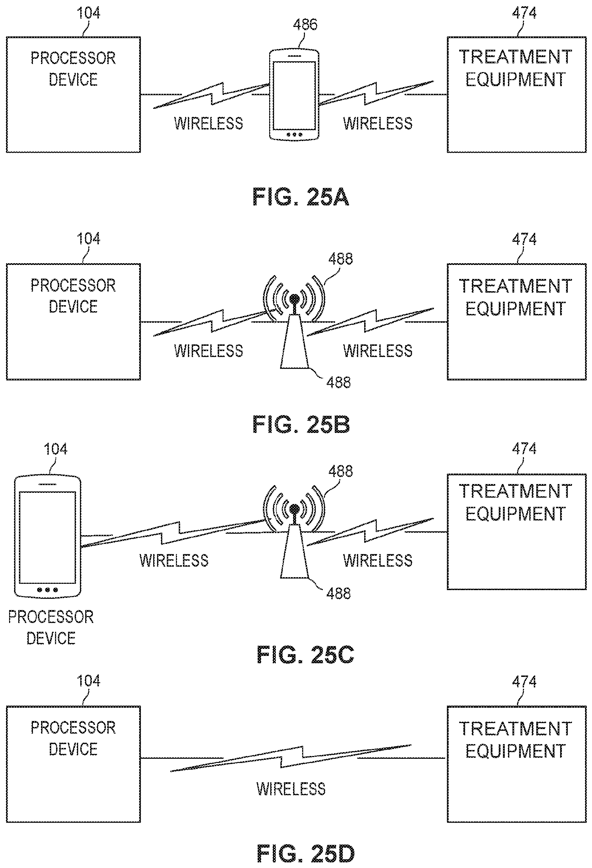

The external equipment may also be treatment equipment 474, in some embodiments depicted in Figs. 25A-25D. The treatment equipment 474 may include devices such as electrical stimulation devices implanted into or in contact with the brain, drug delivery pumps, and the like. The treatment equipment 474 may receive commands or control inputs from the processor device 104, in embodiments, in response to the output of the model 270, 302 and, in particular, in response to detected patterns or events. That is, the processor device 104 may include, stored in the memory 260, one or more routines (not shown) for controlling the treatment equipment 474 in response to the classification results 274. Figs. 25A-25D illustrate possible communication schemes between the processor device 104 and the treatment equipment 474, regardless of whether or not the processor device 104 is integrated with the sensor array 102 (e.g., as in Fig. 23). In Fig. 25A, for example, the processor device 104 may be coupled by a wireless communication connection to a mobile device 486, such as a mobile telephony device, which may, in turn, be wirelessly coupled to the treatment equipment 474. In Fig. 25B, the processor device 104 is coupled to one or more intermediary devices 488 (e.g., a mobile telephony base station, a wireless router, etc.), which in turn provides connectivity to the treatment equipment 474 via a wireless connection. In Fig. 25C, the processor device 104 is itself a mobile device, such as a mobile telephony device, which may be coupled by one or more intermediary devices 488 to the treatment equipment 474 by way of a wireless connection. In Fig. 25D, the processor device 104 communicates directly, via a wireless communication link, with the treatment equipment 474.

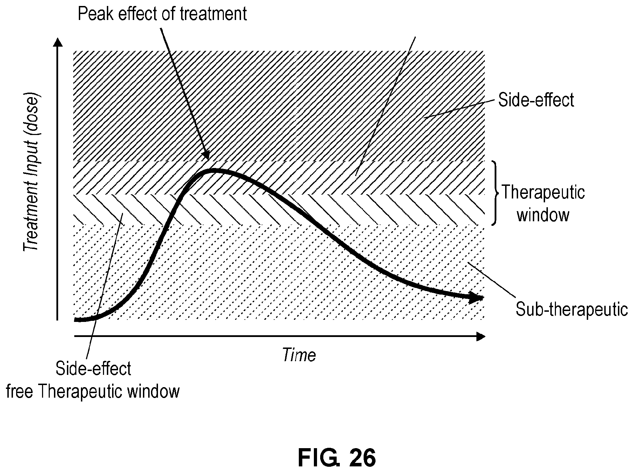

As described above, a second sub-system (e.g., the second sub-system 104B) directed to determining and optimizing a therapeutic window for treatment is also included in embodiments of the contemplated system. The second sub-system may operate sequentially or concurrently with the first sub-system that detects, predicts, and/or categorizes the events as described above, such that the data from the first sub-system is employed to determine an optimized therapeutic input (e.g., pharmacological, neurostimulatory, etc.) for treating the patient's condition(s). Fig. 26 illustrates the general concept that for a given condition being treated by application of a given therapy, there will be a dose of the therapy below which the therapy has no effect (i.e., a sub-therapeutic range of doses), a range of doses for which the therapy improves the condition of the patient (i.e., a therapeutic window), and a range of doses for which the therapy causes one or more side-effects, which range may overlap one or both of the therapeutic range and the sub-therapeutic range. Optimally, the range of doses for which the therapy causes side-effects, while it may overlap with a portion of the therapeutic window, will not overlap with the entirety of the therapeutic window, and will leave a portion of the therapeutic window as a "side-effect free therapeutic window," as depicted in Fig. 26. In embodiments, the second sub-system 104B is configured to determine a therapeutic dose that is in the side-effect free therapeutic window. In other embodiments, the second sub-system 104B is configured to minimize side-effects, or at least minimize certain types of side-effects (e.g., according to patient or physician preferences), while providing therapeutic value.

In view of this, it should be understood that the systems and methods described herein may be adapted to detect, characterize, classify, and predict side-effects of therapeutic treatment, in addition to detecting, characterizing, and predicting events related specifically to the physiological condition. In doing so, the systems and methods may tailor treatment according not only to the presence and/or characteristics of detected and/or predicted events related to the physiological condition and the presence and/or characteristics of the detected and/or predicted effects of those events on patient well-being, but also on the presence and/or characteristics of detected and/or predicted side-effects associated with the therapeutic treatment.

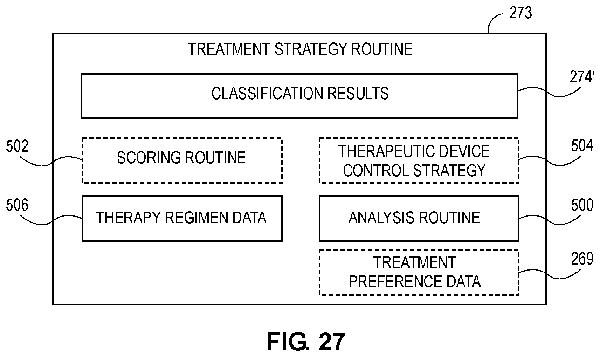

Fig. 27 is a block diagram of the treatment strategy routine 273 which, in embodiments, includes components of the second sub-system. As depicted in Fig. 27, the treatment strategy routine 273 may receive some or all of the classification results 274, 336, 348 output by model 270 or 302. The treatment strategy routine may receive and store a copy of the classification results 274' or, in other embodiments, may read the classification results from their location in the memory 260. In any event, the treatment strategy routine 273 includes an analysis routine 500 configured to receive data from the classification results 274' and to determine a recommended course of action with respect the therapeutic treatment. In embodiments, the treatment strategy routine 273 also includes one or more of a scoring routine 502, a therapeutic device control strategy routine 504, and a store of therapy regimen data 506. In some embodiments, the treatment strategy routine 273 may receive and/or store the treatment preference data 269, which may inform the implementation of the analysis routine 500 and/or the therapeutic device control strategy 504. The treatment preference data 269 may indicate specific therapeutic goal data that may be used (e.g., by the treatment strategy routine 273) to adjust a target therapeutic effect and/or an acceptable level/amount/severity of side-effects. The treatment preference data 269 may be received, in embodiments, from the patient or patient's caretaker via the user interface 106. In other embodiments, the treatment preference data 269 may be received from an external device (e.g., from a physician device communicatively coupled to the system).

Generally speaking, the analysis routine 500 relies on raw data regarding the number of clinical and side-effect events (e.g., from the classification results 274') or scores derived from the classification results 274' by the scoring routine 502, to output recommendations with respect to the optimal dose (in terms of quantity and/or frequency of a pharmacological treatment, amplitude and/or timing of a neurostimulatory treatment, etc.) of a treatment, as described below. In embodiments, the analysis routine 500 may output a recommendation that, in embodiments including a therapeutic device 255 coupled to the system, may be implemented by the therapeutic device control strategy routine 504. The therapeutic device control strategy routine 504 may use, as input to the routine, therapy regimen data 506, which may provide information about acceptable doses, timings, etc., related to the therapy in question. For example, the analysis routine 500 may output a recommendation to increase the dose of the therapy. The therapeutic device control strategy 504 may determine the current dosing regimen being applied, consult the data in the therapy regimen data 506, determine the next higher dose of the therapy, and implement that dose via the therapeutic device 255. Of course, in embodiments, it may be desirable to include a clinician or physician in the therapy control loop. In such embodiments, the analysis routine 500 may output a recommendation that is communicated to a caregiver or physician (e.g., via a message sent to the caregiver device 107A or the physician device 107B). The recommendation may be reviewed and/or approved by the recipient, who may implement the change to the therapy or, in embodiments in which a therapeutic device 255 is implemented, a message may be sent back to the therapeutic device control strategy routine 504 confirming the change, and the routine 504 may control the therapeutic device 255 accordingly.

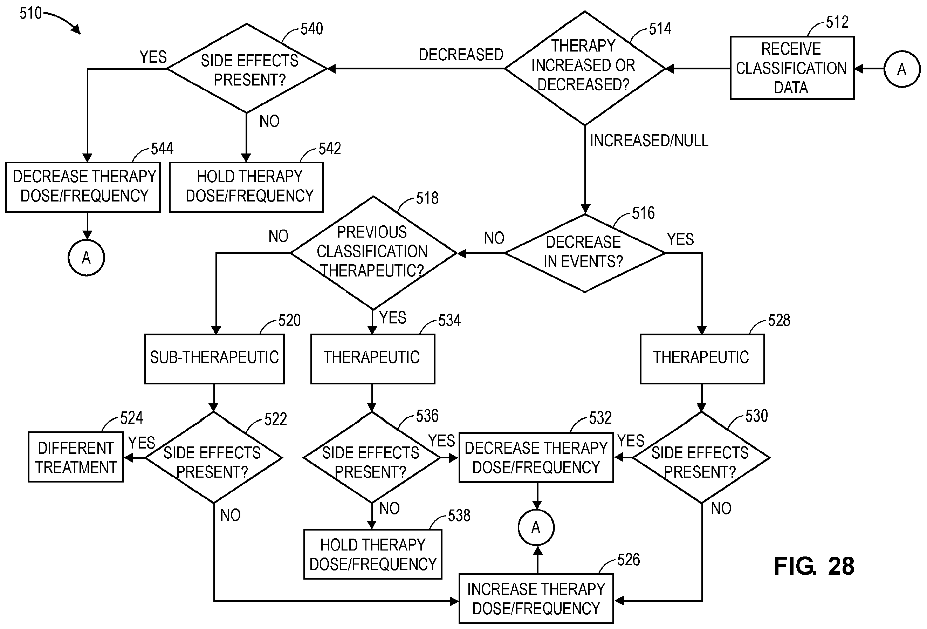

Figs. 28, 29, 30A, and 30B depict various exemplary (i.e., non-limiting) examples of algorithms that may be implemented by the analysis routine 500 to arrive at optimized treatments for a particular patient. Of course, different ones of the algorithms may optimize according to different criteria, as will become apparent in view of the following descriptions. Of course, those of skill in the art will recognize that modifications to these algorithms may be made to achieve different optimization goals, without departing from the contemplated embodiments of this description.

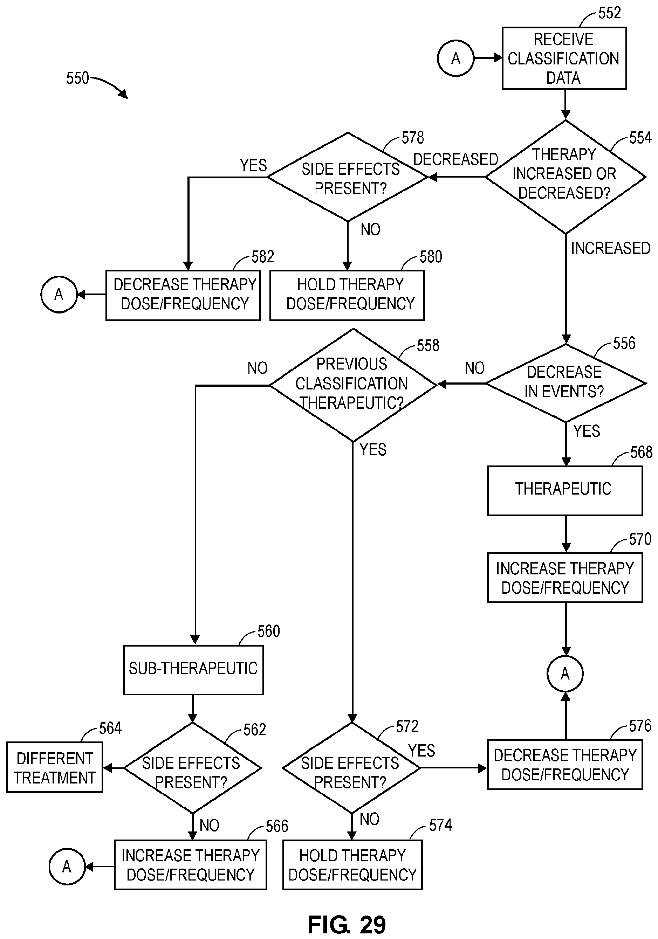

In some embodiments of the algorithms implemented by the analysis routine 500, the analysis routine performs treatment optimization based strictly on the number of clinical events and the presence or absence of side-effects. Such embodiments are depicted in Figs. 28 and 29.

Fig. 28 depicts an exemplary algorithm 510 that may be implemented by the analysis routine 500. Generally speaking, the algorithm 510 operates to increase the therapy dose (i.e., quantity and/or frequency of treatment) until side-effects are detected within a therapeutic observation window, and then decreases the therapy dose until side-effects are eliminated. In the algorithm 510, classification data are received (block 512) by the analysis routine 500. The analysis routine 500 evaluates from previous data stored whether the most recent action was an increase or a decrease in the therapy dose (with a null value - as in the first execution of the algorithm - being treated as an increase) (block 514). If the therapy dose was increased, the algorithm 510 determines from the received classification data whether the increased dose resulted in a decrease in the number of clinical events over the observation window (block 516). The observation window may, for example, correspond to a moving two-week window over which the effects of a treatment two weeks previous are expected to result in a decrease in symptoms or events. Alternatively, the observation window may correspond to a static window extending a particular time frame (e.g., two weeks) from the last change in the dosing regimen of the therapeutic input. However, depending on the types of events and/or the types of therapeutic treatment, the observation window over which data may be compared could be greater than or less than two weeks (e.g., hours, days, one week, three weeks, etc.).

If the increased therapy dose did not result in a decrease in events, the algorithm 510 determines whether the previous dose was classified as therapeutic (with a null value being treated as not therapeutic) (block 518). If the previous dose was not classified as therapeutic, then the algorithm 510 notes that the current does remains sub-therapeutic (block 520) and then looks at the received classification data to determine whether side-effects occurred during the observation window (block 522). If side-effects did occur during the observation window, even while the dose of the therapy was sub-therapeutic, then the algorithm 510 may output a recommendation to consider a different treatment (block 524). On the other hand, if the dose was sub-therapeutic and no side-effects are present, the algorithm 510 may output a recommendation to increase the therapy dose and/or frequency (block 526). This may be repeated until the therapy results in a decrease in events (i.e., until a dose is determined to be therapeutic).

If the increased therapy dose resulted in a decrease in events (block 516), the algorithm 510 notes that the dose is considered to be therapeutic (block 528) and then looks at the received classification data to determine whether side-effects occurred during the observation window (block 530). If no side-effects occurred, then the algorithm 510 may output a recommendation to increase the therapy dose and/or frequency (block 526). If, on the other hand, side-effects are present, algorithm 510 may output a recommendation to decrease the therapy dose and/or frequency (block 532).

If the increased therapy dose did not result in a decrease in events (block 516), the algorithm 510 may evaluate whether the previous dose was considered therapeutic (block 518) and, if so, may note that the current dose also remains therapeutic (i.e., fewer events than the baseline) (block 534). The algorithm 510 may then evaluate the received classification data to determine whether side-effects occurred during the observation window (block 536). If side-effects were present during the observation window, the algorithm 510 may output a recommendation to decrease the therapy dose and/or frequency (block 532). In contrast, if no side-effects were detected during the observation window, the algorithm 510 may output a recommendation to hold the therapy dose and/or frequency steady.

If the therapy dose was decreased previously (block 514), that would indicate that the therapy dose was therapeutic, but that side-effects were present during the observation window. Accordingly the algorithm 510 may continue to evaluate whether side-effects were present as a result of the decreased dose (block 540). If not, then the algorithm 510 may output a recommendation to hold the therapy dose and/or frequency steady (block 542). If side-effects remain present, then the algorithm 510 may output a recommendation to further decrease the therapy dose and/or frequency (block 544).

Fig. 29 depicts a different exemplary algorithm 550 that may be implemented by the analysis routine 500. Generally speaking, the algorithm 550 operates to increase the therapy dose (i.e., quantity and/or frequency of treatment) until the treatment effect stops increasing (i.e., until an increase in dose yields no decrease in clinical events), and then decreases the therapy dose until side-effects are eliminated. In the algorithm 550, classification data are received (block 552) by the analysis routine 550. The analysis routine 550 evaluates from previous data stored whether the most recent action was an increase or a decrease in the therapy dose (with a null value - as in the first execution of the algorithm - being treated as an increase) (block 554). If the therapy dose was increased, the algorithm 550 determines from the received classification data whether the increased dose resulted in a decrease in the number of clinical events over the observation window (block 556).

If the increased therapy dose did not result in a decrease in events, the algorithm 550 determines whether the previous dose was classified as therapeutic (with a null value being treated as not therapeutic) (block 558). If the previous dose was not classified as therapeutic, then the algorithm 550 notes that the current does remains sub-therapeutic (block 560) and then looks at the received classification data to determine whether side-effects occurred during the window (block 562). If side-effects did occur during the observation window, even while the dose of the therapy was sub-therapeutic, then the algorithm 550 may output a recommendation to consider a different treatment (block 564). On the other hand, if the dose was sub-therapeutic and no side-effects are present, the algorithm 550 may output a recommendation to increase the therapy dose and/or frequency (block 566). This may be repeated until the therapy results in a decrease in events (i.e., until a dose is determined to be therapeutic).

If the increased therapy dose resulted in a decrease in events (block 556), the algorithm 550 notes that the dose is considered to be therapeutic (block 568) and outputs a recommendation to increase the therapy dose and/or frequency (block 570). If, on the other hand, the increased therapy dose resulted in no corresponding decrease in events (block 556), and the previous dose was considered therapeutic (block 558), this indicates that a peak treatment effect has been reached, and the algorithm 550 determines whether side-effects are present (block 572). If no side-effects are present, then the algorithm 550 may output a recommendation hold the current dose of the therapy and not to make further adjustments. If, however, side-effects are determined to be present (block 572), then the algorithm 550 outputs a recommendation to decrease the therapy dose and/or frequency (block 576).

Where the algorithm 550 determines that the most recent adjustment was a decrease in the therapy dose (block 554), it is assumed that the reason for doing so was the establishment of a peak treatment effect, and the algorithm 550 checks to see if side-effects remain present after the decrease in the dose of the therapy (block 578). The algorithm 550 outputs a recommendation to hold the current dose of the therapy if no side-effects were observed (block 580) during the observation window, or to further decrease the therapy dose if the side-effects remain (block 582).

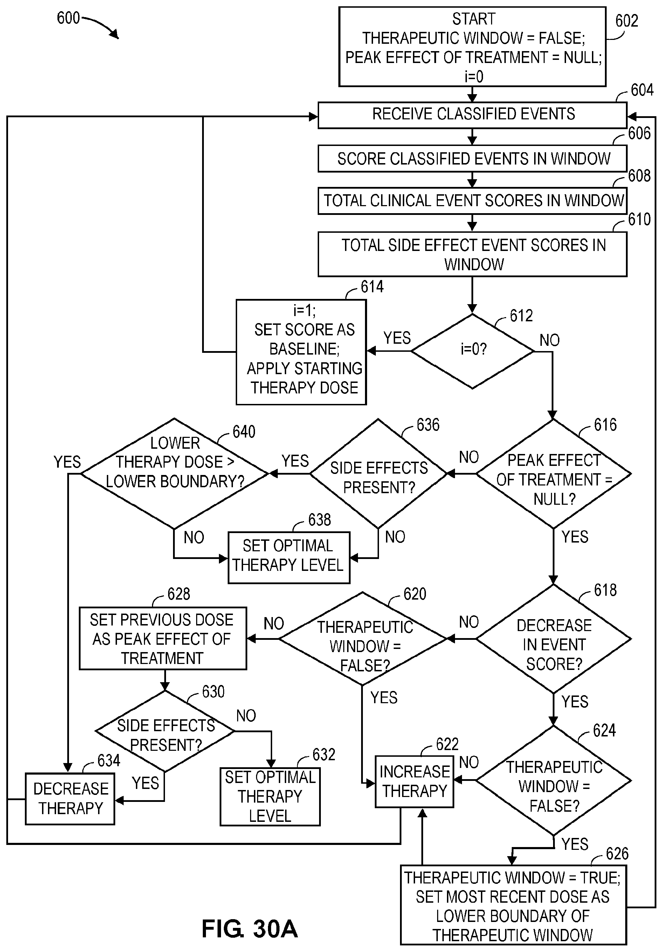

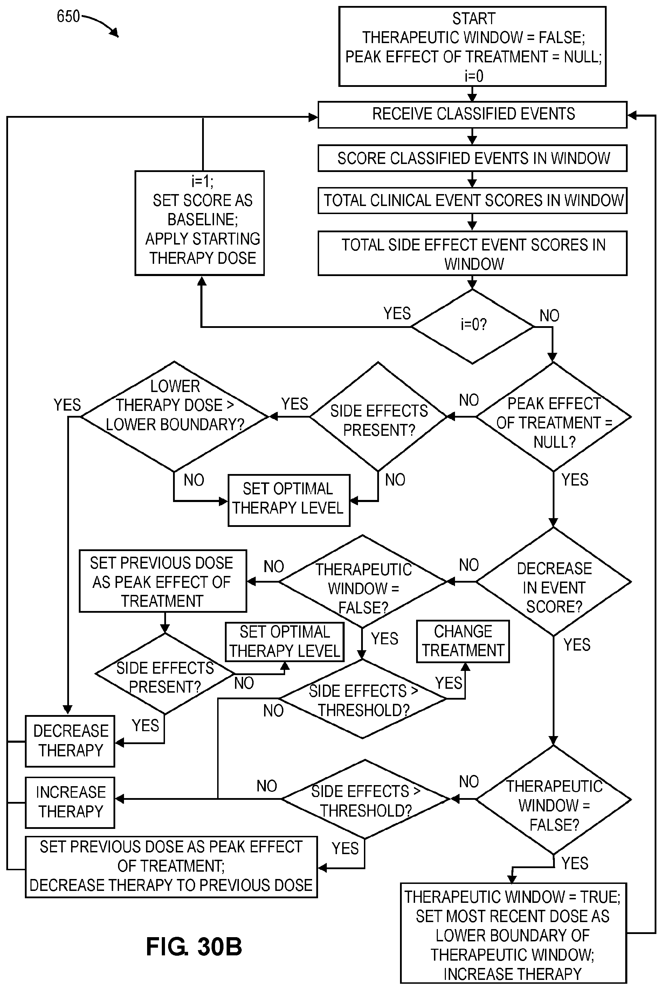

In contrast with Figs. 28 and 29, which provide examples in which algorithms implemented by the analysis routine 500 optimize treatment dose based strictly on the number of clinical events and the presence or absence of side-effects, Figs. 30A and 30B provide examples of algorithms that, when implemented by the analysis routine 500, optimize treatment dose based on scores, computed by the scoring routine 502, corresponding to the events and/or side-effects observed during the observation window.

The algorithm 600 commences with initialization of values (block 602). In particular, a therapeutic window flag may be initialized to "false" or "null," a peak effect of treatment flag or value may be initiated to "false" or "null," and a counter value may be initiated to "0" or "false." The algorithm 600 then receives classified events (block 604) from the most recent observation window.

The algorithm 600 may then employ the scoring routine 502 to score (block 606) the events in the received classified events. The scoring may be based on any number of different schemes, according to the particular condition (e.g., epilepsy, sleep apnea, etc.), the particular treatment (e.g., pharmacological, neurostimulatory, etc.), the types of side-effects experienced and/or expected, and the like. In various embodiments, clinical events and side-effect events may each be scored individually, and a composite score computed. For example, both clinical events and side-effect events may generate positive scores that, summed for the period, generate an overall score that can be employed by the analysis routine 500 to determine whether a therapy is having a positive effect (e.g., generating a decrease in clinical event scores that outweighs any increase in side-effect scores. Alternatively, clinical events and side-effects may each be scored based on a weighting system. By way of example, and without limitation, each clinical and/or side-effect event may be scored by applying weights to event types, severities, durations, effects, and/or time elapsed between the scored event and the previous event (e.g., to consider whether events are becoming less frequent). In this way, certain types of clinical and/or side-effects may be treated as more serious, more severe events may be treated as more serious, and long duration events may be treated as more serious. Additionally, in embodiments, thresholds may be adopted for side-effect scores that, because of the severity of the side-effects, may cause treatment to cease or may cause the dose to be decreased.

In any event, once each of the events has been scored (block 606), the algorithm 600 may total the clinical event scores in the observation window (block 608) and may total the side-effect event scores in the window (block 610). If the counter value is "0" or "false" (block 612) indicating that the algorithm 600 is running for the first time, the counter is set to "1" or "true", and the clinical event score is set as a baseline, and the starting therapy dose is applied (block 614. Thereafter - that is, when the counter value is "1" or "true" (block 612) - the algorithm 600 checks to see whether a peak effect of treatment has been established (block 616). If not, the algorithm 600 evaluates whether the clinical event score (or, in embodiments, the overall score) has decreased (block 618). If the event score did not decrease, and the lower boundary of the therapeutic window has not been established (i.e., is "null") (block 620), then the algorithm 600 outputs a recommendation to increase the therapy dose and/or frequency (block 622), because the algorithm has determined that the current dose is sub-therapeutic.

On the other hand, if there is a decrease in the event score (block 618), and the lower boundary of the therapeutic window has not been established (i.e., is "null") (block 624), then the algorithm 600 may set the current dose as the lower boundary of the therapeutic window (block 626) and may output a recommendation to increase the therapy dose and/or frequency (block 622). If there is a decrease in the event score (block 618), and the lower boundary of the therapeutic window has already been established (i.e., is not "null") (block 624), then the algorithm 600 may output a recommendation to increase the therapy dose and/or frequency (block 622) (e.g., because the previous dose was already in the therapeutic window and the current dose continued to lower the clinical event score).

If the peak effect of treatment has not yet been established (block 616), the most recent observation window does not show a decrease in event score (block 618), and the lower boundary for the therapeutic window has already been established (block 620) - that is, if the current dose is in the therapeutic window but did not cause a further decrease in the clinical event score - then the previous dose is set as the peek effect of treatment (block 628). The algorithm 600 then evaluates whether side-effects are present (block 630). If not, then the previous dose is set as the optimal therapy dose (block 632); if so, then the algorithm 600 outputs a recommendation to decrease the therapy dose and/or frequency (block 634).

Once the peak effect of treatment has been set and a decrease in the dose has been implemented, the algorithm 600 evaluates the observation window events for side-effects (block 636). If no side-effects are present after lowering the dose, the optimal therapy level is set (block 638). If, on the other hand, side-effects remain after lowering the dose (block 636), the algorithm 600 evaluates whether lowering therapy dose again would result in going below the lower boundary of the treatment window (block 640). If so, the current therapy dose is set as the optimal therapy level (block 638); if not, then algorithm 600 outputs a recommendation to lower the therapy dose and/or frequency (block 634).

Fig. 30B depicts an algorithm 650 that is very similar to the algorithm 600 depicted in Fig. 30A. However, in the algorithm 650, the side-effect score is compared to a side-effect score threshold if the previous dose did not result in a decrease in the event score and the lower boundary of the therapeutic window has not yet been determined (i.e., when the dose is sub-therapeutic). If a sub-therapeutic dose of the therapy nevertheless results in side-effects that exceed the threshold, then the algorithm 650 outputs a recommendation to consider changing to a different therapy. Only if the sub-therapeutic dose does not result in side-effects that exceed the threshold does the algorithm 650 output a recommendation to increase the therapy dose.

The side-effect score is compared to a side-effect score threshold if the previous dose resulted in a decrease in the event score and the lower boundary of the therapeutic window has been determined (i.e., when the dose is therapeutic and resulted in a further decrease in the event score). If a therapeutic dose of the therapy results in side-effects that exceed the threshold, then the algorithm 650 sets the previous dose as a peak effect of treatment and outputs a recommendation to decrease the dose and/or frequency to the dose and/or frequency of the prior observation window. Only if the therapeutic dose does not result in side-effects that exceed the threshold does the algorithm 650 output a recommendation to increase the therapy dose.

Of course, as should be understood, each of the algorithms 510, 550, 600, and 650 is exemplary in nature. The analysis routine 500 may implement any number of different algorithms, each of which may use the event classification results to optimize the therapeutic treatment of the condition in question according to specific needs, as would be readily appreciated by a person of skill in the art in view of the present description.

For example, the algorithms described above may be more tolerant of some or all side-effects than suggested by the algorithms described. As indicated in the description above, the patient and/or the clinician may indicate that some side-effects are tolerable if the clinical symptoms (e.g., seizures) abate. Some patients, for example, may be quite happy to accept certain side effects if the clinical symptoms of the underlying condition are eliminated or minimized. One way of accomplishing this may be to include in the scoring of side-effects lower weights for side-effect types that are tolerable by the patient, and higher (or infinite) weights for side-effects that are less tolerable (or entirely intolerable). In this manner, the algorithm may decrease the therapy dose and/or frequency when intolerable events (e.g., arrhythmias) occur at all, while moving toward or staying at the peak therapeutic effect when tolerable events occur. Of course, many variations on the precise implementation of such an algorithm can be readily envisioned in view of this specification.

It will also be understood that certain side-effects may abate as the therapy continues. That is, an increased dose and/or frequency of a treatment may cause increased side-effects temporarily, though the side-effects may abate as the patient's body equilibrates to the new dose and/or frequency of the treatment. Accordingly, algorithms are possible in which earlier side-effect events are weighted lower than later side-effect events, such that the scores of the later side-effect events, which are more likely to occur after the patient's body has equilibrated to some extent, dominate the overall side-effect score. At the same time, clinical efficacy of a treatment, especially a pharmacological therapy, may decline over time as the patient's body adjusts to tolerate the therapy. Accordingly, the system may monitor the number of events (e.g., the number of seizures, etc.) to determine if the efficacy is waning, and the treatment strategy routine 273 may adjust the treatment dose and/or timing to compensate, while taking into account the various considerations relating to side-effects. Further still, in embodiments, the system may receive data from a variety of patients and, as a result, may be configured to predict abatement of therapeutic efficacy (just as it may predict side-effects), and the treatment strategy routine 273 may proactively mitigate the decreasing therapeutic effects while controlling side-effects and maximizing patient well-being.

Still further, the treatment strategy routine 273, in embodiments, may be programmed such that certain side-effects trigger a change in the time of day of treatment administration, rather than a change in the dose and/or frequency of the treatment. As a non-limiting example, some pharmacological therapies may cause a change in wakefulness (e.g., may cause the patient to be more alert or more sleepy). The presence of such side-effects may cause the treatment strategy routine 273 to recommend and/or implement a change in the time of day that the treatment is administered, for example, by administering a drug that causes drowsiness before bedtime instead of first thing in the morning, or by administering a drug that causes wakefulness at a time other than before bedtime or during the night.

In still other embodiments, the treatment strategy routine 273 may recommend a series of sequential and/or concurrent pharmacological therapies. For example, over time, it may become apparent as different pharmacological agents (i.e., drugs) are used to treat the patient, that none of the pharmacological agents by itself sufficiently achieves that treatment goals of the patient (e.g., sufficient treatment of symptoms without unwanted or unacceptable side-effects, etc.), or that the treatment goals are met only briefly until the patient develops a tolerance for the medication. In embodiments, then, the treatment strategy routine 273 may recommend (or implement, via the therapeutic device 255) an increase in the dosage of the drug(s). Alternatively, however, the collected data may indicate that certain combinations and/or sequences of drugs may achieve better results (i.e., fewer, less frequent, and/or less severe symptoms; fewer, less frequent, and/or less severe side effects; etc.) than any one of the drugs by itself. Accordingly, the treatment strategy routine 273 may recommend that a first therapy be followed by a second therapy. In instances, the first and second therapies may overlap - such as when the second therapy is titrated up to a particular dose while the first therapy is titrated down to nothing; in other instances, the first therapy may be stopped (and the drug eliminated from the patient's system) before the second therapy is administered. Still further, the treatments, whether two or more, may be rotated in one order or another according to the patient's response to the various drugs, as monitored, classified, and/or detected by the systems and methods described herein.

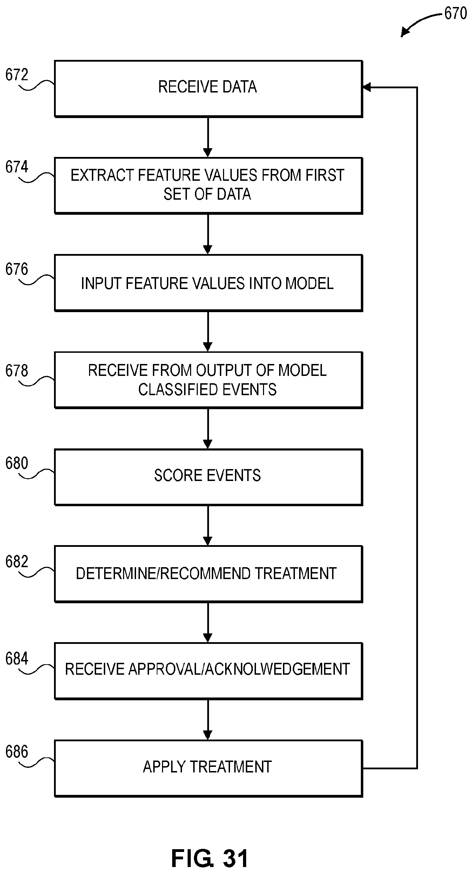

While it should be readily appreciated by this point, the systems and methods herein may detect and classify events, recommend changes in treatment regimen and, in cases having a connected therapeutic device, may apply the change in treatment regimen. Fig. 31, provided to illustrate this concept at a high level, depicts a method 670 in which data (EEG data and PPG data, along with optional microphone and/or accelerometer data, and user reported data) are received (block 672). Feature values are extracted from the received data (block 674) and input into a model (block 676). The model outputs detected and classified events (block 678), which are then scored (block 680) and a treatment recommendation determined (block 682). The treatment recommendation is (optionally) transmitted to a third-party such as a physician and/or caregiver, from which acknowledgement and/or authorization is (optionally) received (block 684), before the determined treatment is applied to the patient (e.g., manually or via the coupled therapeutic device) (block 686).

As will by this point be appreciated, the two sub-systems 104A, 104B may be employed iteratively and/or concurrently to improve the training of the trained Al model 302. For example, in embodiments, the trained Al model 302 may generate classification results 336 including predicted events 372, 387, 399A, 400A that are based, in part, on the current therapeutic regimen. That is, the trained Al model 302 may be trained, at least in part, based on previous data relating treatment doses and times to the occurrence of events and side-effects, to determine predicted events and side-effects based on the detected events and the current treatment dose and times. The trained Al model 302 may thereafter determine whether the predicted data were accurate, and may adjust the model according to later data. The trained Al model 302 may, for instance, determine that previous changes in therapy levels resulted in corresponding changes in detected events and/or side-effects and, as a result, may determine that, based on most recently detected events and side-effects, and the current and/or newly applied therapy regimen, certain concomitant changes in future events and side-effects can be predicted. By iterating this process, the trained Al model 302 may continually update its predictions based on how the therapy applied affects the specific patient or, when data are accumulated across multiple patients, how the therapy applied affects a population of patients.

Additionally or alternatively, the treatment strategy routine 273 may use the predicted event classification data 372, 387, 399A, 400A to adjust the therapy regimen. Accordingly, while the algorithms 510, 550, 600, 650, 670 above generally output and/or apply therapy recommendations based on detected events (i.e., based on events that have already occurred) and by trying to effect a change based on previous events, in embodiments the treatment strategy routine 273 may employ other, similar algorithms based on the predicted event classification data 372, 387, 399A, 400A with the goal of outputting and/or applying therapy recommendations based on predicted events (i.e., based on events that have not yet occurred). In this way, as the trained AI model 302 improves its prediction of future events, the recommendations output by the treatment strategy routine 273 will likewise exhibit improved recommendations, thus improving the overall well-being of the patient.

## Claims

1. A system (100, 300) for analyzing a physiological condition associated with an epilepsy patient, the system comprising:
- a processor device (104) comprising a microprocessor (258), a memory (260), and communication circuitry (256);
- an array (102) of electroencephalogram (EEG) electrodes (110) configured to be disposed on the patient, the array (102)
(i) communicatively coupled to the processor device (104) via the communication circuitry (256),
(ii) generating electroencephalogram (EEG) data (262) from signals detected by the array (102) while the array (102) is disposed on the patient, and
(iii) providing the EEG data (262) to the processor device (104);
- a photoplethysmography sensor (108) configured to be disposed on the patient, the sensor (108)
(i) communicatively coupled to the processor device (104) via the communication circuitry (256),
(ii) generating photoplethysmogram (PPG) data (267) from signals detected by the sensor (108) while the sensor (108) is disposed on the patient, and
(iii) providing the PPG data (267) to the processor device (104);
- a user-interface, stored in the memory (260) and configured to be executed by the microprocessor (258) to cause a display of the processor device (104) to display a user interface (106) configured to receive side-effect data (268) indicating side effects perceived by the patient;
- an analysis routine (270, 302), stored in the memory (260) and configured to be executed by the microprocessor (258), the analysis routine (270, 302) operable to
(1) receive the EEG data (262) and the PPG data (267);
(2) receive the side effect data (268);
(3) determine a plurality of feature values (272), the plurality of feature values (272) including each of: (i) one or more feature values of the EEG data (262), (ii) one or more feature values of the PPG data (267), (iii) one or more features of the side effect data (268); and
(4) based on the plurality of feature values (272), detect and classify events associated with the physiological condition, the classified events (274) including side-effect events,
- a routine (273, 500) configured to monitor the classified events (274, 274') and to determine, from the monitored classified events (274, 274'), an efficacy of an administered therapeutic treatment, wherein the efficacy is classified as (i) sub-therapeutic or (ii) therapeutic and wherein the efficacy is classified as (i) side-effect free or (ii) causing one or more side-effects, and wherein the routine (273, 500) is configured to determine a therapeutic dose that is in a side-effect free therapeutic window.

2. A system according to claim 1, wherein the analysis routine comprises a static model.

3. A system according to claim 1, wherein the analysis routine comprises a trained artificial intelligence (AI) model.

4. A system according to claim 3, wherein the trained Al model is configured according to an Al algorithm based on a previous plurality of feature values.

5. A system according to claim 1, wherein the efficacy is determined according to a number of occurrences of clinical events of the monitored event data.

6. A system according to any one of claims 1 to 5, wherein the events associated with the physiological condition are classified by the analysis routine as epileptic events or non-epileptic events.

7. A system according to any one of claims 1 to 6, the system further comprising a treatment strategy routine, stored in the memory (260) and configured to be executed by the microprocessor (258), the treatment strategy routine operable to (i) recommend a pharmacological agent to treat the physiological condition according to the detected and classified events; and/or (ii) administer, via a treatment device coupled to the microprocessor (258), a pharmacological agent to treat the physiological condition according to the detected and classified events.

8. A system according to any one of claims 1 to 7, the system further comprising a treatment strategy routine, stored in the memory (260) and configured to be executed by the microprocessor (258), the treatment strategy routine operable to (i) recommend a change in a dose, concentration, timing, or frequency of a pharmacological agent to treat the physiological condition according to the detected and classified events; and/or (ii) administer, via a treatment device coupled to the microprocessor, a change in a dose, concentration, timing, or frequency of a pharmacological agent to treat the physiological condition according to the detected and classified events (274, 274').

9. A system according to any one of claims 1 to 8, the system further comprising a treatment strategy routine, stored in the memory (260) and configured to be executed by the microprocessor (258), the treatment strategy routine operable to (i) recommend a vagal nerve stimulation protocol to treat the physiological condition according to the detected and classified events; and/or (ii) administer, via a treatment device coupled to the microprocessor, a vagal nerve stimulation protocol to treat the physiological condition according to the detected and classified events (274, 274').

10. A system according to any one of claims 1 to 9, the system further comprising a treatment strategy routine, stored in the memory (260) and configured to be executed by the microprocessor (258), the treatment strategy routine operable to (i) recommend an epicranial, transcranial, or intracranial stimulation protocol to treat the physiological condition according to the detected and classified events; and/or (ii) administer, via a treatment device coupled to the microprocessor, an epicranial, transcranial, or intracranial stimulation protocol to treat the physiological condition according to the detected and classified events (274, 274').

11. A system according to any one of claims 1 to 10, wherein the analysis routine (270, 302) is further operable to predict, based on the plurality of feature values one or more future events associated with the physiological condition.

## Patentansprüche

1. System (100, 300) zur Untersuchung eines physiologischen Zustands, der mit einem Epilepsiepatienten in Verbindung steht, wobei das System Folgendes umfasst:
- eine Prozessorvorrichtung (104), die einen Mikroprozessor (258), einen Speicher (260) und eine Kommunikationsschaltung (256) umfasst;
- eine Gesamtheit (102) von Elektroenzephalogramm-(EEG-)Elektroden (110), die dafür ausgelegt sind, auf dem Patienten angeordnet zu werden, wobei die Gesamtheit (102)
i) über die Kommunikationsschaltung (256) kommunikationsbezogen mit der Prozessorvorrichtung (104) gekoppelt ist,
(ii) Elektroenzephalogramm-(EEG-)Daten (262) ausgehend von Signalen erzeugt, die von der Gesamtheit (102) erfasst werden, während die Gesamtheit (102) am Patienten angeordnet ist, und
iii) der Prozessorvorrichtung (104) EEG-Daten (262) zuführt;
- einen photoplethysmographischen Sensor (108), der dafür ausgelegt ist, am Patienten angeordnet zu werden, wobei der Sensor (108)
i) über die Kommunikationsschaltung (256) kommunikationsbezogen mit der Prozessorvorrichtung (104) gekoppelt ist,
(ii) Photoplethysmogramm-(PPG-)Daten (267) ausgehend von Signalen erzeugt, die vom Sensor (108) erfasst werden, während der Sensor (108) am Patienten angeordnet ist, und
iii) der Prozessorvorrichtung (104) PPG-Daten (267) zuführt;
- eine Benutzeroberfläche, die im Speicher (260) gespeichert und dafür ausgelegt ist, vom Mikroprozessor (258) ausgeführt zu werden, um zu bewirken, dass eine Anzeige der Prozessorvorrichtung (104) eine Benutzeroberfläche (106) anzeigt, welche dafür ausgelegt ist, Nebenwirkungsdaten (268) zu empfangen, die Nebenwirkungen angeben, welche der Patient verspürt;
- eine Auswertungsroutine (270, 302), die im Speicher (260) gespeichert ist und dafür ausgelegt ist, vom Mikroprozessor (258) ausgeführt zu werden, wobei die Auswertungsroutine (270, 302) derart betrieben werden kann, dass sie
(1) die EEG-Daten (262) und die PPG-Daten (267) empfängt;
(2) die Nebenwirkungsdaten (268) empfängt;
(3) eine Mehrzahl an Merkmalswerten (272) bestimmt, wobei die Mehrzahl an Merkmalswerten (272) jeden der folgenden beinhaltet: i) einen oder mehrere Merkmalswerte der EEG-Daten (262), (ii) einen oder mehrere Merkmalswerte der PPG-Daten (267), (iii) ein oder mehrere Merkmale der Nebenwirkungsdaten (268); und
(4) auf der Grundlage der Mehrzahl an Merkmalswerten (272) Ereignisse im Zusammenhang mit dem physiologischen Zustand erfasst und einstuft, wobei zu den eingestuften Ereignissen (274) Nebenwirkungsereignisse gehören,
- eine Routine (273, 500), die dafür ausgelegt ist, die eingestuften Ereignisse (274, 274') zu überwachen und anhand der überwachten Ereignisdaten (274, 274') die Wirksamkeit einer verabreichten therapeutischen Behandlung zu bestimmen, wobei die Wirksamkeit als i) subtherapeutisch oder ii) therapeutisch eingestuft wird und wobei die Wirksamkeit als i) nebenwirkungsfrei oder ii) eine oder mehrere Nebenwirkungen verursachend eingestuft wird, und wobei die Routine (273, 500) dafür ausgelegt ist, eine therapeutische Dosis zu bestimmen, die in einem nebenwirkungsfreien therapeutischen Fenster liegt.

2. System gemäß Anspruch 1, wobei die Auswertungsroutine ein statisches Modell umfasst.

3. System gemäß Anspruch 1, wobei die Auswertungsroutine ein trainiertes Modell der künstlichen Intelligenz (Kl) umfasst.

4. System gemäß Anspruch 3, wobei das trainierte Kl-Modell gemäß einem KI-Algorithmus ausgelegt ist, welcher auf einer vorhergehenden Mehrzahl an Merkmalswerten beruht.

5. System gemäß Anspruch 1, wobei die Wirksamkeit gemäß einer Anzahl des Auftretens klinischer Ereignisse der überwachten Ereignisdaten bestimmt wird.

6. System gemäß einem beliebigen der Ansprüche 1 bis 5, **dadurch gekennzeichnet, dass** die mit dem physiologischen Zustand in Verbindung stehenden Ereignisse von der Auswertungsroutine als epileptische Ereignisse oder nicht-epileptische Ereignisse eingestuft werden.

7. System gemäß einem beliebigen der Ansprüche 1 bis 6, wobei das System weiterhin eine Behandlungsstrategie-Routine umfasst, die im Speicher (260) gespeichert und dafür ausgelegt ist, vom Mikroprozessor (258) ausgeführt zu werden, wobei die Behandlungsstrategie-Routine derart betrieben werden kann, dass sie (i) entsprechend den erfassten und eingestuften Ereignissen ein pharmakologisches Mittel zur Behandlung des physiologischen Zustands empfiehlt; und/oder (ii) entsprechend den erfassten und eingestuften Ereignissen über eine an den Mikroprozessor (258) gekoppelte Behandlungsvorrichtung ein pharmakologisches Mittel verabreicht, um den physiologischen Zustand zu behandeln.

8. System gemäß einem beliebigen der Ansprüche 1 bis 7, wobei das System weiterhin eine Behandlungsstrategie-Routine umfasst, die im Speicher (260) gespeichert und dafür ausgelegt ist, vom Mikroprozessor (258) ausgeführt zu kann, wobei die Behandlungsstrategie-Routine derart betrieben werden kann, dass sie (i) entsprechend den erfassten und eingestuften Ereignissen eine Änderung empfiehlt, was die Dosis, die Konzentration, den Anwendungszeitpunkt oder die Frequenz eines pharmakologischen Wirkstoffs zur Behandlung des physiologischen Zustands betrifft; und/oder (ii) entsprechend den erfassten und eingestuften Ereignissen (274, 274') über eine an den Mikroprozessor gekoppelte Behandlungsvorrichtung eine geänderte Verabreichung durchführt, was die Dosis, die Konzentration, den Anwendungszeitpunkt oder die Frequenz eines pharmakologischen Wirkstoffs zur Behandlung des physiologischen Zustands betrifft.

9. System gemäß einem beliebigen der Ansprüche 1 bis 8, wobei das System weiterhin eine Behandlungsstrategie-Routine umfasst, die im Speicher (260) gespeichert und dafür ausgelegt ist, vom Mikroprozessor (258) ausgeführt zu werden, wobei die Behandlungsstrategie-Routine derart betrieben werden kann, dass sie (i) entsprechend den erfassten und eingestuften Ereignissen ein Protokoll zur Vagusnervstimulation empfiehlt, um den physiologischen Zustand zu behandeln; und/oder (ii) entsprechend den erfassten und eingestuften Ereignissen (274, 274') über eine an den Mikroprozessor gekoppelte Behandlungsvorrichtung ein Protokoll zur Vagusnervstimulation verabfolgt, um den physiologischen Zustand zu behandeln.

10. System gemäß einem beliebigen der Ansprüche 1 bis 9, wobei das System weiterhin eine Behandlungsstrategie-Routine umfasst, die im Speicher (260) gespeichert und dafür ausgelegt ist, vom Mikroprozessor (258) ausgeführt werden kann, wobei die Behandlungsstrategie-Routine derart betrieben werden kann, dass sie (i) entsprechend den erfassten und eingestuften Ereignissen ein epikranielles, transkranielles oder intrakranielles Stimulationsprotokoll empfiehlt, um den physiologischen Zustand zu behandeln; und/oder (ii) entsprechend den erfassten und eingestuften Ereignissen (274, 274') über eine an den Mikroprozessor gekoppelte Behandlungsvorrichtung ein epikranielles, transkranielles oder intrakranielles Stimulationsprotokoll verabfolgt, um den physiologischen Zustand zu behandeln.

11. System gemäß einem beliebigen der Ansprüche 1 bis 10, wobei die Auswertungsroutine (270, 302) weiterhin derart betrieben werden kann, dass sie auf der Grundlage der Mehrzahl an Merkmalswerten ein oder mehrere zukünftige Ereignisse vorhersagt, die mit dem physiologischen Zustand in Verbindung stehen.

## Revendications

1. Système (100, 300) pour analyser un état physiologique associé à un patient épileptique, le système comprenant :
- un dispositif de processeur (104) comprenant un microprocesseur (258), une mémoire (260) et des circuits de communication (256) ;
- un réseau (102) d'électrodes d'électroencéphalogramme (EEG) (110) configurées pour être disposées sur le patient, le réseau (102)
(i) étant couplé en communication au dispositif de processeur (104) via les circuits de communication (256),
(ii) générant des données d'électroencéphalogramme (EEG) (262) à partir de signaux détectés par le réseau (102) tandis que le réseau (102) est disposé sur le patient, et
(iii) fournissant les données EEG (262) au dispositif de processeur (104) ;
- un capteur de photopléthysmographie (108) configuré pour être disposé sur le patient, le capteur (108)
(i) étant couplé en communication au dispositif de processeur (104) via les circuits de communication (256),
(ii) générant des données de photopléthysmographie (PPG) (267) à partir de signaux détectés par le capteur (108) tandis que le capteur (108) est disposé sur le patient, et
(iii) fournissant les données PPG (267) au dispositif de processeur (104) ;
- une interface utilisateur, stockée dans la mémoire (260) et configurée pour être exécutée par le microprocesseur (258) pour amener un dispositif d'affichage du dispositif de processeur (104) à afficher une interface utilisateur (106) configurée pour recevoir des données d'effets secondaires (268) indiquant des effets secondaires perçus par le patient ;
- une routine d'analyse (270, 302), stockée dans la mémoire (260) et configurée pour être exécutée par le microprocesseur (258), la routine d'analyse (270, 302) étant opérationnel pour
(1) recevoir les données EEG (262) et les données PPG (267) ;
(2) recevoir les données d'effets secondaires (268) ;
(3) déterminer une pluralité de valeurs de caractéristique (272), la pluralité de valeurs de caractéristique (272) incluant chacune parmi : (i) une ou plusieurs valeurs de caractéristique des données EEG (262), (ii) une ou plusieurs valeurs de caractéristique des données PPG (267), (iii) une ou plusieurs caractéristiques des données d'effets secondaires (268) ; et
(4) sur la base de la pluralité de valeurs de caractéristiques (272), détecter et classer des événements associés à l'état physiologique, les événements classés (274) comprenant les événements d'effets secondaires,
- une routine (273, 500) configurée pour surveiller les événements classés (274, 274') et pour déterminer, à partir des données d'événements surveillés (274, 274'), une efficacité d'un traitement thérapeutique administré, dans lequel l'efficacité est classée comme (i) sous-thérapeutique ou (ii) thérapeutique et dans lequel l'efficacité est classée comme (i) sans effet secondaire ou (ii) provoquant un ou plusieurs effets secondaires, et dans lequel la routine (273, 500) est configurée pour déterminer une dose thérapeutique qui est dans une fenêtre thérapeutique sans effet secondaire.

2. Système selon la revendication 1, dans lequel la routine d'analyse comprend un modèle statique.

3. Système selon la revendication 1, dans lequel la routine d'analyse comprend un modèle d'intelligence artificielle (IA) entraîné.

4. Système selon la revendication 3, dans lequel le modèle d'IA entraîné est configuré selon un algorithme d'IA sur la base d'une pluralité précédente de valeurs de caractéristique.

5. Système selon la revendication 1, dans lequel l'efficacité est déterminée en fonction d'un certain nombre d'occurrences d'événements cliniques des données d'événements surveillés.

6. Système selon l'une quelconque des revendications 1 à 5, dans lequel les événements associés à l'état physiologique sont classés par la routine d'analyse comme des événements épileptiques ou des événements non épileptiques.

7. Système selon l'une quelconque des revendications 1 à 6, le système comprenant en outre une routine de stratégie de traitement, stockée dans la mémoire (260) et configurée pour être exécutée par le microprocesseur (258), la routine de stratégie de traitement étant opérationnelle pour (i) recommander un agent pharmacologique pour traiter l'état physiologique en fonction des événements détectés et classés ; et/ou (ii) administrer, via un dispositif de traitement couplé au microprocesseur (258), un agent pharmacologique pour traiter l'état physiologique en fonction des événements détectés et classés.

8. Système selon l'une quelconque des revendications 1 à 7, le système comprenant en outre une routine de stratégie de traitement, stockée dans la mémoire (260) et configurée pour être exécutée par le microprocesseur (258), la routine de stratégie de traitement étant opérationnel pour (i) recommander un changement de dose, de concentration, de synchronisation ou de fréquence d'un agent pharmacologique pour traiter l'état physiologique en fonction des événements détectés et classés ; et/ou (ii) administrer, via un dispositif de traitement couplé au microprocesseur, un changement de dose, de concentration, de synchronisation ou de fréquence d'un agent pharmacologique pour traiter l'état physiologique en fonction des événements détectés et classés (274, 274').

9. Système selon l'une quelconque des revendications 1 à 8, le système comprenant en outre une routine de stratégie de traitement, stockée dans la mémoire (260) et configurée pour être exécutée par le microprocesseur (258), la routine de stratégie de traitement étant opérationnelle pour (i) recommander un protocole de stimulation du nerf vagal pour traiter l'état physiologique en fonction des événements détectés et classés ; et/ou (ii) administrer, via un dispositif de traitement couplé au microprocesseur, un protocole de stimulation du nerf vagal pour traiter l'état physiologique en fonction des événements détectés et classés (274, 274').

10. Système selon l'une quelconque des revendications 1 à 9, le système comprenant en outre une routine de stratégie de traitement, stockée dans la mémoire (260) et configurée pour être exécutée par le microprocesseur (258), la routine de stratégie de traitement étant opérationnelle pour (i) recommander un protocole de stimulation épicrânienne, transcrânienne ou intracrânienne pour traiter l'état physiologique en fonction des événements détectés et classés ; et/ou (ii) administrer, via un dispositif de traitement couplé au microprocesseur, un protocole de stimulation épicrânienne, transcrânienne ou intracrânienne pour traiter l'état physiologique en fonction des événements détectés et classés (274, 274').

11. Système selon l'une quelconque des revendications 1 à 10, dans lequel la routine d'analyse (270, 302) est en outre opérationnelle pour prédire, sur la base de la pluralité de valeurs de caractéristique, un ou plusieurs événements futurs associés à l'état physiologique.
